(19) Europäisches Patentamt / European Patent Office / Office européen des brevets

(11) **EP 4 458 956 A1**

(12) **EUROPEAN PATENT APPLICATION**
published in accordance with Art. 153(4) EPC

(43) Date of publication:
**06.11.2024 Bulletin 2024/45**

(51) International Patent Classification (IPC):
**C12N 5/10** (2006.01)  **C07K 16/28** (2006.01)
**C12N 15/13** (2006.01)  **C12N 15/62** (2006.01)
**A61K 39/395** (2006.01)  **A61P 35/00** (2006.01)

(21) Application number: **22915062.8**

(22) Date of filing: **29.12.2022**

(52) Cooperative Patent Classification (CPC):
**A61K 39/00; A61K 39/395; A61P 35/00;
C07K 16/00; C07K 16/28; C07K 19/00; C12N 5/10;
C12N 15/62; C12N 15/85**

(86) International application number:
**PCT/CN2022/143407**

(87) International publication number:
**WO 2023/125813 (06.07.2023 Gazette 2023/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.12.2021 CN 202111681582**

(71) Applicants:
• **Shanghai Cell Therapy Group Co., Ltd
Jiading District
Anting Town
Shanghai 201805 (CN)**
• **SHANGHAI CELL THERAPY GROUP
PHARMACEUTICAL
TECHNOLOGY CO., LTD.
Shanghai 201805 (CN)**

(72) Inventors:
• **ZHU, Xingli
Shanghai 201805 (CN)**
• **LIU, Xiangzhen
Shanghai 201805 (CN)**
• **SUN, Yan
Shanghai 201805 (CN)**
• **DING, Na
Shanghai 201805 (CN)**
• **QIAN, Qijun
Shanghai 201805 (CN)**

(74) Representative: **Cabinet Beau de Loménie
158, rue de l'Université
75340 Paris Cedex 07 (FR)**

Remarks:
The complete document including Reference Table(s) and the Sequence Listing(s) can be downloaded from the EPO website

(54) **ANTI-MESOTHELIN NANOBODY CHIMERIC ANTIGEN RECEPTOR AND USE THEREOF**

(57) Provided is a chimeric antigen receptor. An antigen binding domain thereof comprises a mesothelin binding melocule containing an anti-mesothelin single-domain antibody. Immune cells containing the chimeric antigen receptor described herein have a higher cell killing effect.

EP 4 458 956 A1

**Description**

TECHNICAL FIELD

The present description relates to the field of chimeric antigen receptor therapy, specifically to an anti-mesothelin nanoantibody chimeric antigen receptors and use thereof.

BACKGROUND

The mesothelin (MSLN) gene is located on chromosome 1p13.3, with a total length of 8kD. The gene comprises an open reading frame of 1884bp, encoding 17 exons and 628 amino acids. The precursor protein of MSLN is a glycoprotein of about 69kD anchored on the cell membrane by a glycosyl phospholipid peptide inositol, which can be hydrolyzed into two parts by a protease, wherein the N-terminal is a soluble protein of 31kD with megakaryocyte stimulating activity, which is known as megakaryocyte potentiating factor (MPF); and the C-terminal is a membrane-bound protein of about 40kD with cell adhesion, which is called MSLN. The structure of MSLN in membrane protein can be divided into three different segments, wherein segment I is the binding site for ligand CA125.
Mesothelin, as a differentiation antigen, is highly expressed in a variety of malignant tumors and is closely related to the occurrence and development of tumors. This target is one of the most potential anti-tumor targets. Many large pharmaceutical companies have targeted this target for development. Currently, the drug forms entering clinical trials include CAR-T, monoclonal antibody, ADC, etc. In the past 20 years, a variety of monoclonal antibodies against mesothelin have been developed, mainly including SS1P immunotoxin and MORAB-009. SS1P is a recombinant immunotoxin composed of scFv fused with truncated pseudo extracellular toxin, which mainly mediates cell killing; and MORAB-009 is an IgG1 antibody formed by recombining SS1P sequence, which mainly initiates antibody dependent cell-mediated cytotoxicity (ADCC).
SS1P antibody binds to the segment I of mesothelin and can block the binding of CA125 to mesothelin. This antibody has also been used in the treatment of CAR-T, but the efficacy is not ideal (Jiang, H., et. al., Protein Cell 8, 926-931, 2017; Adusumilli, P. S. et. al., Sci. Transl. Med. 6, 261ra151261ra151, 2014; Lanitis, E. et. al., Mol. Ther. 20, 633-643, 2012). Nanobodies have natural advantages of high stability, strong penetration and wide range of binding epitopes (Muyldermans S. AnnuRev Biochem. 2013; 82:775-97). There are no reports in this field using MSLN Nanobodies for CAR-T treatment.
At present, although CAR-T therapy has achieved great results in the treatment of hematological tumors, the response rate for solid tumors is low. CAR-T therapy combined with PD-1 inhibitors is an emerging therapy for the treatment of solid tumors. Researchers at the Memorial Sloan Kettering Cancer Center in the United States constructed a CAR-T cell that can secrete anti-PD-1 single-chain antibody (scFv) (Sarwish Rafiq et al., Nat Biotechnol 2018 Oct; 36(9): 847-856). However, studies have found that traditional scFv antibodies have low expression levels in T cells and being difficult to fully exert the effect of PD-1 inhibitors. This is mainly due to the properties of scFv antibodies, such as easy aggregation, easy mismatching in folding, and low thermal stability (Martin N et al., Macromol Biosci. 2017 Feb; 17(2)). Nanobodies have natural advantages such as easy expression, high stability, and high affinity. Based on the advantages of Nanobodies and the biological mechanisms of MSLN and PD-1, the development of a CAR-T based on MSLN Nanobodies, as well as a therapy combined with self-secreting PD-1 Nanobodies, has become an urgent problem to be solved.

SUMMARY

The present description provides a chimeric antigen receptor, comprising an optional signal peptide sequence, a mesothelin-binding molecule containing an anti-mesothelin single domain antibody, a hinge region, a transmembrane region, and an intracellular region.
In one or more embodiments, the intracellular domain includes an intracellular costimulatory domain and/or an intracellular signaling domain.
In one or more embodiments, the anti-mesothelin single domain antibody is a specific single domain antibody to the segment III of mesothelin (the proximal membrane end).
In one or more embodiments, the anti-mesothelin single domain antibody comprises CDR1, CDR2 and CDR3, wherein CDR1 comprises the sequence shown in SEQ ID NO:1, CDR2 comprises the sequence shown in SEQ ID NO:2, and CDR3 comprises the sequence shown in SEQ ID NO:3.
In one or more embodiments, the CDR1 of the anti-mesothelin single domain antibody comprises the sequence shown in any one of SEQ ID NOs: 4-27, 73. Preferably, the CDR1 comprises the sequence shown in any one of SEQ ID NO: 5, 11, 14, 15, 17, 21, 27, 73.
In one or more embodiments, the CDR2 of the anti-mesothelin single domain antibody comprises the sequence shown in any one of SEQ ID NO: 28-47, 74. Preferably, the CDR2 comprises the sequence shown in any one of SEQ ID NO: 29, 35, 37, 38, 39, 43, 40, 74.

In one or more embodiments, the CDR3 of the anti-mesothelin single domain antibody comprises the sequence shown in any one of SEQ ID NO: 48-72, 75. Preferably, the CDR3 comprises the sequence shown in any one of SEQ ID NO: 49, 55, 58, 59, 61, 65, 72, 75.

In one or more embodiments, the FR region of the anti-mesothelin single domain antibody is the FR region of any VHH selected from SEQ ID NOs: 76-120.

In one or more embodiments, the VHH of the anti-mesothelin single domain antibody is as shown in any one of SEQ ID NOs: 76-120. Preferably, the anti-mesothelin single domain antibody is as shown in any one of SEQ ID NO: 77, 83, 86, 87, 89, 93, 100, 103.

In one or more embodiments, the mesothelin binding molecule is a monovalent or multivalent single domain antibody, a multispecific single domain antibody, a heavy chain antibody or an antigen binding fragment thereof, an antibody or an antigen binding fragment thereof comprising one, two or more of the single domain antibodies.

In one or more embodiments, the chimeric antigen receptor comprises a signal peptide, a mesothelin-binding molecule including an anti-mesothelin single-domain antibody, a hinge region, a transmembrane region, an intracellular costimulatory domain, and an intracellular signaling domain in sequence from N-terminal to C-terminal.

In one or more embodiments, the signal peptide is a CD8 signal peptide, a CD28 signal peptide, a CD4 signal peptide or a light chain signal peptide. More preferably, the signal peptide is a CD8 signal peptide. Preferably, the amino acid sequence of the CD8 signal peptide is shown as amino acid residues of positions 1-22 of SEQ ID NO: 121.

In one or more embodiments, the hinge region is selected from the group consisting of: a CD8 hinge region, an IgD hinge region, an IgG1 Fc CH2CH3 hinge region, and an IgG4 Fc CH2CH3 hinge region. Preferably, the hinge region is a CD8 hinge region. More preferably, the amino acid sequence of the CD8 hinge region is shown as amino acid residues of positions 149-203 of SEQ ID NO: 121.

In one or more embodiments, the transmembrane region is one of the groups of: CD28 transmembrane region, CD8 transmembrane region, CD3$\zeta$ transmembrane region, CD134 transmembrane region, CD137 transmembrane region, ICOS transmembrane region and DAP10 transmembrane region. Preferably the transmembrane region is the CD28 transmembrane region. More preferably, the amino acid sequence of the transmembrane region is shown as amino acid residues of positions 204-231 of SEQ ID NO: 121.

In one or more embodiments, the intracellular costimulatory domain comprises an intracellular domain of a costimulatory signaling molecule, comprising the intracellular domains of CD28, CD134/OX40, CD137/4-1BB, lymphocyte-specific protein tyrosine kinase, inducible T cell co-stimulator (ICOS), and DNAX activating protein 10. Preferably, the intracellular costimulatory domain is the intracellular domain of CD28. Preferably, the amino acid sequence of the intracellular domain of CD28 is shown as amino acid residues of positions 231-272 of SEQ ID NO: 121.

In one or more embodiments, the intracellular signaling domain is a CD3$\zeta$ intracellular signaling domain or an Fc$\epsilon$RI$\gamma$ intracellular signaling domain. Preferably the intracellular signaling domain is the CD3$\zeta$ intracellular signaling domain. Preferably, the amino acid sequence of the CD3$\zeta$ intracellular signaling domain is shown as amino acid residues of positions 273-384 of SEQ ID NO: 121.

In one or more embodiments, the chimeric antigen receptor comprises CD8 signal peptide, anti-MSLN Nanobody, CD8 hinge region, CD28 transmembrane region, CD28 costimulatory domain, and CD3$\zeta$ intracellular signaling domain in sequence from N-terminal to C-terminal.

In one or more embodiments, the amino acid sequence of chimeric antigen receptor is as shown in SEQ ID NO: 121 or 122 or 123.

The present description also provides a chimeric antigen receptor whose antigen-binding domain comprises an anti-mesothelin single domain antibody.

In one or more embodiments, the anti-mesothelin single domain antibody can be as described in any of the above embodiments.

The present description also provides a nucleic acid molecule comprising sequences selected from the following:

    (1) the sequence encoding the chimeric antigen receptor described in any embodiment herein;
    (2) a complementary sequence of (1);
    (3) a 5-50 bp fragment of any sequence of (1) or (2).

In one or more embodiments, the fragment is primer.

In one or more embodiments, the encoding sequence is DNA or RNA.

The second aspect of the present description also provides a nucleic acid construct comprising the nucleic acid molecule described in any of the embodiments herein.

In one or more embodiments, the nucleic acid construct further comprises a coding sequence of a PD-1 binding molecule.

In one or more embodiments, the nucleic acid construct further comprises a coding sequence of a transposase and/or a coding sequence of a PD-1 binding molecule. The transposase is used to transpose the sequence encoding the chimeric antigen receptor and/or the PD-1 binding molecule. For example, the encoding sequence or the expression cassette of the transposable chimeric antigen receptor and/or the PD-1 binding molecule is integrated into the genome.

In one or more embodiments, the sequence encoding the transposase is DNA or RNA.

In one or more embodiments, the sequence encoding the PD-1 binding molecule is DNA or RNA.

In one or more embodiments, the PD-1 binding molecule comprises: PD-1 antibodies or antigen-binding fragments thereof, PD-1 heavy chain antibodies or antigen-binding fragments thereof, monovalent or multivalent single domain antibodies, and multispecific single domain antibodies comprising one, two or more of the PD-1 single domain antibodies.

In one or more embodiments, the PD-1 binding molecule (e.g., PD-1 single domain antibody) also comprises an Fc region. Preferably, the Fc region is that of IgG4, the sequence of which is shown in SEQ ID NO: 133.

In one or more embodiments, the nucleic acid construct is a cloning vector, an expression vector, or an integration vector.

In one or more embodiments, the expression vector is a constitutive expression vector. Preferably, the constitutive expression vector is a transposable vector.

In one or more embodiments, the sequence coding the chimeric antigen receptor is located between the recognition sequences of the transposase.

In one or more embodiments, the sequence coding the PD-1 binding molecule is located between the recognition sequences of the transposase.

In one or more embodiments, the transposase is PiggyBac transposase (PB enzyme).

In one or more embodiments, the transposase is as described in CN105154473A.

In one or more embodiments, the nucleic acid construct comprises an expression cassette of a CAR. In one or more embodiments, the nucleic acid construct also comprises an expression cassette of the transposase and/or an expression cassette of the PD-1 binding molecule. The one, two or three expression cassettes are contained in one or more vectors.

In one or more embodiments, the nucleic acid construct comprises an expression cassette of the chimeric antigen receptor and an expression cassette of the transposase. Alternatively, the nucleic acid construct is an expression cassette, wherein a coding sequence of the chimeric antigen receptor and a coding sequence of the transposase are within the expression cassette.

In one or more embodiments, the nucleic acid construct comprises an expression cassette of the chimeric antigen receptor and an expression cassette of the PD-1 binding molecule. Alternatively, the nucleic acid construct is an expression cassette, wherein a coding sequence of the chimeric antigen receptor and a coding sequence of the PD-1 binding molecule are within the expression cassette.

In one or more embodiments, the nucleic acid construct comprises an expression cassette of the chimeric antigen receptor, an expression cassette of the transposase and an expression cassette of the PD-1 binding molecule. Alternatively, the nucleic acid construct is two expression cassettes, one comprises any one of the sequences encoding the chimeric antigen receptor, the sequence encoding the transposase, and the sequence encoding the PD-1 binding molecule, and the other of which comprises other coding sequences. Alternatively, the nucleic acid construct is an expression cassette, wherein the sequence encoding the chimeric antigen receptor, the sequence encoding the transposase, and the sequence encoding the PD-1 binding molecule are within the expression cassette.

In one or more embodiments, the nucleic acid construct is one or more vectors. Each vector comprises one or two or three expression cassettes as described in any embodiment herein.

In one or more embodiments, the nucleic acid construct comprises:

one vector, comprising (a) an expression cassette of a CAR and optional (b) an expression cassette of the transposase and/or an expression cassette of the PD-1 binding molecule.

two vectors, wherein, the two vectors (a) respectively comprising an expression cassette of the CAR and an expression cassette of the PD-1 binding molecule, and optionally, either one of the two vectors also comprising an expression cassette of the transposase; or (b) one vector of the two comprising an expression cassette of the transposase, and the other comprising an expression cassette of the CAR and an expression cassette of the PD-1 binding molecule.

three vectors, respectively comprising an expression cassette of the CAR, an expression cassette of the PD-1 binding molecule, and an expression cassette of the transposase.

Another aspect of the present description also provides a combination of coding sequences, comprising a sequence encoding the chimeric antigen receptor according to any one of claims 1 to 4, and also comprising a coding sequence of the transposase and/or a coding sequence of the PD-1 binding molecule.

In one or more embodiments, any sequence of the combination of the encoding sequences can be freely selected to be DNA or RNA. For example, three coding sequences are all DNA, or three coding sequences are all RNA, or any one or two of the three are DNA.

In one or more embodiments, the combination of the encoding sequences comprises a sequence encoding the chimeric antigen receptor, a sequence encoding the transposase and a sequence encoding the PD-1 binding molecule, and all three coding sequences are DNA.

In one or more embodiments, the combination of the encoding sequences comprises a sequence encoding the chimeric antigen receptor, a sequence encoding the transposase and a sequence encoding the PD-1 binding molecule, and the sequence encoding the chimeric antigen receptor and the sequence encoding the PD-1 binding molecule are DNA, and the sequence encoding the transposase is RNA, and more preferably, the sequence encoding the transposase is mRNA.

In one or more embodiments, in the combination of encoding sequences, an encoding sequences can be located in the same nucleic acid molecule or in different nucleic acid molecules. For example, the sequence encoding the chimeric antigen receptor, the sequence encoding the transposase, and the sequence encoding the PD-1 binding molecule are located in the same nucleic acid molecule. Alternatively, the sequence encoding the chimeric antigen receptor and the sequence encoding the transposase are located in the same nucleic acid molecule, while the sequence encoding the PD-1 binding molecule is located in another nucleic acid molecule. Alternatively, the sequence encoding the chimeric antigen receptor, the sequence encoding the transposase, and the sequence encoding the PD-1 binding molecule are respectively located in three nucleic acid molecules.

Another aspect of the present description provides a host cell, the host cell:

(1) comprising and/or expressing the chimeric antigen receptor described in any embodiment herein, and/or optionally comprising, expressing or secreting a PD-1 binding molecule, and/or
(2) comprising the nucleic acid molecule and/or the nucleic acid construct described herein, and/or
(3) comprising the combination of the encoding sequences described herein.

The transposase and the PD-1 binding molecule are described in the second aspect herein.

In one or more embodiments, the cell is an immune cell, preferably a T cell.

In one or more embodiments, the cell comprises: (a) the sequence encoding the chimeric antigen receptor described in any embodiment herein, and optional comprises (b) the sequence encoding the PD-1 binding molecule described in any embodiment herein.

In one or more embodiments, the cell comprises: the sequence encoding the chimeric antigen receptor described in any embodiment herein, and the sequence encoding the PD-1 binding molecule described in any embodiment herein.

Another aspect of the present description provides a pharmaceutical composition, comprising pharmaceutically acceptable excipients and:

(1) the chimeric antigen receptor and/or the sequences encoding thereof described in any embodiment herein, and optionally comprising (i) the transposase and/or the sequences encoding thereof, and/or (ii) the PD-1 binding molecule and/or the sequences encoding thereof, or
(2) the nucleic acid molecule, the nucleic acid construct, the combination of the encoding sequences or the cells described in any embodiment herein.

The transposase and the PD-1 binding molecule are described in the second aspect herein.

In one or more embodiments, the pharmaceutical composition is used for treating cancer.

In one or more embodiments, the cancer comprises mesothelin-mediated cancers. Preferably, the cancer also comprises PD-1-mediated cancers.

In one or more embodiments, the cancer comprises one or more selected from the group consisting of: mesothelioma, pancreatic cancer, ovarian cancer, cervical cancer, endometrial cancer, rectal cancer, esophageal cancer, lung adenocarcinoma, gastric cancer, melanoma, lung cancer, head and neck cancer, renal cell carcinoma, urothelial cancer, and non-Hodgkin lymphoma.

Another aspect of the present description provides the use of a reagent for preparing activated immune cells (such as T cells), the reagent comprising:

(1) the chimeric antigen receptor and/or the sequences encoding thereof described in any embodiment herein, and optionally comprising (i) the transposase and/or the sequences encoding thereof, and/or (ii) the PD-1 binding molecule and/or the sequences encoding thereof, or
(2) the nucleic acid molecule, the nucleic acid construct, the combination of the coding sequences or the cells described in any embodiment herein.

The transposase and the PD-1 binding molecule are described in the second aspect herein.

Another aspect of the present description provides the use of a reagent for preparing a drug for treating cancer, the reagent comprising:

(1) the chimeric antigen receptor and/or the sequences encoding thereof described in any embodiment herein, and optionally comprising (i) the transposase and/or the sequences encoding thereof, and/or (ii) the PD-1 binding molecule and/or the sequences encoding thereof, or

(2) the nucleic acid molecule, the nucleic acid construct, the combination of the coding sequences or the cells described in any embodiment herein.

The transposase and the PD-1 binding molecule are described in the second aspect herein.

In one or more embodiments, the cancer comprises mesothelin-mediated cancers. Preferably, the cancer also comprises PD-1-mediated cancers.

In one or more embodiments, the cancer comprises one or more selected from the group consisting of: mesothelioma, pancreatic cancer, ovarian cancer, cervical cancer, endometrial cancer, rectal cancer, esophageal cancer, lung adenocarcinoma, gastric cancer, melanoma, lung cancer, head and neck cancer, renal cell carcinoma, urothelial cancer, and non-Hodgkin lymphoma.

The present description also provides a method of treating or preventing cancer, the method comprising administering to a patient in need a therapeutically effective amount of the cells or the pharmaceutical composition according to any embodiment of the present invention.

In one or more embodiments, the cancer comprises mesothelin-mediated cancers. Preferably, the cancer also comprises PD-1-mediated cancers.

In one or more embodiments, the cancer is mesothelioma, pancreatic cancer, ovarian cancer, cervical cancer, endometrial cancer, rectal cancer, esophageal cancer, lung adenocarcinoma, gastric cancer, melanoma, lung cancer, head and neck cancer, renal cell carcinoma, urothelial cancer, non-Hodgkin lymphoma, etc.

In one or more embodiments, the therapeutically effective dose is $1\text{-}100*10^6$ cells/mouse, preferably $5*10^6$ cells/mouse.

In one or more embodiments, the cells or the pharmaceutical compositions are administered via intravenous injection.

BRIEF DESCRIPTION OF DRAWINGS

Figure 1: The proliferation curve and proliferation multiple of MSLN CAR-T cells from D5 to D13. Annotations are shown in Figure 1, D. A-C: The proliferation curve of CAR-T from D5 to D13 prepared from peripheral blood of three volunteers (AC-201908B, AC-201909A, AC-201910A); D: The average proliferation curve of CAR-T from D5 to D13 prepared from the peripheral blood of the above three volunteers; E: The average proliferation multiple curve of CAR-T from D5 to D13 prepared from the peripheral blood of the above three volunteers.

Figure 2: The cell viability rate curve of MSLN CAR-T cultured from D5 to D13.

Figure 3: Changes of the number of MSLN CAR positive T cells and CAR positive rates on D13. A: The number of MSLN CAR positive T cells; B: CAR positivity rate.

Figure 4: The ratio of CD4+T cells and CD8+T cells among CD3+ cells detected on D13

Figure 5: The proportion of memory T cells in the Day13 cell population.

Figure 6: Statistical results of in vitro killing of different mesothelin-negative (A: H520) and positive (B: H226; C: ASPC-1) tumor cells by cryopreserved and revived MSLN CAR-T in the preliminary experiment.

Figure 7: The killing rate of mock-T and MSLN CAR-T against two Mesothelin-positive tumor cells at 72h (E:T=1:1). A: SKOV3; B: H226.

Figure 8: The release of cytokine after mock-T and different MSLN CAR-T were co-incubated with mesothelin-positive tumor cells for 72 hours. A: IFN-gamma; B: IL-2; C: TNF-alpha.

Figure 9: The cell proliferation curve and the proliferation fold from D5 to D13. A-C: The proliferation curve of CAR-T from D5 to D13 prepared from peripheral blood of three volunteers (AC-201906B, AC-201908A, AC-201909A); D: The average proliferation curve of CAR-T from D5 to D13 prepared from the peripheral blood of the above three volunteers; E: The average proliferation fold curve of CAR-T from D5 to D13 prepared from the peripheral blood of the above three volunteers. Annotations are shown in Figure 9, D.

Figure 10: The cell viability rate of cells cultured from D5 to D13.

Figure 11: Changes of the number of MSLN CAR positive T cells and CAR positive rates on Day 13. A: The number of MSLN CAR positive T cells; B: CAR positivity rate.

Figure 12: The ratio of CD4+T cells and CD8+T cells among CD3+ cells detected on D13.

Figure 13: The proportion of memory T cells in the Day13 cell population.

Figure 14: Changes in the proportion of two types of memory T cells before and after an antigen stimulation.

Figure 15: Comparison of antibody concentrations secreted by cells before and after an antigen stimulation.

Figure 16: The killing rates of mock-T and CAR-T against SKOV3-PDL1 under different efficacy-to-target ratios at 48h.

Figure 17: The killing rates of mock-T and CAR-T against H226 under different efficacy-to-target ratios at 48h.

Figure 18: The killing curve of mock-T and MSLN CAR-T against tumor cells. A: Statistics on the killing rate of CAR-T cells against H226 and SKOV-PDL1 cells (E:T=1:4, killing time=72h); B: The representative killing curve for H226 cells; C: The representative killing curve for SKOV3-PDL1 cells.

Figure 19: The release of cytokines after mock-T and MSLN CAR-T co-incubated with mesothelin&PDL1-positive tumor cells for 48 hours. A: IL-2; B: IFN-gamma; C: TNF-alpha.

Figure 20: Changes in NCI-H226-PDL1 tumor volume at high, medium and low doses.

Figure 21: Changes in Skov3-PDL1 tumor volume at high, medium and low doses.

Figure 22: Body weight changes of NCI-H226-PDL1 tumor-bearing mice at high, medium and low doses.

Figure 23: Body weight changes of Skov3-PDL1 tumor-bearing mice at high, medium and low doses.

Figure 24: The survival curve of NCI-H226-PDL1 tumor-bearing mice at high, medium and low doses.

Figure 25: The survival curve of Skov3-PDL1 tumor-bearing mice at high, medium and low doses.

Figure 26: Changes of plasma concentrations of the PD1 antibody in NCI-H226-PDL1 tumor-bearing mice at high, medium and low doses. A, B: Low doses; C, D: Medium doses; E, F: High doses.

Figure 27: Changes of plasma concentrations of the PD1 antibody in Skov3-PDL1 tumor-bearing mice at high, medium and low doses. A, B: Low doses; C, D: Medium doses; E, F: High doses.

Figure 28: Changes in NCI-H226-PDL1 tumor volume at high doses.

Figure 29: Body weight changes of NCI-H226-PDL1 tumor-bearing mice at high doses.

Figure 30: The survival curve of NCI-H226-PDL1 tumor-bearing mice at high doses.

Figure 31: Changes of plasma concentrations of the PD1 antibody in NCI-H226-PDL1 tumor-bearing mice at high doses (A: mass concentration, B: molality).

Figure 32: Changes of plasma concentrations of CAR-T (A: CD3+T cells, B: CD8+T cells, C: CAR+T cells, D: the ratio of CAR+T cells in CD3+T cells) per microliter of blood in NCI-H226-PDL 1 tumor-bearing mice at high doses.

Figure 33: Changes of peripheral blood cytokine concentrations of NCI-H226-PDL1 tumor-bearing mice at high doses. A: IL-2; B: IL-4; C: TNF-alpha; D: IL-6; E: IFN-gamma; F: IL-10.

Figure 34: Changes of the tumor volume and the body weight at conventional dosages (A and C: changes of the tumor volume and the body weight of NCI-H226-PDL1; B and D: changes of the tumor volume and the body weight of Skov3-PDL1).

Figure 35: CAR-T cell preparation data. A: Proliferation multiple; B: Cell viability rate; C: CAR positive rate; D: Tcm and Tern; E: CD4/8; F: Antibody secretion.

Figure 36: PD-1 antibody secretion and cytokine secretion by CAR-T cells. A: The amount of PD-1 antibody secreted by CAR-T cells after 6 hours and 48 hours of antigen stimulation; B: The number of cytokines secreted by CAR-T cells after 6 hours and 48 hours without antigen stimulation and with antigen stimulation.

Figure 37: The killing properties of CAR-T cells. A-B:The killing rate of Mock-T and CAR-T prepared from PBMC derived from two different volunteers (09B and 09A01) against H226-PDL1 tumor cells at 80 hours; C-D: The killing curve of Mock-T and CAR-T prepared from PBMC derived from two different volunteers (09B and 09A01) against H226-PDL1 tumor cells.

Figure 38: The killing properties of CAR-T cells. A-B: The killing rate of Mock-T and CAR-T prepared from PBMC derived from three different volunteers (09B, 09A01, 12A) against SKOV3-PDL1 tumor cells at 100 hours; C-D: The killing curve of Mock-T and CAR-T prepared from PBMC derived from two different volunteers (09B and 09A01) against H226-PDL1 tumor cells.

Figure 39: Schematic diagram of the experimental procedure in mice.

Figure 40: The inhibitory effect of different doses of CAR-T on tumors. A-D represent the results of doses of 0.2E6, 1E6, 5E6, and 20E6, respectively.

Figure 41: The body weight and the survival rate of mice at different doses. A-D represent the body weight of mice at doses of 0.2E6, 1E6, 5E6, and 20E6, respectively; E: The survival rate of mice at doses of 20E6.

Figure 42: Peripheral blood CD3+ T cell numbers at different doses.

Figure 43: The proportion of CAR-positive cells. A: The ratio of peripheral blood CAR+T cells to CD3+T cells at different doses after 14 days of CAR-T reinfusion. B: CAR+ proportion of cells in each group before reinfusion.

Figure 44: pNB338B-1444-CAR vector structure diagram.

Figure 45: Changes of plasma concentrations of the PD1 antibody at conventional doses (A: NCI-H226-PDL1, B: Skov3-PDL1).

Figure 46: Changes of plasma concentration of CAR-T (A-D: NCI-H226-PDL1, E-H: Skov3-PDL1) at conventional doses.

DETAILED DESCRIPTION

The present description provides a mesothelin-targeted chimeric antigen receptor (CAR). The CAR comprises optional signal peptide sequences, mesothelin-binding molecules containing anti-mesothelin single domain antibody sequences, hinge regions, transmembrane regions, intracellular costimulatory domains and intracellular signaling domains.

"Binding molecule" of an antigen as used herein is a protein that specifically binds antigen, including but not limited to antibodies, antigen binding fragments of antibodies, heavy chain antibodies, nanoantibodies, micro antibodies, affibodies, target binding regions of receptors, cell adhesion molecules, ligands, enzymes, cytokines, and chemokines. Term "antibody" as used herein includes monoclonal antibodies (including full-length antibodies with immunoglobulin Fc region), antibody compositions with multi epitope specificity, multi-specific antibodies (e.g., bispecific antibodies), double antibodies and single chain molecules, and antibody fragments, especially antigen binding fragments, such as Fab, F(ab')2, and FV). Terms "immunoglobulin" (Ig) and "antibody" are used interchangeably herein.

"Heavy chain antibody" described herein is an antibody derived from Camelidae or sharks. Compared with the above 4-chain antibody, the heavy chain antibody lacks light chain and heavy chain constant region 1 (CH1), and only contains two heavy chains composed of variable region (VHH) and other constant regions, wherein the variable region is connected to the constant region through a hinge region like structure. Each heavy chain of camelid heavy chain antibody contains one variable region (VHH) and two constant regions (CH2 and CH3), and each heavy chain of shark heavy chain antibody contains one variable region and five constant regions (CH1-CH5). Antigen binding fragments of heavy chain antibodies include VHH and single chain heavy chain antibodies. Heavy chain antibodies may have CH2 and CH3 of human IgG Fc by fusing with the constant region of human IgG Fc. Exemplarily, the heavy chain constant region comprises the sequence as shown in SEQ ID NO: 132.

As used herein, the terms "single domain antibody", "heavy chain variable region domain of heavy chain antibody", "VHH" "Nanobody" and "single variable domain" can be used interchangeably, and all refer to single-domain polypeptides or proteins that specifically recognize and bind to antigens. Single domain antibodies are variable regions of heavy chain antibodies. Typically, single domain antibodies contain three CDRs and four FRs. Single domain antibodies are the smallest functional antigen binding fragments. Generally, after obtaining an antibody with natural deletion of light chain and heavy chain constant region 1 (CH1), the variable region of antibody heavy chain is cloned to construct a single domain antibody consisting of only one heavy chain variable region.

Binding molecules comprising two or more single domain antibodies are multivalent single domain antibodies; and binding molecules comprising two or more single domain antibodies with different specificities are multispecific single domain antibodies. Multivalent single domain antibodies or multispecific single domain antibodies are connected to multiple single domain antibodies through linkers. The linker usually consists of 1-15 amino acids selected from G and S.

Heavy chain antibodies and antibodies herein are distinguished by different combinations of antibodies. Due to the similarity of their structures, the following structural descriptions for antibodies also apply to heavy chain antibodies except for light chains.

The "variable region" or "variable domain" of an antibody refers to the amino terminal domain of the heavy or light chain of the antibody. The variable domains of the heavy and light chains can be referred to as "VH" and "VL", respectively. These domains are usually the most variable parts of the antibodies (relative to other antibodies of the same type) and contain the antigen binding sites.

The term "variable" refers to cases in which certain segments of the variable domain differ widely in antibody sequences. Variable domains mediate antigen binding and define the specificity of a specific antibody to its specific antigen. However, variability is not evenly distributed across all amino acids spanned by the variable domain. On the contrary, it is concentrated in three segments called hypervariable region (HVR) (both in the light chain and the heavy chain variable domains), namely HCDR1, HCDR2 and HCDR3 of heavy chain variable region (CDR1, CDR2 and CDR3 in heavy chain antibodies for short) and LCDR1, LCDR2 and LCDR3 of light chain variable region. The more highly conserved part of the variable domain is called the framework regions (FR). The variable domains of natural heavy and light chains each comprise four FR regions (FR1, FR2, FR3, and FR4), most of which adopt beta-sheet conformation, connected by three HVRs through the formation of ring links and in some cases part of the beta-sheet structure. The HVR in each chain is hold together by the FR region in close proximity, and contributes to the formation of the antigen binding site of the antibody together with the HVR of the other chain. Generally, the structure of the light chain variable region is FR1-LCDR1-FR2-LCDR2-FR3-LCDR3-FR4, and the structure of the heavy chain variable region is FR1-HCDR1-FR2-HCDR2-FR3-HCDR3-FR4. The constant domain is not directly involved in the binding of antibody to antigen, but exhibits a variety of effector functions, such as the involvement of antibody in antibody dependent cell-mediated toxicity.

"Fc region" ( crystallizable fragment region) or "Fc domain" or "Fc" refers to the C-terminal region of the antibody heavy chain , which mediates the binding of immunoglobulins to host tissues or factors, including binding to Fc receptors on various cells (e.g., effector cells) of the immune system, or binding to the first component (Clq) of the classical complement system. In IgG, IgA and IgD antibody isotypes, the Fc region is composed of two identical protein fragments from the CH2 domain and CH3 domain of two heavy chains of the antibody; the Fc regions of IgM and IgE contain three heavy chain constant domains (CH domains 2-4) in each polypeptide chain. Although the boundary of the Fc region of an immunoglobulin heavy chain may vary, the Fc region of human IgG heavy chain is usually defined as a sequence segment from the amino acid residue of heavy chain position C226 or P230 to the carboxyl terminus, which is numbered according to the EU index, as in Kabat. Each heavy chain of camelid heavy chain antibody comprises one variable region (VHH) and two constant regions (CH2 and CH3). Heavy chain antibodies may have CH2 and CH3 of human IgG Fc by fusing with the constant region of human IgG Fc.

"antibody fragment" comprises a part of the complete antibody, preferably the antigen binding region and/or variable region of the complete antibody. The antibody fragment is preferably an antigen binding fragment of the antibody. Examples of antibody fragments include Fab, Fab', F(ab')2 and Fv fragments; diabody; linear antibody; single chain antibody molecule, scFv-Fc fragment; multispecific antibody formed from an antibody fragment; and any fragment that should be able to increase the half-life by chemical modification or by incorporation into liposomes. Digestion of antibody with papain produces two identical antigen binding fragments called "Fab" fragments, and a residual "Fc" fragment, whose name reflects its ability to crystallize easily. The Fab fragment consists of a complete light chain and heavy chain variable domain (VH) and the first constant domain (CH1) of a heavy chain. Each Fab fragment is monovalent in terms of antigen binding, that is, it has a single antigen binding site . Pepsin treated antibody produces a larger F(ab')2 fragment, which is roughly equivalent to two Fab fragments connected by disulfide bonds, with different antigen binding activities and still able to cross link antigens. Fab' fragment is different from Fab fragment due to the addition of some additional residues (including one or more cysteines from the antibody hinge region) at the carboxyl terminus of the CH1 domain . F(ab')2 antibody fragment was initially generated as a pair of Fab' fragments with a hinge cysteine between the Fab' fragments . Other chemical conjugations of antibody fragments are also known. The Fc fragment comprises the carboxyl terminal portion of two heavy chains held together by disulfide bonds. The effector function of an antibody is determined by the sequence in the Fc region, which is also recognized by Fc receptors (FcRs) found on some types of cells. Antigen binding fragments of heavy chain antibodies comprise VHH and single chain heavy chain antibodies.

Antibodies also include "chimeric" antibodies herein, wherein a part of the heavy and/or light chain is the same or homologous with the corresponding sequence in antibodies derived from a specific species or belonging to a specific antibody class or subclass, while the remaining part of the chain is the same or homologous with the corresponding sequence in antibodies derived from another species or belonging to another antibody class or subclass, as well as fragments of such antibodies, as long as they exhibit the desired biological activity.

The "Humanized" form of non-human (e.g., murine) antibody refers to chimeric antibodies that contain, at a minimum, sequences derived from non-human immunoglobulins . Therefore, "humanized antibody" usually refers to a non-human antibody whose variable domain framework region exchanges with the sequence found in a human antibody . Usually in a humanized antibody, the entire antibody (except CDRs) is encoded by or identical to a human derived polynucleotide (except CDRs). CDRs (some or all of which are encoded by nucleic acids derived from non-human objects) are transplanted into the human antibody variable region β-sheet in the backbone to produce an antibody, and its specificity is determined by the transplanted CDRs. The production methods of such antibodies are well known in the art, such as using mice with genetically engineered immune systems.

A "human antibody" means an antibody having an amino acid sequence corresponding to the amino acid sequence of an antibody generated by a human and/or produced using any of the techniques disclosed herein for generating human antibodies. This definition of human antibodies clearly excludes a humanized antibody that contain non-human antigen binding residues. Human antibodies can be generated using a variety of techniques known in the art, including phage display libraries. The binding molecule described in any embodiment of the present description is a chimeric antibody or a fully human antibody; preferably, a fully human antibody.

The term "immune cells" refers to cells that participate in or are related to immune responses, examples of which include but are not limited to lymphocytes, dendritic cells, monocytes/macrophages, granulocytes, mast cells, etc. Preferably, immune cells are, for example T cells, NK cells, NKT cells, tumor-infiltrating lymphocytes and macrophages. In certain embodiments, the immune cells as used herein include T cells, for example CD4+T cells and/or CD8+T cells.

"Mesothelin binding molecule" as used herein comprises an anti-mesothelin single domain antibody, wherein the complementarity determining region CDR of the single domain antibody comprises CDR1, CDR2 and CDR3, and CDR1 comprises the sequence shown in SEQ ID NO: 1, CDR2 comprises the sequence shown in SEQ ID NO: 2, and CDR3 comprises the sequence shown in SEQ ID NO: 3.

In one or more embodiments, SEQ ID NO: 1 is $X_1X_2X_3X_4X_5X_6X_7X_8X_9X_{10}X_{11}$, wherein $X_1$ is G, E, T or A, $X_2$ is S, N, P, A, H, D, K, I or F, $X_3$ is I, S, V, T, D, L, A, M or H, $X_4$ is F, S, I, A, L or G, $X_5$ is N, S, G, A, D, T, E, R or H, $X_6$ is I, L, F, Y, E or N, $X_7$ is N, A, G, D, K, Y, L or S, $X_8$ is A, Y, V, D or N, $X_9$ is E or null, $X_{10}$ is F or null, $X_{11}$ is A or null. In one or more embodiments, SEQ ID NO: 1 is $X_1X_2X_3X_4X_5X_6X_7X_8$, wherein $X_1$ is G, E, T or A, $X_2$ is S, N, P, A, H, D, K,

I or F, $X_3$ is I, S, V, T, D, L, A, M or H, $X_4$ is F, S, I, A, L or G, $X_5$ is N, S, G, A, D, T, E, or H, $X_6$ is I, L, F, Y or N, $X_7$ is N, A, G, D, K, Y or S, $X_8$ is A, Y, V or N. In one or more embodiments, SEQ ID NO: 1 is $GX_2X_3X_4X_5X_6X_7A$, wherein $X_2$ is S, N, A, D, I or F, $X_3$ is I, V, T, D, A, L or S, $X_4$ is F, S, I, A or L, $X_5$ is N, S, A, D, E, T or H, $X_6$ is I, F, N or Y, $X_7$ is N, G, D or Y. Preferably, SEQ ID NO: 1 is $X_1X_2X_3X_4X_5X_6X_7X_8$, wherein $X_1$ is G, E, T or A, $X_2$ is S, N, P, H or I, $X_3$ is I, S, T, D, L or A, $X_4$ is F, I or L, $X_5$ is S, T or E, $X_6$ is I, F or Y, $X_7$ is N, A, D or Y, $X_8$ is A or Y.

In one or more embodiments, the CDR1 of the anti-mesothelin single domain antibody comprises the sequence shown in any one of SEQ ID NO: 4-27, 73. Preferably, CDR1 comprises the sequence shown in any one of SEQ ID NOs: 5, 11, 14, 15, 17, 21, 27, 73.

In one or more embodiments, SEQ ID NO: 2 is $X_1X_2X_3X_4X_5X_6X_7X_8X_9X_{10}X_{11}X_{12}$, wherein $X_1$ is I, M, A, T or L, $X_2$ is S, G, N, D, T or V, $X_3$ is S, N, A, R, G or T, $X_4$ is S, T, G, D or N, $X_5$ is N, G, T or I, $X_6$ is S, R, N, D, K, T or G, $X_7$ is D, T, S, I or K, $X_8$ is N, T, G, D or null, $X_9$ is T, K, G or null, $X_{10}$ is G, R or null, $X_{11}$ is V, T or null, $X_{12}$ is T or null. In one or more embodiments, $X_3$ is S, N, A, R or T. In one or more embodiments, SEQ ID NO: 2 is $IX_2X_3X_4X_5X_6X_7X_8X_9$, wherein $X_2$ is S, G, N or D, $X_3$ is S, N, A or T, $X_4$ is S, T, G, D or N, $X_5$ is N or G, $X_6$ is S, R, N, D or K, $X_7$ is D, T, S or K, $X_8$ is N, T or null, $X_9$ is T, K or null. Preferably, SEQ ID NO: 2 is $X_1X_2X_3X_4X_5X_6X_7X_8X_9X_{10}X_{11}X_{12}$, wherein $X_1$ is I, A or T, $X_2$ is S, G, N or T, $X_3$ is S or R, $X_4$ is T, G or D, $X_5$ is N, G or T, $X_6$ is S, R, N, K or T, $X_7$ is D, T, S, I or K, $X_8$ is N, G or null, $X_9$ is T, G or null, $X_{10}$ is G or null, $X_{11}$ is V or null, $X_{12}$ is T or null.

In one or more embodiments, the CDR2 of the anti-mesothelin single domain antibody comprises the sequence shown in any one of SEQ ID NOs: 28-47, 74. Preferably, CDR2 comprises the sequence shown in any one of SEQ ID NOs: 29, 35, 37, 38, 39, 43, 40, 74.

In one or more embodiments, SEQ ID NO: 3 is $X_1X_2X_3X_4X_5X_6X_7X_8X_9X_{10}X_{11}X_{12}X_{13}X_{14}X_{15}X_{16}X_{17}X_{18}X_{19}X_{20}X_{21}X_{22}X_{23}$, wherein $X_1$ is N, H, A or Q, $X_2$ is L, A, G or V, $X_3$ is S, R, E, G, D or T, $X_4$ is N, A, R, G, D, K, V, T, I, E or Q, $X_5$ is Y, F, K, S, C, I, D, G, H or R, $X_6$ is D, A, V, G, R, N, P, T, C, Q or S, $X_7$ is R, Y, T, A, D, K, L, E, S or G, $X_8$ is K, S, F, T, D, Q, E, G, P, R, Y or N, $X_9$ is D, G, I, H, S, Y, Q, K, T, R or V, $X_{10}$ is R, Y, H, N, T, D, P, V, K, C, L or S, $X_{11}$ is Y, D, E, P, F, S, L, I or null, $X_{12}$ is P, V, Q, A, Y, F, N, D, R or null, $X_{13}$ is D, A, Y, V, Q, P or null, $X_{14}$ is Y, L, P, A, E or null, $X_{15}$ is C, S, M, P, T or null, $X_{15}$ is C, S, M, P or null, $X_{16}$ is V, D, S, Y or null, $X_{17}$ is L, F, V, D or null, $X_{18}$ is R, G, M, S or null, $X_{19}$ is D, N, S or null, $X_{20}$ is Y, L or null, $X_{21}$ is Y or null, $X_{22}$ is A or null, $X_{23}$ is D or null. In one or more embodiments, $X_1$ is N, H, A or Q, $X_2$ is L, A, G or V, $X_3$ is S, R, E, G, D or T, $X_4$ is N, A, R, G, D, K, V, T, I or Q, $X_5$ is Y, F, K, S, C, I, D, G, H or R, $X_6$ is D, A, V, G, R, N, P, T, C, Q or S, $X_7$ is R, Y, T, A, D, K, L, E, S or G, $X_8$ is K, S, F, T, D, Q, E, G, P, R, Y or N, $X_9$ is D, G, I, H, S, Y, Q, K, T or V, $X_{10}$ is R, Y, H, N, T, D, P, V, K, C or S, $X_{11}$ is Y, D, E, P, F, S, L or null, $X_{12}$ is P, V, Q, A, Y, F, N, D or null, $X_{13}$ is D, A, Y, V, Q or null, $X_{14}$ is Y, L, P, A, E or null, $X_{15}$ is C, S, M, P or null, $X_{16}$ is V, D, S, Y or null, $X_{17}$ is L, F, V, D or null, $X_{18}$ is R, G, M, S or null, $X_{19}$ is D, N, S or null, $X_{20}$ is Y, L or null, $X_{21}$ is Y or null, $X_{22}$ is A or null, $X_{23}$ is D or null. In one or more embodiments, SEQ ID NO: 3 is $AX_2X_3X_4X_5X_6X_7X_8X_9X_{10}X_{11}X_{12}X_{13}X_{14}X_{15}X_{16}X_{17}X_{18}X_{19}$, wherein $X_2$ is A, G or V, $X_3$ is S, E, G, D or T, $X_4$ is N, R, D, K, T or I, $X_5$ is Y, F, K, I, D, G, H or R, $X_6$ is D, A, G, N, P, T, C, Q or S, $X_7$ is R, Y, T, A, D, K, L or G, $X_8$ is K, S, F, D, Q, G, P, Y or N, $X_9$ is D, G, I, S, Y, Q, K or V, $X_{10}$ is R, Y, H, T, D, P, V, K or C, $X_{11}$ is Y, D, E, F, S, L or null, $X_{12}$ is P, V, A, Y, F, N, D or null, $X_{13}$ is D, A, Y, V or null, $X_{14}$ is Y, P, E or null, $X_{15}$ is S, P or null, $X_{16}$ is D, Y or null, $X_{17}$ is F, D or null, $X_{18}$ is G, S or null, $X_{19}$ is N or null. Preferably, SEQ ID NO: 3 is $AX_2X_3X_4X_5X_6X_7X_8X_9X_{10}X_{11}X_{12}X_{13}X_{14}X_{15}X_{16}X_{17}X_{18}X_{19}$, wherein $X_2$ is A or V, $X_3$ is S, E, G or D, $X_4$ is N, R, D or I, $X_5$ is Y, F, K, D or H, $X_6$ is D, G, P, C or S, $X_7$ is R, Y, D, K or L, $X_8$ is K, Q, G or N, $X_9$ is D, S, Y or Q, $X_{10}$ is R, Y, D, V or S, $X_{11}$ is Y, D, F, S, L or null, $X_{12}$ is P, F or null, $X_{13}$ is D, V or null, $X_{14}$ is P or null, $X_{15}$ is S or null, $X_{16}$ is D or null, $X_{17}$ is F or null, $X_{18}$ is G or null, $X_{19}$ is N or null.

In one or more embodiments, the CDR3 of the anti-mesothelin single domain antibody comprises the sequence shown in any one of SEQ ID NOs: 48-72, 75. Preferably, CDR3 comprises the sequence shown in any one of SEQ ID NOs: 49, 55, 58, 59, 61, 65, 72, 75.

In one or more embodiments, CDR1 of the anti-mesothelin single domain antibody comprises the sequence shown in any one of SEQ ID NOs: 4-27 and 73, CDR2 comprises the sequence shown in any one of SEQ ID NOs: 28-47 and 74, and CDR3 comprises the sequence shown in any one of SEQ ID NOs:48-72 and 75. Preferably, the anti-mesothelin single domain antibody contains CDR1, CDR2 and CDR3 shown in any of the following groups a1 to a26:

| Group | CDR1 | CDR2 | CDR3 |
|-------|------|------|------|
| a1 | 4 | 28 | 48 |
| a2 | 5 | 29 | 49 |
| a3 | 6 | 30 | 50 |
| a4 | 7 | 31 | 51 |
| a5 | 8 | 32 | 52 |

(continued)

| Group | CDR1 | CDR2 | CDR3 |
|-------|------|------|------|
| a6 | 9 | 33 | 53 |
| a7 | 10 | 34 | 54 |
| a8 | 11 | 35 | 55 |
| a9 | 12 | 36 | 56 |
| a10 | 13 | 32 | 57 |
| a11 | 14 | 37 | 58 |
| a12 | 15 | 38 | 59 |
| a13 | 16 | 31 | 60 |
| a14 | 17 | 39 | 61 |
| a15 | 18 | 40 | 62 |
| a16 | 19 | 41 | 63 |
| a17 | 20 | 42 | 64 |
| a18 | 21 | 43 | 65 |
| a19 | 22 | 32 | 66 |
| a20 | 13 | 32 | 67 |
| a21 | 23 | 44 | 68 |
| a22 | 24 | 45 | 69 |
| a23 | 25 | 46 | 70 |
| a24 | 26 | 47 | 71 |
| a25 | 27 | 40 | 72 |
| a26 | 73 | 74 | 75 |

In one or more embodiments, the FR1 of the anti-mesothelin single domain antibody can be selected from the FR1 of VHH of each antibody numbering in Table A, the FR2 of VHH can be selected from the FR2 of VHH of each antibody numbering in Table 1, the FR3 of VHH can be selected from the FR3 of VHH of each antibody numbering in Table 1, and the FR4 of VHH can be selected from the FR4 of VHH of each antibody numbering in Table 1. In one or more embodiments, the FR region of the anti-mesothelin single domain antibody is the FR region of any VHH selected from SEQ ID NOs: 76-120.

In one or more embodiments, the anti-mesothelin single domain antibody is as shown in any one of SEQ ID NO: 76-120. Preferably, the single domain antibody is as shown in any one of SEQ ID NOs: 77, 83, 86, 87, 89, 93, 100, 103.

The mesothelin binding molecule described herein is a monovalent or multivalent single domain antibody, a multispecific single domain antibody, a heavy chain antibody or an antigen binding fragment thereof, an antibody or an antigen binding fragment thereof comprising one, two, or more of the anti-mesothelin single domain antibodies described herein. The multivalent single domain antibody or the multispecific single domain antibody connects a plurality of single domain antibodies through a linker. The linker consists of 1-15 amino acids selected from G and S. The heavy chain antibody is a camelid heavy chain antibody or a shark heavy chain antibody. The antigen binding fragment of the heavy chain antibody is a single chain heavy chain antibody. The heavy chain antibody further comprises a heavy chain constant region. In one or more embodiments, the heavy chain constant region is a constant region of camelid heavy chain antibody, comprising CH2 and CH3. In one or more embodiments, the CH2 and CH3 are CH2 and CH3 of human IgG Fc, such as CH2 and CH3 of IgG4.

The mesothelin-binding molecules and the anti-mesothelin single domain antibodies mentioned herein include all mesothelin-binding molecules and anti-mesothelin single domain antibodies described in CN202011584014 or CN202011582948. The entirety of the above patent description is incorporated herein in its each embodiment being specifically and individually by reference.

Optional signal peptides of CAR can be selected as desired. For example, CD8 signal peptide, CD28 signal peptide, CD4 signal peptide or light chain signal peptide, the sequences of which are within the knowledge of those skilled in the art. The CD8 signal peptide suitable for the present description can be various human CD8 signal peptide sequences commonly used for CAR in the art. In certain embodiments, the amino acid sequence of the human CD8 signal peptide can be as shown in amino acids of positions 1-22 of SEQ ID NO: 121.

The hinge region of the CAR is selected from the group consisting of: CD8α hinge region, IgD hinge region, IgG1 Fc CH2CH3 hinge region or IgG4 Fc CH2CH3 hinge region, and its sequence being within the knowledge of those skilled in the art. The CD8 hinge region suitable for the present description can be various human CD8 hinge region sequences commonly used for CAR in the art. In certain embodiments, the amino acid sequence of the human CD8 hinge region can be as shown in amino acids of positions 149-203 of SEQ ID NO: 121.

The transmembrane region of the CAR is selected from one of the CD28 transmembrane region, the CD8 transmembrane region, the CD3ζ transmembrane region, the CD134 transmembrane region, the CD137 transmembrane region, the ICOS transmembrane region and the DAP10 transmembrane region; preferably, the CD28 transmembrane region, and its sequence being within the knowledge of those skilled in the art. The CD8 transmembrane region suitable for the present description can be various human CD8 transmembrane region sequences commonly used for CAR in the art. In certain embodiments, the amino acid sequence of the human CD8 transmembrane region can be as shown in amino acids of positions 204-231 of SEQ ID NO: 121.

The intracellular costimulatory domain can be selected as needed, including an intracellular domain of a costimulatory signaling molecule, for example, the intracellular domains of CD28, CD134/OX40, CD137/4-1BB, lymphocyte-specific protein tyrosine kinase, inducible T cell co-stimulator (ICOS) and DNAX activating protein 10. Human CD28 costimulatory domains suitable for use in the present description can be various human CD28 costimulatory domain sequences commonly used for CARs in the art. In certain embodiments, the amino acid sequence of the CD28 costimulatory domain can be as shown in amino acids of positions 231-272 of SEQ ID NO: 121.

Similarly, intracellular signaling domains of the CAR can be selected as needed, including but not limited to the CD3ζ intracellular signaling domain or the FcεRIγ intracellular signaling domain. The CD3ζ intracellular signaling domain suitable for the present description can be various CD3ζ intracellular signaling domain commonly used for CAR in the art. In certain embodiments, the amino acid sequence of the CD3ζ intracellular signaling domain is as shown in the amino acid sequence of positions 273-384 of SEQ ID NO: 121.

The above-mentioned parts that form the chimeric antigen receptor of the present description, such as the CD8 signal peptide, the anti-MSLN Nanobody, the CD8 hinge region, the CD28 transmembrane region, the CD28 costimulatory domain, the CD3ζ intracellular signaling domain, etc., can be connected directly to each other or through linker sequences. The linker sequence may be one well known in the art and suitable for use with antibodies, such as a G-and-S-containing linker sequence. Typically, linkers contain one or more tandemly repeated motifs. For example, the motif can be GGGS, GGGGS, SSSSG, GSGSA and GGSGG. Preferably, the motifs are contiguous in linker sequences with no intervening amino acid residues between the repeats. Linker sequences can consist of 1, 2, 3, 4 or 5 repeat motifs. The length of the linker can be 3 to 25 amino acid residues, such as 3 to 15, 5 to 15, or 10 to 20 amino acid residues. In certain embodiments, the linker sequence is a polyglycine linker sequence. The number of glycines in the linker sequence is not particularly limited, usually 2 to 20, such as 2 to 15, 2 to 10, 2 to 8. In addition to glycine and serine, the linker may further contain other known amino acid residues, such as alanine (A), leucine (L), threonine (T), glutamic acid (E), phenylalanine (F), arginine (R), glutamine (Q), etc. As an example, the linker can consist of any of the amino acid sequences of SEQ ID NO: 7-18. In certain embodiments, the linker sequence is a (GGGGS)n linkage, where n is an integer from 1 to 5.

In an exemplary embodiment, the CAR comprises CD8 signal peptide, anti-MSLN Nanobody, CD8 hinge region, CD28 transmembrane region, CD28 costimulatory domain, and CD3ζ intracellular signaling domain in sequence from N-terminus to C-terminus. In specific embodiments, an exemplary CAR having the above structure is shown in any of SEQ ID NOs: 121-123. In certain embodiments, the amino acid sequence of the CAR of the present description is shown as amino acids of positions 23-384 of SEQ ID NO: 121, or amino acids of positions 23-377 of SEQ ID NO: 122, or amino acids of positions 23-378 of SEQ ID NO: 123.

It should be understood that in gene cloning operation, it is often necessary to design a suitable cleavage site, which is bound to introduce one or more incoherent residues at the ends of the expressed amino acid sequence, and this does not affect the activity of the sequence. Also for constructing fusion proteins, facilitating the expression of recombinant proteins, obtaining recombinant proteins that are automatically secreted outside the host cell, or facilitating the purification of recombinant proteins, it is often necessary to add some amino acids to the N-terminus, C-terminus, or other suitable regions within the recombinant protein, for example, including but not limited to, suitable linker peptides, signal peptides, leader peptides, terminal extensions, etc. Therefore, the amino terminus or carboxyl terminus of the CAR of the present description may also contain one or more polypeptide fragments as protein tags. Any suitable tag may be used herein. For example, the tags may be FLAG, HA, HA1, c-Myc, Poly-His, Poly-Arg, Strep-TagII, AU1, EE, T7, 4A6, ε, B, gE and Ty1. There tags can be used to purify proteins.

The present description also includes mutants of the CAR of any embodiment. These mutants include the amino acid sequence having at least 80%, preferably at least 85%, preferably at least 90%, preferably at least 95%, preferably at least 97% sequence identity with the CAR and retaining the biological activity of the CAR (such as activated T cells). The sequence identity between two aligned sequences can be calculated using, for example, NCBI's BLASTp.

These mutants further include the amino acid sequence having one or more mutations (insertions, deletions, or substitutions) in the amino acid sequence of the CAR of any embodiment while still retaining the biological activity of the CAR. The more mutations usually refer to within 1-10, such as 1-8, 1-5 or 1-3. Substitutions are preferably conservative substitutions. For example, in this field, conservative substitutions with amino acids with similar or identical properties usually do not change the function of the protein. The "amino acids with similar or identical properties" includes, for example, families of amino acid residues with similar side chains. These families comprise amino acids with basic side chains (e.g., lysine, arginine, histidine), amino acids with acidic side chains (e.g., aspartic acid, glutamic acid), amino acids with uncharged polar side chains (e.g., glycine, asparagine, glutamine, serine, threonine, tyrosine, cysteine), amino acids with non-polar side chains (e.g., alanine, valine, leucine, isoleucine proline, phenylalanine, methionine, tryptophan), amino acids with β-branched side chains (e.g., threonine, valine, isoleucine), and amino acids with aromatic side chains (e.g., tyrosine, phenylalanine, tryptophan, histidine). Therefore, substitutions of one or more positions in a polypeptide of the present description with another amino acid residue from the same type of side chain will not materially affect its activity.

The present description comprises polynucleotide sequences encoding the fusion proteins of the present description. The polynucleotide sequences of the present description can be in the form of DNAs or RNAs. DNAs include cDNAs, genomic DNAs, or synthetic DNAs. DNAs can be single stranded DNAs or double stranded DNAs. DNAs can be coding or noncoding strand DNAs. The present description also comprises degenerate variants of the polynucleotide sequence encoding the fusion protein, i.e., nucleotide sequences encoding the same amino acid sequence but with different nucleotide sequences.

The polynucleotide sequence described herein can generally be obtained by PCR amplification. Specifically, primers can be designed according to the nucleotide sequence disclosed herein, especially the open reading frame sequence, and a commercially available cDNA library or a cDNA library prepared by a conventional method known to those skilled in the art can be used as a template, and related sequences were amplified. When the sequence is long, it is often necessary to carry out two or more PCR amplifications, and then assemble the amplified fragments in correct order.

The present description also relates to nucleic acid constructs. The term "nucleic acid construct" or "polynucleotide construct" refers to one or more single- or double-stranded nucleic acid molecules, isolated from a naturally occurring gene or modified to contain a nucleic acid fragment in a manner that does not occur in nature. The term "nucleic acid molecule" mainly refers to physical nucleic acid molecules, and the term "nucleotide sequence" primarily refers to the sequence of nucleotides on a nucleic acid molecule, but the two terms are used interchangeably, especially with respect to a nucleic acid molecule, or nucleic acid sequence, capable of encoding a protein or protein domain. The nucleic acid construct contains the polynucleotide sequence described herein, and one or more regulatory sequences operably linked to these sequences. The polynucleotide sequence described herein can be manipulated in a variety of ways to ensure expression of the CAR. The nucleic acid construct can be manipulated according to the difference or requirements of the expression vector prior to insertion of the nucleic acid construct into the vector. Techniques that utilize recombinant DNA methods to alter polynucleotide sequences are known in the art.

Regulatory sequences can be suitable promoter sequences. The promoter sequence is usually operably linked to the sequence encoding the protein to be expressed. The promoter can be any nucleotide sequence that shows transcriptional activity in the host cell of choice, comprising mutated, truncated, and hybrid promoters, and can be derived from genes encoding extracellular or intracellular polypeptides homologous or heterologous to the host cell. The regulatory sequence can also be a suitable transcription termination sequence, a sequence recognized by a host cell to terminate transcription. A terminator sequence is operably linked to the 3' terminal of the nucleotide sequence encoding the polypeptide. Any terminator that is functional in the host cell of choice can be used in the present description. The regulatory sequence can also be a suitable leader sequence, an untranslated region of the mRNA important for translation by the host cell. A leader sequence is operably linked to the 5' terminal of the nucleotide sequence encoding the polypeptide. Any terminator that is functional in the host cell of choice can be used in the present description.

In some embodiments of the present description, the nucleic acid construct also comprises a coding sequence of a PD-1 binding molecule. The PD-1 binding molecule includes but are not limited to: PD-1 antibodies or antigen-binding fragments thereof, PD-1 heavy chain antibodies or antigen-binding fragments thereof, monovalent or multivalent single domain antibodies, and multispecific single domain antibodies, comprising one, two or more of the PD-1 single domain antibodies. The PD-1 binding molecules and anti-PD-1 single domain antibodies mentioned described herein respectively comprise all PD-1 binding molecules and all anti-PD-1 single domain antibodies described in CN202011582908. The entirety of the above patent description is incorporated herein in its each embodiment being specifically and individually by reference. As stated in the present description, the PD-1 binding molecule is a monovalent or multivalent single domain antibody, a multispecific single domain antibody, a heavy chain antibody or an antigen binding fragment thereof, and an antibody or an antigen binding fragment thereof comprising one, two or more of the anti-PD-1 single domain antibodies.

Therefore, the complementarity determining region CDR of the anti-PD-1 single domain antibody described herein comprises CDR1, CDR2 and CDR3, wherein CDR1, CDR2 and CDR3 comprise the sequences shown in SEQ ID NO: 1, 2 and 3 in CN202011582908, respectively. Exemplarily, in the anti-PD-1 single domain antibody described herein, CDR1 comprises the sequence shown in any one of SEQ ID NOs: 4-39 and 320 in CN202011582908, CDR2 comprises the sequence shown in any one of SEQ ID NOs: 40-75 in CN202011582908; CDR3 comprises the sequence shown in any one of SEQ ID NOs: 76-183 in CN202011582908. In one or more embodiments, the anti-PD-1 single domain antibody described herein comprises CDR1, CDR2, and CDR3 shown in any of groups a1 to a136 in CN202011582908. The anti-PD-1 single domain antibody VHH described herein can be as shown in any of SEQ ID NO: 184-319 in CN202011582908. Preferably, the anti-PD-1 single domain antibody VHH described herein can be as shown in any of SEQ ID NO: 197, 200, 223-309 in CN202011582908. In addition, each CDR or the full of the anti-PD-1 single domain antibody described herein can be as shown in any embodiment in CN202011582908.

Exemplarily, the CDR1 of the anti-PD-1 single domain antibody is as shown in SEQ ID NO: 124 or 128, the CDR2 being as shown in SEQ ID NO: 125 or 129, and the CDR3 being as shown in SEQ ID NO: 126 or 130; the sequence of the anti-PD-1 single domain antibody VHH being as shown in SEQ ID NO: 127 or 131.

The PD-1 binding molecule described herein can be a monovalent or multivalent single domain antibody, a multispecific single domain antibody, a heavy chain antibody, or an antigen binding fragment thereof, an antibody, or an antigen binding fragment thereof comprising one, two, or more of the anti-PD-1 single domain antibodies described herein. The multivalent single domain antibody or the multispecific single domain antibody is linked to multiple single domain antibodies through linkers. The linker consists of 1-15 amino acids selected from G and S. Herein, the heavy chain antibody is a camelid heavy chain antibody or a shark heavy chain antibody. The antigen binding fragment of the heavy chain antibody is a single chain heavy chain antibody. The heavy chain antibody further comprises a heavy chain constant region. In one or more embodiments, the heavy chain constant region is a constant region of camelid heavy chain antibody, comprising CH2 and CH3. In one or more embodiments, the CH2 and CH3 are CH2 and CH3 of human IgG Fc, such as CH2 and CH3 of IgG4.

Therefore, in one or more embodiments, the nucleic acid construct comprises the coding sequences of the CAR and the PD-1 binding molecule. In one or more embodiments, the nucleic acid construct comprises an expression cassette of the chimeric antigen receptor and an expression cassette of the PD-1 binding molecule. The two expression cassettes are contained in one or more vectors. Alternatively, the nucleic acid construct is an expression cassette, wherein the sequence encoding the chimeric antigen receptor and the sequence encoding the PD-1 binding molecule are within the expression cassette.

In certain embodiments, the nucleic acid construct is a vector. The term "vector" can transfer a genetic sequence into a target cell. Generally, "vector construct", "expression vector" and "gene transfer vector" mean any nucleic acid construct capable of directing the expression of a gene of interest and of transferring the gene sequence into a target cell, either through the whole or part of the vector genomic integration, or as a vector of transient or heritable maintenance of extrachromosomal elements. Thus, the term comprises cloning vectors, expression vectors, and integration vectors. Expression of the present polynucleotide sequences is generally achieved by operably linking the polynucleotide sequences of the description to a promoter and incorporating the construct into an expression vector. This vector may be suitable for replicating and integrating eukaryotic cells. A typical cloning vector comprises transcriptional and translational terminators, initiation sequences and a promoter useful for regulating the expression of the desired nucleic acid sequence. The nucleic acid construct can be one or more vectors, each vector containing one or two or three expression cassettes as described in any embodiment herein.

The polynucleotide sequences of the present description can be cloned into many types of vectors. For example, plasmids, phagemids, phage derivatives, animal viruses, and cosmids can be cloned into. Further, the vector is an expression vector. Expression vectors can be provided to cells in the form of viral vectors. Viral vector technology is well known in the art and described, for example, in Sambrook et al. (2001, Molecular Cloning: A Laboratory Manual, Cold Spring Harbor Laboratory, New York) and other handbooks of virology and molecular biology. Viruses that can be used as vectors include, but are not limited to, retroviruses, adenoviruses, adeno-associated viruses, herpesviruses, and lentiviruses.

In general, suitable vectors comprise an origin of replication functional in at least one organism, a promoter sequence, convenient restriction sites and one or more selectable markers (e.g., WO 01/96584; WO01/29058; and U.S. Patent No. 6,326,193).

The nucleic acid construct can be a cloning vector or an expression vector. The expression vector is preferably a constitutive expression vector, such as a transposable vector (or " transposon vector"). In certain embodiments, the transposon vector is a eukaryotic expression vector comprising a transposable element selected from piggybac, sleeping beauty, frog prince, Tn5 or Ty. This type of transposon vector comprises the 5' inverted terminal repeat (5'ITR) of the corresponding transposon and the 3' inverted terminal repeat (3'ITR) of the corresponding transposon. Therefore, in certain embodiments, the nucleic acid construct also comprises a coding sequence of a transposase used to transpose a sequence encoding a chimeric antigen receptor and/or a PD-1 binding molecule, for example, integrating the coding sequence or expression cassette of the chimeric antigen receptor and/or PD-1 binding molecule into the genome. The transposase can be any transposase known in the art suitable for human cells, for example a transposase from piggybac, sleeping beauty, frog prince, Tn5 or Ty transposase systems. When transposases from different transposition systems are used, the sequences of the 5'ITR and 3'ITR in the vector are also correspondingly changed to sequences compatible with the transposition system, which can be easily determined by those skilled in the art. The expression cassette of the CAR or antibody of the present description is contained between the 5'ITR and the 3'ITR, comprising the corresponding promoter sequence, the sequence encoding the CAR or antibody, and the polyA tailing signal sequence. An exemplary transposase is PBase. In certain embodiments, the transposon 5' inverted terminal repeat sequence is as shown in SEQ ID NO: 1 of CN105154473A (the contents of which are incorporated herein by reference). In certain embodiments, the transposon 3' inverted terminal repeat sequence is as shown in SEQ ID NO: 4 of CN105154473A. In certain embodiments, the piggybac transposase is a transposase containing a sequence encoding a c-myc nuclear localization signal. In certain embodiments, the sequence encoding the piggybac transposase is shown in SEQ ID NO: 5 of CN 201510638974.7. Exemplary vector backbones are pNB338B and pS338B described in CN111206043A and CN114729361A.

Therefore, in one or more embodiments, the nucleic acid construct comprises the coding sequences of the CAR and the transposase. In one or more embodiments, the nucleic acid construct comprises an expression cassette of the chimeric antigen receptor and an expression cassette of the transposase. The two expression cassettes are contained in one or more vectors. Alternatively, the nucleic acid construct is one expression cassette, wherein the sequence encoding the chimeric antigen receptor and the sequence encoding the transposase are within the expression cassette.

In certain cases, the nucleic acid constructs described herein comprise coding sequences for the CAR, the transposase, and the PD-1 binding molecule. The nucleic acid construct can contain three expression cassettes, namely the expression cassette of the chimeric antigen receptor, the expression cassette of the transposase and the expression cassette of the PD-1 binding molecule; and the three expression cassettes can be contained in one, two or three vectors. Alternatively, the nucleic acid construct contains two expression frames, one of which comprises any one of the sequences encoding the chimeric antigen receptor, the sequence encoding the transposase and the sequence encoding the PD-1 binding molecule, and the other of which comprises remaining coding sequences. The two expression cassettes can be included in one or two vectors. Alternatively, the nucleic acid construct contains an expression cassette, wherein the sequence encoding the chimeric antigen receptor, the expression cassette of the transposase and the sequence encoding the PD-1 binding molecule are within the expression cassette.

In one or more embodiments, the nucleic acid construct comprises: one vector, which comprises (a) an expression cassette of the CAR and optional (b) an expression cassette of the transposase and/or an expression cassette of the PD-1 binding molecule; two vectors, wherein the two vectors (a) respectively comprise an expression cassette of the CAR and an expression cassette of the PD-1 binding molecule, and optionally, either one of the two vectors also comprises an expression cassette of the transposase, or (b) one of the two vector comprises an expression cassette of a transposase, and the other comprises an expression cassette of the CAR and an expression cassette of the PD-1 binding molecule; or three vectors, which comprise an expression cassette of the CAR, an expression cassette of the PD-1 binding molecule and an expression cassette of a transposase.

An example of a suitable promoter is the immediate early cytomegalovirus (CMV) promoter sequence. The promoter sequence is a strong constitutive promoter sequence capable of driving high-level expression of any polynucleotide sequence operably linked thereto. Another example of a suitable promoter is elongation growth factor-1α (EF-1α). Another example of a suitable promoter is an IFNγ driven promoter. However, other constitutive promoter sequences can also be used, including but not limited to the early stage of simian virus 40 (SV40) promoter, mouse mammary tumor virus (MMTV), human immunodeficiency virus (HIV) long terminal repeat (LTR) promoter, MoMuLV promoter, avian leukemia virus promoter, Epstein-Barr virus immediate early promoter, Rous sarcoma virus promoters, as well as human gene promoters, such as, but not limited to, actin promoter, myosin promoter, heme promoter, and creatine kinase promoter. Furthermore, the use of inducible promoters may also be considered. The use of inducible promoters provides a molecular switch capable of turning on expression of a polynucleotide sequence operably linked to the inducible promoter when expression is required and turning off expression when expression is undesirable. Examples of inducible promoters include but are not limited to metallothionein promoters, glucocorticoid promoters, progesterone promoters, and tetracycline promoters.

To assess the expression of a CAR polypeptide or portion thereof, the expression vector introduced into the cell can also comprise either or both of a selectable marker gene or a reporter gene to facilitate identification and selection of expressing cells accessed from a cluster of transfected or infected cells by the viral vector. In other aspects, selectable markers can be carried on a single DNA fragment and used in co-transfection procedures. Both the selectable marker and the reporter gene can be flanked by appropriate regulatory sequences to enable expression in the host cell. Useful selectable markers include, for example, antibiotic resistance genes such as neo and the like.

Reporter genes are used to identify potentially transfected cells and to assess the functionality of regulatory sequences. Expression of the reporter gene is measured at an appropriate time after the DNA has been introduced into the recipient cells. Suitable reporter genes may include genes encoding luciferase, β-galactosidase, chloramphenicol acetyltransferase, secreted alkaline phosphatase, or the green fluorescent protein gene. Suitable expression systems are well known and can be prepared using known techniques or obtained commercially.

Methods of introducing genes into cells and expressing genes into cells are known in the art. Vectors can be readily introduced into host cells, e.g., mammalian, bacterial, yeast or insect cells, by any method known in the art. For example, expressing vectors can be transfected into host cells by physical, chemical or biological means.

Physical methods of introducing polynucleotides into host cells comprise calcium phosphate precipitation, lipofection, particle bombardment, microinjection, electroporation, and the like. Biological methods for introducing polynucleotides of interest into host cells comprise the use of DNA and RNA vectors. Chemical means of introducing polynucleotides into host cells comprise colloidal dispersion systems, such as macromolecular complexes, nanocapsules, microspheres, beads; and lipid-based systems, including oil-in-water emulsions, micelles, mixed micelles, and lipids plastid.

T cells suitable for use in the present description can be various types of T cells from various sources. For example, T cells can be derived from PBMCs of patients with B-cell malignancies. In certain embodiments, the obtained T cells can be first stimulated and activated with an appropriate amount (for example, 30-80ng/ml, such as 50ng/ml) of CD3 antibodies, and then cultured in a medium containing an appropriate amount of IL2 (for example, 30 to 80 IU/ml, such as 50 IU/ml) for later use.

Therefore, in certain embodiments, the present description provides a genetically modified cell that comprises the polynucleotide sequence described herein, or comprises the nucleic acid construct described herein, or is prepared by using the method described herein, or stably expresses a CAR and optional an anti-PD-1 binding molecule described herein. Cells described herein (e.g., T cells) can also comprise or express a transposase or its coding sequence.

The CAR-T cells of the present description can undergo robust in vivo T cell expansion and persist at high levels in the blood and bone marrow for an extended amount of time and form specific memory T cells. Without wishing to be bound by any particular theory, CAR-T cells of the present description can differentiate into a central memory-like state in vivo upon encountering and subsequent elimination of target cells expressing surrogate antigens.

The present description also encompasses a type of cell therapy in which T cells are genetically modified to express a CAR and optional an anti-PD-1 binding molecule as described herein, and the CAR-T cells are infused into a recipient in need thereof. The injected cells can kill the recipient's tumor cells. Unlike antibody therapies, CAR-T cells are able to replicate in vivo, producing long-term persistence that can lead to sustained tumor control.

The anti-tumor immune response caused by CAR-T cells can be an active or passive immune response. Additionally, a CAR-mediated immune response can be part of an adoptive immunotherapy step, in which CAR-T cells induce an immune response specific for the antigen-binding portion of the CAR.

Therefore, the disease that can be treated using the CAR, coding sequence thereof, nucleic acid construct, expression vector and CAR-T cells of the present description is preferably mesothelin-mediated cancer. Preferably, the disease also benefits from anti-PD-1 antibody therapy, being a PD-1 mediated cancer.

Specifically, as used herein, " mesothelin-mediated cancers" especially comprise, various types of ovarian cancer, pleural mesothelioma (such as epithelial mesothelioma), pancreatic cancer, and squamous cell carcinoma of the cervix, endometrium, head, neck, vagina, lungs, and esophagus. In certain embodiments, the mesothelin-mediated disease is malignant pleural mesothelioma, pancreatic cancer, ovarian cancer, cervical cancer, endometrial cancer, and lung adenocarcinoma. Diseases that "benefit from anti-PD-1 antibody therapy" or "PD-1-mediated cancers" comprise gastric cancer, melanoma, lung cancer, head and neck cancer, renal cell carcinoma, urothelial cancer, non-Hodgkin lymphoma, etc.

The CAR-modified T cells of the present description can be administered alone or as a pharmaceutical composition in combination with a diluent and/or with other components such as relevant cytokines or cell populations. Briefly, pharmaceutical compositions of the present description can comprise CAR-T cells as described herein, combined with one or more pharmaceutically or physiologically acceptable excipients (e.g., carriers, diluents or excipients). Such compositions can comprise buffers such as neutral buffered saline, sulfate buffered saline, and the like; carbohydrates such as glucose, mannose, sucrose or dextran, mannitol; protein; polypeptides or amino acids such as glycine; antioxidants; chelating agents such as EDTA or glutathione; adjuvants (e.g., aluminum hydroxide); and preservatives.

The pharmaceutical compositions of the present description can be administered in a manner suitable for the disease to be treated (or prevented). The amount and frequency of administration will be determined by such factors as the patient's condition, and the type and severity of the patient's disease.

When an "immunologically effective amount", "anti-tumor effective amount", "tumor-inhibitory effective amount" or "therapeutic amount" is indicated, the precise amount of the composition of the present description to be administered can be determined by a physician, considering patients' (subjects') age, weight, tumor size, extent of infection or metastasis, and individual differences in the condition. It may generally be stated that pharmaceutical compositions comprising T cells described herein may be administered at a dose of $10^4$ to $10^9$ cells/kg body weight, preferably $10^5$ to $10^6$ cells/kg body weight. T cell compositions can also be administered multiple times at these dosages. Cells can be administered using infusion techniques well known in immunotherapy (see, e.g., Rosenberg et al., New Eng. J. of Med. 319:1676, 1988). The optimal dosage and treatment regimen for a particular patient can be readily determined by one skilled in the medical field by monitoring the patient for signs of disease and adjusting treatment accordingly.

Administration of subject compositions may be in any convenient manner, including by spraying, injection, swallowing, infusion, implantation or transplantation. The compositions as used herein can be administered to a patient subcutaneously, intradermally, intratumorally, intranodally, intraspinally, intramuscularly, by intravenous injection, or intraperitoneally. In one embodiment, the T cell composition of the invention is administered to the patient by intradermal or subcutaneous injection. In one embodiment, the T cell composition of the present description is preferably administered by intravenous injection. The T cell composition can be injected directly into tumors, lymph nodes, or sites of infection.

In some embodiments of the description, the CAR-T cells or compositions thereof in the present description can be combined with other therapies known in the art. Such therapies include, but are not limited to, chemotherapy, radiotherapy, and immunosuppressants. For example, treatments may be combined with radiotherapy or chemotherapy agents known in the art to treat mesothelin-mediated diseases.

Herein, "anti-tumor effect" refers to a biological effect, which can be expressed by a reduction in tumor volume, a reduction in the number of tumor cells, a reduction in the number of metastases, an increase in life expectancy, or an improvement in various physiological symptoms related to cancer.

"Patient", "subject", "individual" and the like are used interchangeably herein to refer to a living organism, such as a mammal, that can elicit an immune response. Examples include, but are not limited to, humans, dogs, cats, mice, rats, and transgenic species thereof.

The present description is described in further detail with reference to the following experimental examples. These examples are provided for illustrative purposes only and are not intended to be limiting unless otherwise specified. Accordingly, the present description should in no way be construed as limited to the following examples, but should be construed to include any and all changes that become apparent in light of the teachings provided herein. Unless otherwise specified, the methods and materials used in the examples are conventional materials and methods in the art.

EXAMPLE

Experimental methods of operation:

Isolation and cryopreservation of human peripheral blood mononuclear cells (PBMC)

Firstly, the human apheresis blood is diluted 1-2 times with DPBS, and then the diluted blood is gently added to the upper layer of ficoll to avoid mixing the two solutions together (blood: Ficoll=3:4). The solutions are centrifuged at 800g for 20 minutes (the up speed of centrifuge is 1, the down speed is 0), after centrifugation, a pipette is used to absorb the buffy coat layer to a new centrifuge tube. The volume of DPBS is added 3 times and mixed thoroughly, put into a centrifuge and centrifuged at 1500rpm, and the supernatant is removed after 10 minutes, and this step are repeated 2-3 times. After counting, AIM-V culture medium is added at a volume of 5*10^6 cells/ml and incubated in a 5% $CO_2$ incubator overnight. After culturing, the suspended cells are collected, counted and frozen.

CAR-T preparation steps

Human mononuclear cells (PBMCs) are isolated from peripheral blood of healthy human donors using Ficoll density gradient centrifugation (GE). The isolated PBMCs are cultured overnight in AIM-V culture medium at a concentration of $1\times10^6$/mL and then cryopreserved and stored in the Shanghai Cell Therapy Group Cell Bank with serum-free cryopreservation solution (NCM Biotech) at a cryopreservation density of $1\times10^7$ /mL. Fresh or cryopreserved PBMCs are electroporated using the Lonza 4D electroporation instrument. The electroporation process is optimized according to the operating procedures of the electroporation instrument. 5-7 $\times$ $10^6$ PBMCs are resuspend in 100 $\mu$l electroporation solution, and 6 $\mu$g pNB338B-1444-CAR plasmid, or 4 $\mu$g pNB338B-1444-CAR + 4 $\mu$g pS338B-1182 plasmid are added to the cell suspension, and electroporation is performed by EO-115 program. The electroporated PBMCs are added to a 12-well plate containing 2 ml of 37°C preheated AIM-V (+2% FBS) culture medium and cultured for 4-6 hours, and then transferred to an antigen-coated six-well plate (5 $\mu$g/ml CD28 antibody + 5 $\mu$g/ml MNSL antigen), and the culture medium is supplemented to 4 ml, and IL-2 is added with a final concentration of 500 IU/ml. The negative control group is electroporated with 6 $\mu$g Mock-T plasmid, and the cell culture plate is coated with 5 $\mu$g/ml CD3 antibody + 5 $\mu$g/ml CD28 antibody. The rest of the operations are the same as the above experimental group and are performed simultaneously. The electroporated PBMCs are cultured in the above-mentioned culture medium for 5 days before being passaged for the first time, and then every 2-3 days. The preparation of CAR-T cells is completed on the 13th day. PBMCs during passage are cultured in AIM-V culture medium containing 2% FBS and 100 IU/ml IL-2, and the passage cell density is $5 \times 10^5$/mL. Cell numbers and cell viability rates are detected and recorded using CountStar.

After the CAR-T cell preparation process and preparation are completed, flow cytometry is used to detect the CAR positive rates and other surface markers on the cell surface. The following antibodies are used for flow cytometry detection: Anti-mesothelin, Anti-human CD3 PE-Cy5/CD4 PE/CD8 FITC Cocktail (Biolegend), anti-CD45RO (Biolegend), anti-CD62L (Biolegend), anti-CCR7 (Biolegend), anti-CD107a, anti-PD1, anti-TIM3, anti-LAG3 and anti-PDL1.

RTCA cell killing

The killing effect of CAR-T cells on tumor cells is detected using xCELLigence RTCA equipment (Roche Applied Science, Canada), and the detection process is carried out according to the equipment operating instructions. An appropriate amount ($5\times10^3$-$2\times10^4$) of target cells (tumor cells) are resuspended in 50 $\mu$l of culture medium and inoculated them into the microplate (16-well or 96-well) provided by the RTCA equipment. Tumor cells are cultured on RTCA equipment for about 24 hours. Different numbers of effector cells (CAR-T cells) are resuspended in 50 $\mu$l of culture medium according to different effect-to-target ratios (effector cells: target cells), mixed with tumor cells, and continued to be cultured on RTCA equipment for 3-5 days, until the cell index (CI, indicating cell growth) reaches about 1.5. Cell killing curves are recorded by RTCA equipment, and the data are analyzed with RTCA Pro 2.3.0 software.

Antibody secretion detection

The amount of PD1 nanoantibody secreted during and after the preparation of MSLN-targeted CAR-T cells with self-secreting PD1 nanoantibody is detected using the ELISA (Enzyme-linked immunosorbent assay) method. During the preparation process, the cells are passaged at a density of $5\times10^5$/mL, and the supernatants on days 5-7 and 11-13 are collected to detect the amount of PD1 antibody secreted. After preparation, $5\times10^5$/mL CAR-T cells are inoculated into 5 $\mu$g/ml MSLN antigen-coated cell culture plates. After culture for 48h, the supernatant is collected to detect PD1 antibody secretion. The following antibodies are used for ELISA to detect the secretion of PD1 nanoantibody: anti-1182-VHH (CHENGHUANG NANOMAB TECHNOLOGY CO. LTD., the amino acid sequence is shown in SEQ ID NO: 134).

Example 1, CAR And CAR-T Cells Containing Mesothelin Nanoantibodies

Non-viral vector MSLN CAR-T cells are constructed by using MSLN CAR derived from nanoantibodies of different sequences and MSLN CAR in the benchmark to construct plasmids and transfer the genes into PBMCs derived from healthy people by electroporation, to verify the performance of CAR-T cells in vitro, comprising indicators such as proliferation ability, CAR positive rate, killing ability of target cells in vitro, and cytokine secretion levels.

1.1 Construction of MSLN CAR-T cell plasmid

Experimental instruments: ordinary PCR instrument (TRIO, Jena), electrophoresis instrument (Bio-Rad, 1645050), electrophoresis tank (Bio-Rad, 1704483), desktop microcentrifuge (Thermo, Pico17), metal bath heating module (Thermo, DCD), electric thermostatic water tank (Shanghai Bluepard, DK-8D), automatic gel imager (Tanon, 2500)

Experimental Reagents

| Reagent | Manufacturer |
|---|---|
| PCR tube | AXYGEN |
| 1.5mL EP tube | AXYGEN |
| Restriction endonucleases | BioLabs |
| DNA ligase | TaKaRa |
| PCR enzyme | TOYOBO |

(continued)

| Reagent | Manufacturer |
|---|---|
| Homologous recombinase | BioLabs |
| Agrose | TIANGEN |
| Agarose gel recovery kit | ZYMO RESEARCH |
| TRYPTONE | OXOID |
| YEAST EXTRACT | OXOID |
| NaCl | HUSHI |
| Plasmid mini preparation kit | TIANGEN |
| Plasmid Purification MaxiPrep Kit | Introgen Thermo Fisher Scientific |
| Kana | SANGON |

Experimental Methods

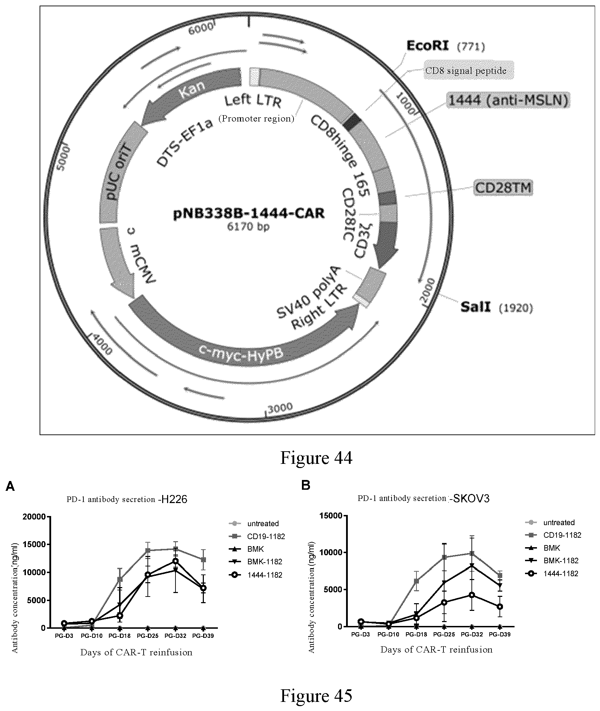

The full-length nucleic acid sequence and primers were synthesized based on the sequence of MSLN nanoantibody, and a large number of target fragments are obtained through PCR. The pNB338B vector (for its sequence and structure, please refer to "pNB338B-E" in CN109988759A) was subjected to double enzyme digestion (EcoR1 and Sal1) to obtain vector fragments, and the target fragment and the vector fragment were connected through DNA ligase to obtain the plasmid. The constructed vector is sequenced. After confirming the sequence was correct through sequencing, the plasmid was extracted in large quantities. Among them, the vector structure of pNB338B-1444-CAR is shown in Figure 44. The amino acid sequence of MSLN3[1444] nanoantibody is shown in SEQ ID NO:83 (the CAR containing it is shown in SEQ ID NO:122), and the amino acid sequence of MSLN3[1450] nanoantibody is shown in SEQ ID NO:85 (the CAR containing it is shown in SEQ ID NO: 123). The amino acid sequence of MSLN3 [Scfv] is shown in SEQ ID NO: 3 of CN109306014A.

1.2 Preparation of Mesothelin-targeted CAR-T Cells

Mock-T and MSLN CAR-T are prepared by electroporation.

Experimental instruments: electroporation instrument (Lonza 4D), automated cell counter (Invitrogen Countess II FL), microscope (Jiangnan XD-202), biological safety cabinet (Thermo Fisher Scientific), refrigerator (Qingdao Haier), carbon dioxide incubator (Thermo), desktop low-speed centrifuge (Xiangyi), Pico17 desktop microcentrifuge (Thermo)

Experimental Reagents

| Reagent | Manufacturer |
|---|---|
| CD3 antibody | Biolegend 317302 |
| CD28 antibody | Biolegend 302902 |
| Mesothelin antigen | Kactus Biosystems |
| AIM-V culture medium | GIBCO |
| Ficoll-Paque PREMIUM | GE |
| P3 Primary cell solution Box, small volume electroporation kit | LONZA |
| FBS | GIBCO |
| DPBS | Hyclone |
| Recombinant human interleukin-2 for injection | Shandong Quangang Pharmaceutical Co., Ltd. |
| Serum-free cell cryopreservation solution | New Cell & Molecular Biotech Co., Ltd. |

(continued)

| Reagent | Manufacturer |
|---|---|
| 0.4%Trypan blue dye | Gibco |

Experimental Methods

Isolation and cryopreservation of human peripheral blood mononuclear cells (PBMC)

See experimental procedures for details.

PBMC cell recovery and MSLN CAR-T preparation

The densities of the three donor PBMCs (AC-201909A, AC-201908B, and AC-201910A) were 6.08E+06~9.67E+06 cells/mL, and the viability rates exceeded 90%. When electroporation of a single plasmid, the plasmid concentration is 6 $\mu$g per sample. See experimental procedures for details.

Experimental results

| Donor No. | Cell Group | Plasmid | Plasmid concentratio n ($\mu$g/$\mu$L) |
|---|---|---|---|
| AC-201908B | Mock-T | pNB338B-MCS | 4.128 |
| | MSLN3 [Scfv]CAR-T | pNB338B-meso3CAR-8E | 3.575 |
| AC-201909A | MSLN3[1444]CAR-T | pNB338B-1444-CAR | 3.6 |
| AC-201910A | MSLN3[1450]CAR-T | pNB338B-1450-CAR | 3.797 |

1.3 Detection of in vitro proliferation and positive rate of MSLN CAR-T cells

The culture period after preparation of non-viral vector CAR-T is generally 13 days. The final yield of CAR-T cells is obtained through the proliferation of total cells and the final proportion of CAR-positive cells. Therefore, the purpose of this experiment is to determine the proliferation and final yield of CAR-T cells. The positive rate was used to evaluate the quality of the prepared mock-T cells and MSLN CAR-T cells.

Experimental instruments: automated cell counter (Invitrogen Countess II FL), microscope (Jiangnan XD-202), biological safety cabinet (Thermo Fisher Scientific), refrigerator (Qingdao Haier), carbon dioxide incubator (Thermo), desktop low-speed centrifuge (Xiangyi), Pico17 desktop microcentrifuge (Thermo)

Experimental Reagents

| Reagent | Manufacturer |
|---|---|
| AIM-V culture medium | GIBCO |
| FBS | GIBCO |
| DPBS | Hyclone |
| Recombinant human interleukin-2 for injection | Shandong Quangang Pharmaceutical Co., Ltd. |
| Serum-free cell cryopreservation solution | New Cell & Molecular Biotech Co., Ltd. |
| 0.4% Trypan blue dye | Gibco |
| Biotinylated human mesothelin antigen | Kactus Biosystems |
| PE streptavidin | BD |

Experimental Methods

1) CAR-T cell culture and passage

The proliferation multiple of each group of cells was calculated and the cell proliferation curve was drawn through cell counting and density methods. The passage density was $5 \times 10^5$ cells/mL, and the cells were passaged and counted on days 5, 8, and 11. The IL-2 concentration was 100 U/mL. See experimental procedures for details.

2) The proportion of MSLN CAR-T positive cell proportion detection

Flow cytometry was used to detect changes in the CAR positive rate during the cell proliferation process after antigen enrichment to compare different CAR sequences. See experimental procedures for details.

Experimental results

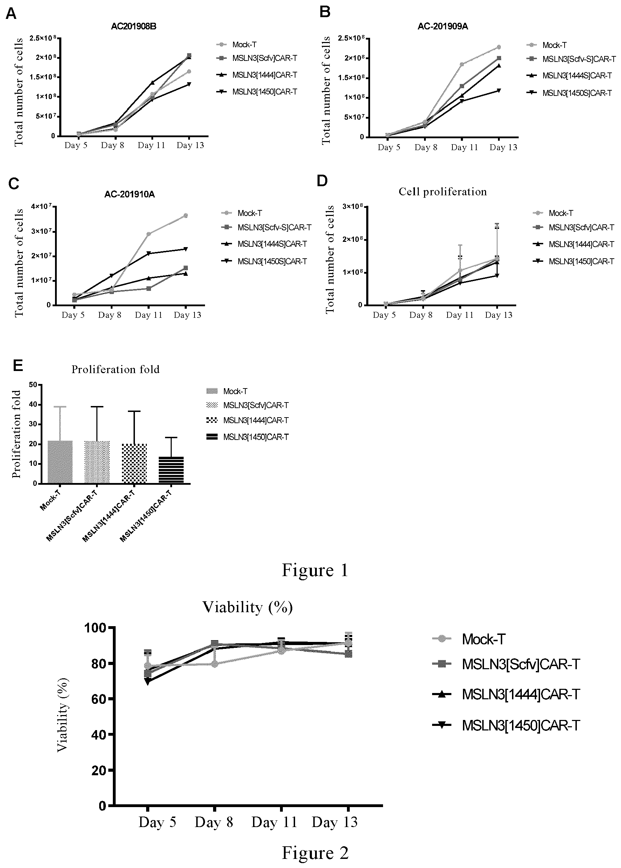

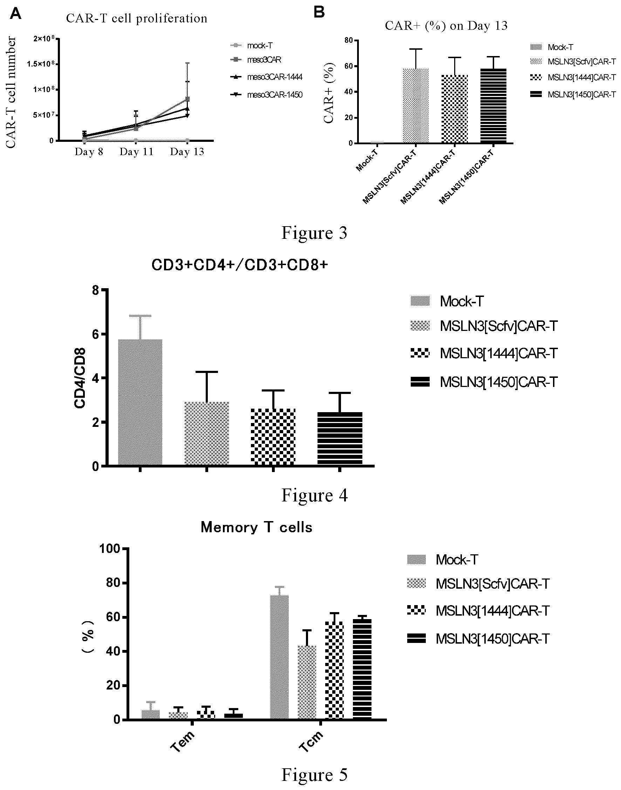

The results are shown in Figure 1-3 and Table 1-3. The results show that: there are certain differences in the proliferation ability of CAR-T cells prepared from PBMCs derived from three donors. Two of the donors proliferated well, with amplification times of about 20-38 times, while one donor had poor proliferation, only 1.3-3.7 times. Overall, MSLN3[1444]CAR-T cells can be successfully expanded, and their proliferation ability is comparable to Mock-T cells and MSLN3[Scfv]CAR-T cells (Figure 1). In addition, MSLN3[1444]CAR-T cells, like Mock-T cells and MSLN3[Scfv] CAR-T cells, have a high viability rate (>90%) (Figure 2). The CAR positive rate of MSLN3[1444]CAR-T cells reached approximately 60%, which is comparable to MSLN3[Scfv]CAR-T (Figure 3).

Table 1, MSLN CAR-T cell proliferation data from D5 to D13

| Sample number | Type | Day5 | Day8 | Day11 | Day13 | Proliferat ion multiple |
|---|---|---|---|---|---|---|
| AC-201908 B | Mock-T | 4.06E+ 06 | 1.09E+ 05 | 5.99E+ 05 | 1.44E+ 06 | 24.00 |
| | MSLN3 [Scfv]C AR-T | 5.85E+ 06 | 6.80E+ 06 | 1.41E+ 07 | 8.93E+ 07 | 30.15 |
| | MSLN3 [1444] CAR-T | 4.97E+ 06 | 8.15E+ 06 | 3.34E+ 07 | 7.49E+ 07 | 29.40 |
| | MSLN3[1450] CAR-T | 3.44E+ 06 | 5.40E+ 06 | 2.71E+ 07 | 6.37E+ 07 | 19.19 |
| AC-201909 A | Mock-T | 5.82E+ 06 | 1.57E+ 04 | 1.85E+ 06 | 1.40E+ 06 | 37.73 |
| | MSLN3 [Scfv]C AR-T | 6.02E+ 06 | 2.59E+ 06 | 5.44E+ 07 | 1.49E+ 08 | 33.07 |
| | MSLN3 [1444] CAR-T | 6.30E+ 06 | 2.00E+ 07 | 5.84E+ 07 | 1.10E+ 08 | 29.99 |
| | MSLN3[1450] CAR-T | 4.67E+ 06 | 1.60E+ 07 | 4.96E+ 07 | 6.86E+ 07 | 19.42 |
| AC-201910 A | Mock-T | 4.41E+ 06 | 4.03E+ 04 | 5.64E+ 05 | 2.66E+ 05 | 3.77 |
| | MSLN3 [Scfv]C AR-T | 2.15E+ 06 | 1.17E+ 06 | 2.75E+ 06 | 8.67E+ 06 | 1.58 |
| | MSLN3 [1444] CAR-T | 2.25E+ 06 | 2.39E+ 06 | 4.70E+ 06 | 8.05E+ 06 | 1.34 |
| | MSLN3[1450] CAR-T | 2.73E+ 06 | 5.02E+ 06 | 9.76E+ 06 | 1.54E+ 07 | 2.36 |

Table 2, Cell viability rates of MSLN CAR-T cultured from D5 to D13

| Sample number | Type | Day 5 | Day 8 | Day1 1 | Day1 3 |
|---|---|---|---|---|---|
| AC-201908B | Mock-T | 78% | 69% | 87% | 93% |
| | MSLN3 [Scfv]CAR-T | 78% | 89% | 89% | 90% |
| | MSLN3[1444]CAR-T | 78% | 88% | 92% | 93% |
| | MSLN3[1450]CAR-T | 68% | 88% | 93% | 93% |

(continued)

| Sample number | Type | Day 5 | Day 8 | Day1 1 | Day1 3 |
|---|---|---|---|---|---|
| AC-201909A | Mock-T | 85% | 84% | 93% | 96% |
| | MSLN3 [Scfv]CAR-T | 85% | 92% | 91% | 86% |
| | MSLN3[1444]CAR-T | 85% | 92% | 90% | 94% |
| | MSLN3[1450]CAR-T | 74% | 88% | 89% | 92% |
| AC-201910A | Mock-T | 73% | 86% | 81% | 85% |
| | MSLN3 [Scfv]CAR-T | 60% | 91% | 85% | 80% |
| | MSLN3[1444]CAR-T | 66% | 91% | 90% | 86% |
| | MSLN3[1450]CAR-T | 67% | 88% | 93% | 88% |

Figure 3, Changes of the positive rate during MSLN CAR-T culture and the number of CAR-positive cells on D13

| Sample number | Type | Day8 | Day11 | Day13 | Total number of CAR-T cells (D13) |
|---|---|---|---|---|---|
| AC-201908B | Mock-T | 0.65% | 0.56% | 0.87% | - |
| | MSLN3[Scfv]CAR-T | 23.13% | 14.26% | 43.07% | 3.85E+07 |
| | MSLN3[1444]CAR-T | 24.07% | 24.36% | 37.00% | 2.77E+07 |
| | MSLN3[1450]CAR-T | 28.15% | 29.09% | 48.20% | 3.07E+07 |
| AC-201909A | Mock-T | 0.04% | 1.00% | 0.61% | - |
| | MSLN3[Scfv]CAR-T | 8.30% | 41.88% | 74.06% | 1.10E+08 |
| | MSLN3[1444]CAR-T | 50.42% | 55.14% | 60.39% | 6.65E+07 |
| | MSLN3[1450]CAR-T | 58.86% | 54.38% | 58.06% | 3.98E+07 |
| AC-201910A | Mock-T | 0.64% | 1.94% | 0.73% | - |
| | MSLN3[Scfv]CAR-T | 21.04% | 39.87% | 56.64% | 4.91E+06 |
| | MSLN3[1444]CAR-T | 33.03% | 42.27% | 61.91% | 4.98E+06 |
| | MSLN3[1450]CAR-T | 41.39% | 46.27% | 67.38% | 1.04E+07 |

1.4 MSLN CAR-T cell phenotype detection

The phenotype of the CART cells we prepared was evaluated by detecting the CD3/CD4/CD8 and TCM/TEM of MSLN CART using flow cytometry.

Experimental instruments: automated cell counter (Invitrogen Countess II FL), microscope (Jiangnan XD-202), Pico17 desktop microcentrifuge (Thermo), flow cytometer (Navios, AZ46353)

Experimental Reagents

| Reagent | Manufacturer | Number |
|---|---|---|
| DPBS | Hyclone | SH30028. 02 |
| Anti-human CD3 PE-Cy5/CD4 PE/CD8 FITC Cocktail | Biolegend | 319001 |
| PE/Cy5 anti-human CD45RO | Biolegend | 304208 |
| FITC anti-human CD197 | Biolegend | 353216 |
| PE anti-human CD62L | Biolegend | 304806 |

Experimental Methods

1 MSLN CAR-T CD3/CD4/CD8 detection

For details of the experimental process of the anti-human CD3 PE-Cy5/CD4 PE/CD8 FITC cocktail, see experimental procedures for details.

2 MSLN CAR-T TCM/TEM detection

For details of the experimental process of the 2 μL PE/Cy5 anti-human CD45RO, 2 μL FITC anti-human CD197 and 2 μL PE anti-human CD62L flow cytometry antibodies, see experimental procedures for details.

Experimental results

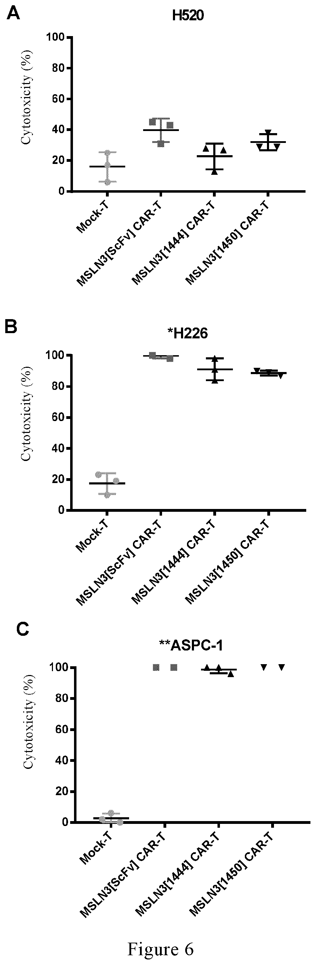

The results are shown in Table 4-5 and Figure 4-5. The results show that the CD3 positive ratio of CAR-T cells in the mock-T group and the nanoantibody mesothelin group (1444 and 1450) is high, about 85%-97%; the CD3 positive ratio of MSLN3[Scfv]CAR-T cells is slightly lower, about 75%-84%. The overall trend of the ratio of CD4 to CD8 in CD3 positive cells in all groups is consistent, and the CD4 ratio is higher than the CD8 ratio. The overall trend of the ratio of Tcm to Tem in all groups is consistent, and the Tcm ratio is higher than the Tem ratio. Compared with Mock-T cells, the CD4/CD8 and Tcm/Tem of MSLN[Scfv]CAR-T cells and MSLN3[1444]CAR-T cells both decrease to a certain extent (Figure 4, Figure 5).

Table 4, T cell Clustering of MSLN CAR-T detected on Day 13

| Sample number | Type | **CD3+** | **CD3+CD4 +** | **CD3+CD8 +** | **CD4+/CD8 +** |
|---|---|---|---|---|---|
| AC-201908B | Mock-T | 95.43 % | 79.04% | 17.13% | 4.61 |
| | MSLN3 [Scfv]CAR -T | 83.77 % | 69.02% | 23.55% | 2.93 |
| | MSLN3[1444]CA R-T | 90.27 % | 69.49% | 25.24% | 2.75 |
| | MSLN3[1450]CA R-T | 85.49 % | 70.27% | 25.24% | 2.78 |
| AC-201909A | Mock-T | 94.79 % | 84.00% | 14.06% | 5.97 |
| | MSLN3[Scfv]CAR -T | 75% | 55.83% | 35.83% | 1.56 |
| | MSLN3[1444]CA R-T | 89.13 % | 58.77% | 33.83% | 1.74 |
| | MSLN3[1450]CA R-T | 88.38 % | 55.58% | 37.84% | 1.47 |
| AC-201910A | Mock-T | 97.17 % | 82.54% | 12.30% | 6.71 |
| | MSLN3[Scfv]CAR -T | 83.78 % | 75.28% | 17.61% | 4.27 |
| | MSLN3[1444]CA R-T | 90.92 % | 71.54% | 21.28% | 3.36 |
| | MSLN3[1450]CA R-T | 90.61 % | 71.72% | 23.09% | 3.11 |

Table 5, Clustering of memory T cells in cells detected on Day 13

| Sample number | Type | **Tem** | **Tcm** |
|---|---|---|---|
| AC-201908B | Mock-T | 3.46% | 74.57% |
| | MSLN3[Scfv]CAR-T | 2.28% | 53.74% |
| | MSLN3[1444]CAR-T | 3.31% | 63.04% |
| | MSLN3[1450]CAR-T | 2.18% | 57.75% |

(continued)

| Sample number | Type | Tem | Tcm |
|---|---|---|---|
| AC-201909A | Mock-T | 2.73% | 76.56% |
| | MSLN3[Scfv]CAR-T | 3.15% | 36.38% |
| | MSLN3[1444]CAR-T | 3.87% | 56.12% |
| | MSLN3[1450]CAR-T | 1.82% | 61.14% |
| AC-201910A | Mock-T | 11.19% | 67.72% |
| | MSLN3[Scfv]CAR-T | 7.83% | 40% |
| | MSLN3[1444]CAR-T | 8.33% | 53.33% |
| | MSLN3[1450]CAR-T | 6.92% | 57.82% |

1.5 Detection of MSLN CAR-T cytotoxicity in vitro

This example studies the in vitro pharmacodynamic properties of MSLN CAR-T of different sequences, and detects the secretion of cytokines after co-culture with mesothelin-positive tumor cells and its specific killing ability of target cells. In vitro killing experiments are performed using mesothelin-negative tumor cells and mesothelin-positive tumor cells to prove that the candidate sequence has the ability to specifically kill the mesothelin antigen.

Experimental samples

AC-201908B, AC-201909A and AC-201910A shown in Table 2 and YP-201903A04, YP-201903A23 and YP-201903A24 obtained using the same method, their sample information is shown in the table below.

| Sample number | Type | Cell viability rate during recovery | CAR+ |
|---|---|---|---|
| YP-201903A0 4 | Mock-T | 55% | 0.54% |
| | MSLN3[ScFv] CAR-T | 51% | 76.02% |
| | MSLN3[1444] CAR-T | 52% | 84.45% |
| | MSLN3[1450] CAR-T | 62% | 90.07% |
| YP-201903A2 3 | Mock-T | 60% | 0.97% |
| | MSLN3[ScFv] CAR-T | 31% | 94.27% |
| | MSLN3[1444] CAR-T | 56% | 79.43% |
| | MSLN3[1450] CAR-T | 54% | 86.97% |
| YP-201903A2 4 | Mock-T | 57% | 0.21% |
| | MSLN3[ScFv] CAR-T | 39% | 72.68% |
| | MSLN3[1444] CAR-T | 37% | 81.4% |
| | MSLN3[1450] CAR-T | 29% | 86.24% |

Target cell information is shown in the table below:

| Cell Type | MSLN+ on the surface | Viability rate |
|---|---|---|
| H520 | 10.3% | 91% |
| AsPC-1 | 78.1% | 96% |
| H226 | 99.1% | 98% |
| SKOV3 | 75.4% | 96% |

Experimental instruments: automated cell counter (Invitrogen Countess II FL YSB195), microscope (Jiangnan XD-202 YSB126), biological safety cabinet, refrigerator (TCL), carbon dioxide incubator (Thermo), real-time cell analyzer (ACEA XCELLIgence RTCA TP, SB0475, supporting consumables: E-Plate 16, E-Plate 96), desktop centrifuge (small) (Thermo Fisher), desktop centrifuge (large) (Xiangyi).

Experimental Reagents

| Reagent | Manufacturer | Specification | Number |
|---|---|---|---|
| AIM-V culture medium | Gibco | 1000mL | A3021002 |
| Fetal bovine serum (FBS) | Gibco | 500mL | 10099141 |
| IL-2 | Shandong Quangang Pharmaceutical Co., Ltd. | 2 million U/unit | National medicine permission number 20220004 |
| Trypan Blue Stain (0.4%) | Invitrogen | 100mL | 15250-061 |
| DMEM cell culture medium | CORNING | 500mL/bottle | 10-013-CER |
| 0.25% trypsin digestion solution | New Cell & Molecular Biotech Co., Ltd. | 125mL | C125C1 |
| Human IL-2 ValukineTM ELISA KIT | NOVUS | 96 well | VAL110 |
| Human TNF-$\alpha$ ValukineTM ELISA KIT | NOVUS | 96 well | VAL105 |
| Human IFN-$\gamma$ ValukineTM ELISA KIT | NOVUS | 96 well | VAL104 |

Experimental Methods

1) Cell killing

(1) YP-201903A04, YP-201903A23 and YP-201903A24: Target cells are H520, AsPC -1, H226, effector-target ratio (E:T) =1:1
(2) AC-201908B, AC-201909A and AC-201910A: Target cells: SKOV3, H226, effector-target ratio (E:T) = 1:1, 1:4, 1:16

$$\text{Effector cell killing rate at 72 hours (\%)} = \frac{\text{Cell index of group Tumor-Cell index of the test group}}{\text{Cell index of the test group}} \times 100$$

Cell index: cell index reflects the growth status and cell number of tumor cells.
For details of the experimental process, see experimental procedures for details.

2) Cytokine release assay

Incubation with target cells for 72 h, the 96-well plate is taken out and the release of cytokine in the supernatant is detected by ELISA.

Experimental results

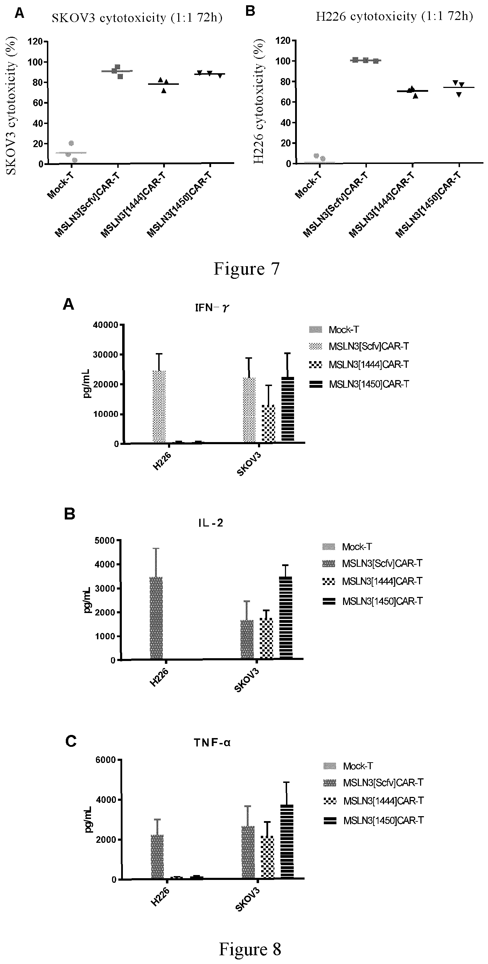

The results are shown in Figure 6-8 and Table 6. The results show that CAR-T cells prepared by our transposase plasmid system have the function of specifically killing mesothelin-positive tumor cells (Figure 6, Figure 7). Co-culture with mesothelin-positive tumor cells can effectively stimulate MSLN3[Scfv]CAR-T cells and MSLN3[1444]CAR-T cells to secrete IFN-y, IL-2 and TNF-α (Figure 8).

Table 6, The release of cell cytokines of mock-T and MSLN CAR-T after 72h of co-incubation with mesothelin-positive tumor cells

| Sample number | Type | Skov3共孵育 | | | H226共孵育 | | |
|---|---|---|---|---|---|---|---|
| | | IL-2 | IFN-γ | TNF-α | IL-2 | IFN-γ | TNF-α |
| AC-2019 08B | Mock-T | 0 | 0 | 0.3953 1 | 0 | 0 | 0 |
| | MSLN3[Scfv ] CAR-T | 1763.4 579 | 18290 .34 | 2334.6 3075 | 2284.0 818 | 19403 .37 | 1599.2 1927 |
| | MSLN3[144 4] CAR-T | 1886.2 7515 | 17225 .99 | 2694.7 3065 | 0 | 364.8 497 | 87.820 08 |
| | MSLN3[145 0] CAR-T | 3751.5 672 | 29442 .08 | 4236.6 2589 | 0 | 378.5 243 | 90.680 46 |
| AC-2019 09A | Mock-T | 0 | 0 | 0 | 0 | 0 | 0 |
| | MSLN3[Scfv ] CAR-T | 2360.2 263 | 29623 .99 | 3753.9 324 | 4683.2 562 | 30534 .71 | 3078.9 8445 |
| | MSLN3[144 4] CAR-T | 1942.3 879 | 16011 .21 | 2381.6 385 | 0 | 667.5 305 | 118.16 064 |
| | MSLN3[145 0] CAR-T | 3674.9 1765 | 23115 .72 | 4447.6 6734 | 0 | 646.3 277 | 188.42 688 |
| AC-2019 10A | Mock-T | 0 | 0 | 0 | 0 | 0 | 0 |
| | MSLN3[Scfv ] CAR-T | 796.91 285 | 17961 .66 | 1831.0 9569 | 3360.6 87 | 23405 .68 | 1946.1 5112 |
| | MSLN3[144 4] CAR-T | 1348.8 5065 | 5177. 633 | 1339.3 4217 | 0 | 418.6 903 | 115.26 768 |
| | MSLN3[145 0] CAR-T | 2885.7 848 | 13585 .77 | 2393.5 0866 | 0 | 363.0 271 | 123.16 485 |

The above results show that the MSLN CAR sequence derived from nanoantibodies has similar or better proliferation ability, MSLN CAR positive rate, CD4+T/CD8+T clustering ratio, and memory T cell ratio in vitro screening compared with the single-chain antibody sequence. It has the ability to specifically kill mesothelin-positive tumor cells.

Example 2, in vitro experiment of MSLN CAR-T cells secreting PD-1 nanoantibodies

Plasmids are constructed by using the PD1 antibody sequence derived from the nanoantibody and the sequence of the benchmark Keytruda, and the gene is transferred into PBMC derived from healthy people through a dual-plasmid electroporation system to construct non-viral vector aPD1 MSLN CAR-T cells. By comparing the in vitro performance of these CAR-T cells, including proliferation ability, CAR positive rate, antibody secretion level, and the blocking effect of secreted antibodies on immune checkpoint inhibitors, the function of CAR-T cell secreting antibodies is verified.

2.1 Plasmid construction of self-secreting PD1 nanoantibodies

An exemplary PD1 nanoantibody (aPD1[1182] , SEQ ID NO: 127) is used as the target fragment, and pS338B (see CN111206043A) vector is used as the backbone vector for double enzyme digestion (EcoR1 and BamH1). The remaining experimental instruments, experimental reagents, and experimental methods are the same as those in section 1.1 of Example 1.

2.2 Preparation of MSLN CAR-T cells secreting PD1 nanoantibodies

Mock-T and aPD1-MSLN CAR-T are prepared by electroporation, and cells and cell supernatant samples are obtained for detection and analysis.

The experimental instruments, reagents and methods are the same as those in part 1.2 of Example 1. Among them, aPD1-MSLN CAR-T is a CAR-T cell electroporated with dual plasmids (the plasmids constructed by part 1.1 of Example 1 and part 2.1 of Example 2), which expresses the MSLN CAR and PD1 nanoantibodies. When single plasmid electroporation is performed, the plasmid concentration is 6 μg per sample; when dual plasmid electroporation was performed, the concentration of each plasmid is 4 μg per sample.

Experimental results

| Donor number | Cell Group | Plasmid | Plasmid concentration (μg/μL) |
|---|---|---|---|
| AC-201906 A | Mock-T | pNB338B-MCS | 4.128 |
| | MSLN3[1444]CAR-T | pNB338B-1444-8E | 3.6 |
| AC-201908 A<br>AC-201909 A | aPD1[1182]-MSLN3[144 4]CAR-T | pNB338B-1444-8E | 3.6 |
| | | pS338B-aPD1-VHH | 3.549 |

2.3 Detection of the in vitro proliferation and positive rate of MSLN CAR-T cells

To verify whether MSLN CAR-T cells can achieve normal cell proliferation and ensure a certain MSLN CAR positive rate under the dual-plasmid system when they can auto secrete PD1 (aPD1-MSLN CAR-T), and the cells and cell supernatant samples are obtained for detecting the secretion of PD -1 antibodies by the cells. The experimental instruments, reagents and methods are the same as those in part 1.3 of Example 1.

Experimental results

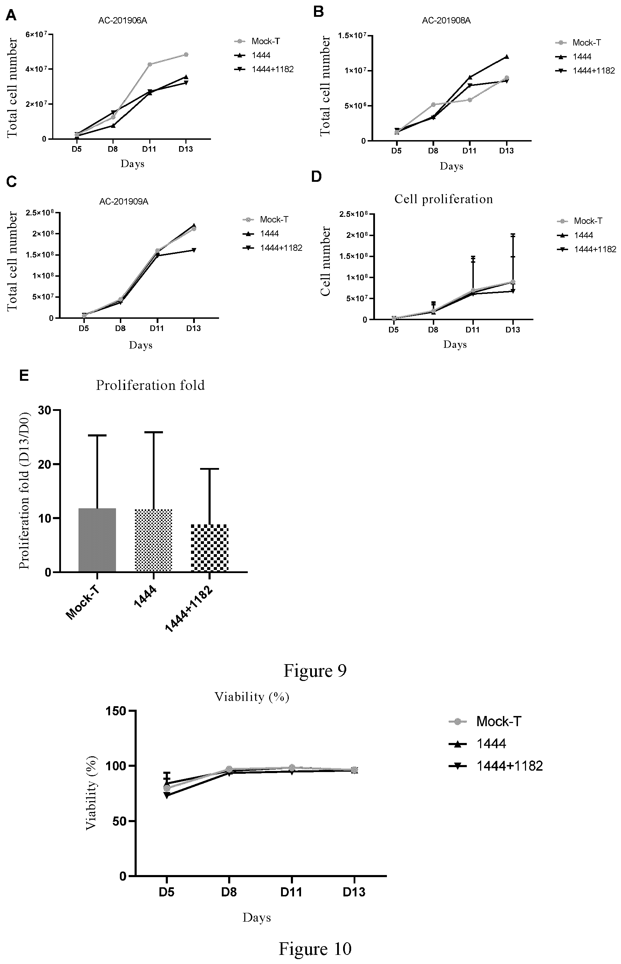

The results are shown in Table 7-9 and Figure 9-11.

Table 7: The total number of cells proliferated and the proliferation multiple of cells from D5 to D13

| Sample number | Type | Day5 | Day8 | Day11 | Day13 | Proli ferati on multi ple |
|---|---|---|---|---|---|---|
| AC-201906A | Mock-T | 2.43E+0 6 | 1.24E+07 | 4.27E+0 7 | 4.84E+07 | 7.36 |
| | MSLN3[1444]CAR -T | 1.67E+0 6 | 7.62E+06 | 2.64E+0 7 | 3.56E+07 | 5.42 |
| | aPD1[1182]-MSLN 3 [1444] CAR-T | 2.70E+0 6 | 1.51E+07 | 2.72E+0 7 | 3.21E+07 | 4.88 |
| AC-201908A | Mock-T | 1.25E+0 6 | 5.17E+06 | 5.86E+0 6 | 9.02E+06 | 1.13 |
| | MSLN3[1444]CAR -T | 1.26E+0 6 | 3.44E+06 | 9.07E+0 6 | 1.20E+07 | 1.50 |
| | aPD1[1182]-MSLN 3 [1444] CAR-T | 1.54E+0 6 | 3.31E+06 | 7.88E+0 6 | 8.54E+06 | 1.07 |
| AC-201909A | Mock-T | 5.61E+0 6 | 4.46E+07 | 1.60E+0 8 | 2.12E+08 | 26.97 |
| | MSLN3[1444]CAR -T | 6.83E+0 6 | 4.22E+07 | 1.57E+0 8 | 2.20E+08 | 27.94 |
| | aPD1[1182]-MSLN 3 [1444] CAR-T | 6.76E+0 6 | 3.67E+07 | 1.48E+0 8 | 1.61E+08 | 20.51 |

Table 8, The cell viability rate of cells cultured from D5 to D13.

| Sample number | Type | Day5 | Day8 | Day1 1 | Day1 3 |
|---|---|---|---|---|---|
| AC-201906 A | Mock-T | 70% | 81% | 91% | 91% |
| | MSLN3[1444]CAR-T | 62% | 85% | 92% | 92% |
| | aPD1[1182]-MSLN3[1 444]CAR-T | 80% | 87% | 86% | 86% |
| AC-201908 A | Mock-T | 57% | 94% | 85% | 85% |
| | MSLN3[1444]CAR-T | 55% | 91% | 86% | 86% |
| | aPD1[1182]-MSLN3[1 444]CAR-T | 68% | 74% | 86% | 86% |
| AC-201909 A | Mock-T | 88% | 94% | 94% | 94% |
| | MSLN3[1444]CAR-T | 89% | 95% | 93% | 93% |
| | aPD1[1182]-MSLN3[1 444]CAR-T | 87% | 91% | 91% | 91% |

Table 9, Changes of the MSLN CAR-T positive rate and the amount of CAR+ cells obtained of cells cultured from D8 to D13

| Sample number | Type | Day8 | Day11 | Day13 | The number of CAR-T cells |
|---|---|---|---|---|---|
| AC-201906A | Mock-T | 0.58% | 0.10% | 0.40% | - |
| | MSLN3[1444]CAR-T | 75.68% | 80.56% | 82.20% | 2.93E+07 |
| | aPD1[1182]-MSLN3 [1444]CAR-T | 67.54% | 70.57% | 76.56% | 2.46E+07 |
| AC-201908A | Mock-T | 0.26% | 0.28% | 1.40% | - |
| | MSLN3[1444]CAR-T | 76.99% | 86.02% | 86.26% | 1.03E+07 |
| | aPD1[1182]-MSLN3 [1444]CAR-T | 73.24% | 83.48% | 81.18% | 6.93E+06 |
| AC-201909A | Mock-T | 0.14% | 0.12% | 0.29% | - |
| | MSLN3[1444]CAR-T | 54.72% | 70.16% | 71.07% | 1.56E+08 |
| | aPD1[1182]-MSLN3 [1444]CAR-T | 51.60% | 65.33% | 66.73% | 1.08E+08 |

2.4 Cell phenotype detection

Purpose

The phenotype of the CART cells we prepared was evaluated by detecting the CD3/CD4/CD8 and TCM/TEM of MSLN CART secreting PD1 antibody by flow cytometry.

The experimental instruments, reagents and methods are the same as those in part 1.4 of Example 1.

Experimental results

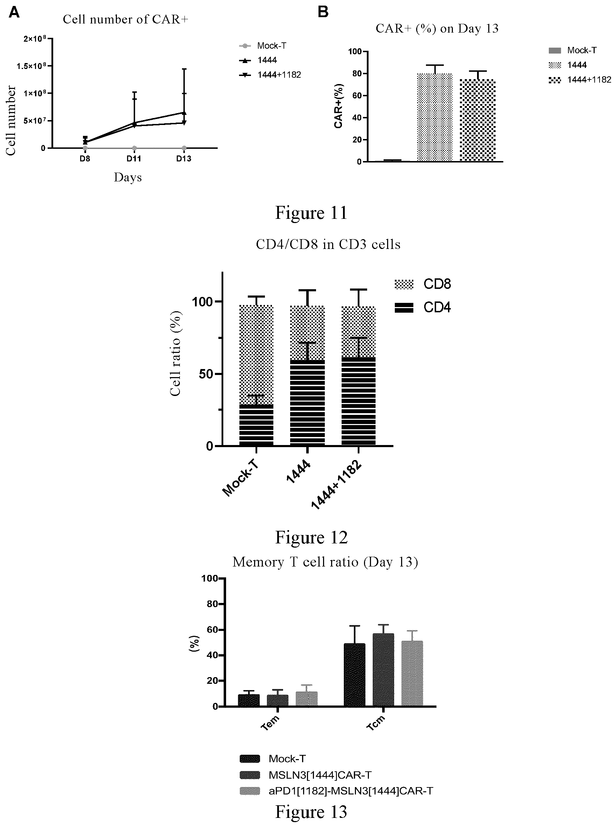

The results are shown in Table 10-11 and Figure 12-13 (AC-201908A).

Table 10, T cell Clustering of MSLN CAR-T detected on Day 13

| Sample number | Type | CD3+ | CD3+CD4 + | CD3+CD8 + | CD4+/CD 8+ |
|---|---|---|---|---|---|
| AC-2019 06A | Mock-T | 82.39 % | 31.99% | 61.41% | 0.52 |
| | MSLN3[1444]CA R-T | 77.60 % | 57.17% | 37.64% | 1.52 |
| | aPD1[1182]-MSL N3[1444]CAR-T | 75.69 % | 41.67% | 50.96% | 0.82 |
| AC-2019 08A | Mock-T | 88.92 % | 20.10% | 75.52% | 0.27 |
| | MSLN3[1444]CA R-T | 75.58 % | 34.34% | 60.21% | 0.57 |
| | aPD1[1182]-MSL N3[1444]CAR-T | 78.61 % | 34.82% | 59.11% | 0.59 |
| AC-2019 09A | Mock-T | 84.75 % | 19.72% | 76.71% | 0.26 |
| | MSLN3[1444]CA R-T | 78.33 % | 32.50% | 62.39% | 0.52 |
| | aPD1[1182]-MSL N3[1444]CAR-T | 75.34 % | 30.06% | 64.88% | 0.46 |

Table 11, Clustering of memory T cells in MSLN CAR-T on D13

| Sample number | Type | Tcm | Tem |
|---|---|---|---|
| AC-201906A | Mock-T | 59.80% | 8.58% |
| | MSLN3[1444]CAR-T | 64.62% | 5.24% |
| | aPD1[1182]-MSLN3[1444]CAR-T | 58.45% | 8.86% |
| AC-201908A | Mock-T | 35.96% | 12.12% |
| | MSLN3[1444]CAR-T | 49.48% | 13.70% |
| | aPD1[1182]-MSLN3[1444]CAR-T | 41.26% | 17.50% |
| **AC-201909A** | **Mock-T** | 45.02% | 9.49% |
| | **MSLN3[1444]CAR-T** | 55.15% | 6.78% |
| | **aPD1[1182]-MSLN3[1444]CAR-T** | 52.23% | 6.98% |

2.5 Cell-secreting antibody detection

During the cell preparation process, the ELISA method is used to detect whether the cells have the ability to secrete antibodies and the range of secreted antibody concentration before the end of preparation.

Experimental instrument: Multifunctional microplate reader (TECAN Spark)

Experimental Reagents

| Reagent | Manufacturer | Number |
|---|---|---|
| Disposable sterile sample tank | Coming | 4870 |
| 96-well single strip detachable ELISA plate | costar | 42592 |
| bovine serum albumin | SIGMA | B2064-100G |
| Sodium chloride | Sinopharm Chemical Reagent Co., Ltd. | 10019318 |
| Potassium chloride | Sinopharm Chemical Reagent Co., Ltd. | 10016308 |
| Sodium bicarbonate | Sinopharm Chemical Reagent Co., Ltd. | 10020318 |
| Potassium dihydrogen phosphate | Sinopharm Chemical Reagent Co., Ltd. | 10017618 |

(continued)

| Reagent | Manufacturer | Number |
|---|---|---|
| Sodium carbonate | Sangon Biotech | A100585-0500 |
| Sodium bicarbonate | Sangon Biotech | A100865-0500 |
| Twain | Shanghai Sangon Biotech Co., Ltd. | A100777-0500 |
| Commercial DPBS 1X | HyClone | SH30028.02 |
| TMB ELISA Substrate (Fast Kinetic Rate) | abcam | ab171524 |
| Streptavidin (HRP) | abcam | Ab7403 |
| Human PD-1 / PDCD1 Protein (HPLC- verified) | Aero | PD1-H5221 |
| MonoRabTM rabbit antibody Biotin, mAb | GenScript | A01995-200 |

Experimental Methods

The cell supernatant sample of $5\times10^5$ cells cultured for two days was taken, the concentration of antibody secretion in the supernatant was detected by ELISA, and the concentration of amount-of-substance secreted by the cells in the supernatant was calculated. See experimental procedures for details.
The calculation formula is: the concentration of amount-of-substance = mass concentration/molecular weight kDa/1000
In this experiment, the cell supernatant samples are set to 4-fold, 40-fold, and 400-fold dilutions.

Experimental results

The results are shown in Table 12, which indicate that the mesothelin-targeted CAR-T cells that secret the PD1 nanoantibody can successfully secrete the PD1 nanoantibody during the preparation process, and the secretion capacity of the PD 1 nanoantibody tends to decrease over time.

Table 12, Antibody secretion concentrations on D11-13 after cell preparation

| Sample number | Type | Day11-13 (ng/mL) | Day11-13 (nmol/mL) |
|---|---|---|---|
| AC-201906A | Mock-T | NA | NA |
| | MSLN3[1444]CAR-T | NA | NA |
| | aPD1[1182]-MSLN3[1444]C AR-T | 177.59 | 0.0118 |
| AC-201908A | Mock-T | NA | NA |
| | MSLN3[1444]CAR-T | NA | NA |
| | aPD1[1182]-MSLN3[1444]C AR-T | 145.80 | 0.0097 |
| AC-201909A | Mock-T | NA | NA |
| | MSLN3[1444]CAR-T | NA | NA |
| | aPD1[1182]-MSLN3[1444]C AR-T | 206.09 | 0.0137 |

2.6 Cell function detection

By using the antigen targeted by the CAR-T cells to stimulate the prepared CAR-T cells to a certain stimulation, changes of cell phenotype and antibody secretion are detected, and the responsiveness of CAR-T cells after being stimulated by specific antigens can be evaluated.
The experimental instruments, reagents and methods are the same as those in part 1.4 of Example 1.

Experimental Methods

12-well cell culture plates coated with 5 µg/mL mesothelin antigen, $5\times10^5$ cell samples were added and cultured in 1 mL of culture system for 48h. Cells and cell supernatants were collected for flow cytometry and ELISA detection, respectively. The ratio of Tcm (CD45RO+CCR7+CD62L+) and Tem (CD45RO+CCR7-CD62L-) and the secretion of PD1 nanoantibodies are analyzed according to the analysis methods of 2.4 and 2.5.

Experimental results

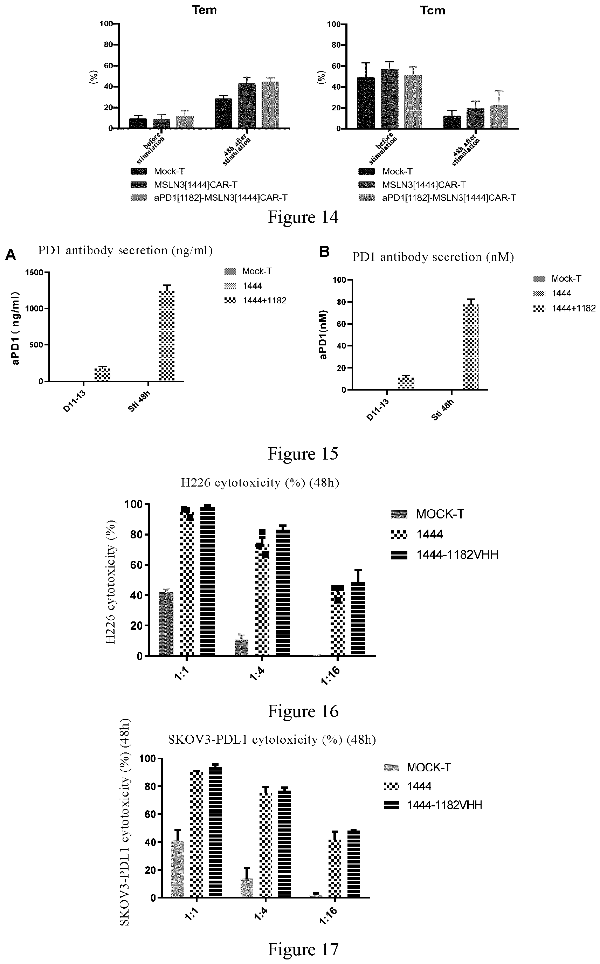

The results are shown in Table 13-14 and Figure 14-15. The results show that after CAR-T cells are stimulated by antigens, the proportion of Tcm decreased while the proportion of Tem increased; after CAR-T cells are stimulated by antigens, the secretion of PD1 nanoantibodies is significantly increased compared with before stimulation.

Table 13, Clustering ratio of memory T cells in cells after mesothelin antigen stimulation for 48 hours

| Sample number | Type | Tem (48h after stimulation) | Tcm (48h after stimulation) |
|---|---|---|---|
| AC-201906A | Mock-T | 27.98% | 18.13% |
| | MSLN3[1444]CAR-T | 45.90% | 25.01% |
| | aPD1[1182]-MSLN3[1 444]CAR-T | 42.27% | 18.19% |
| AC-201908A | Mock-T | 31.41% | 7.13% |
| | MSLN3[1444]CAR-T | 46.54% | 11.46% |
| | aPD1[1182]-MSLN3[1 444]CAR-T | 49.28% | 10.35% |
| AC-201909A | Mock-T | 23.95% | 9.64% |
| | MSLN3[1444]CAR-T | 34.36% | 21.44% |
| | aPD1[1182]-MSLN3[1 444]CAR-T | 41.15% | 37.81% |

Table 14, Antibody concentration in cell supernatant after mesothelin antigen stimulation for 48h

| Sample number | Type | 48h after stimulation (ng/mL) | 48h after stimulation (nmol/mL) |
|---|---|---|---|
| AC-201906A | Mock-T | NA | NA |
| | MSLN3[1444]CAR-T | NA | NA |
| | aPD1[1182]-MSLN3[1444] CAR-T | 1333.54 | 0.0889 |
| AC-201908A | Mock-T | NA | NA |
| | MSLN3[1444]CAR-T | NA | NA |
| | aPD1[1182]-MSLN3[1444] CAR-T | 1219.256 | 0.0813 |
| AC-201909A | Mock-T | NA | NA |
| | MSLN3[1444]CAR-T | NA | NA |
| | aPD1[1182]-MSLN3[1444] CAR-T | 1174.064 | 0.078271 |

2.7 aPD1-MSLN CAR-T in vitro cytotoxicity assay

To validate the in vitro pharmacodynamic properties of MSLN CAR-T cells that secret PD-1 antibodies, CAR-T cells were evaluated by detecting the secretion of cytokines and specific killing ability against target cells thereof after co-culture with tumor cells that positively expressed mesothelin and highly expressed PDL1.

Effector cells

| Sample number | Type | cell viability rate | CAR+ |
|---|---|---|---|
| AC-201906A | Mock-T | 94% | 0.40% |
| | MSLN3[1444]CAR-T | 88% | 82.20% |
| | aPD1[1182]-MSLN3[1444]CAR-T | 88% | 76.56% |
| AC-201908A | Mock-T | 86% | 1.40% |
| | MSLN3[1444]CAR-T | 88% | 86.26% |
| | aPD1[1182]-MSLN3[1444]CAR-T | 87% | 81.18% |
| AC-201909A | Mock-T | 97% | 0.29% |
| | MSLN3[1444]CAR-T | 94% | 71.07% |
| | aPD1[1182]-MSLN3[1444]CAR-T | 92% | 66.73% |

Target cells

| Cell Type | MSLN+ on the surface | PDL1 on the surface | Viability rate |
|---|---|---|---|
| SKOV3-PDL1 | 72.3% | 99.9% | 96% |
| H226 | 98.3% | 37.7% | 96% |

The experimental instruments, reagents and methods are the same as those in part 1.5 of Example 1. The target cells selected are SKOV3-PDL1, H226, and the effector-target ratio (E:T) is 1:1, 1:4, 1:16.

In the cytokine release test, all experimental samples of IL-2 are uniformly diluted 5 times and 10 times before testing; all experimental samples of TNF-$\alpha$ are uniformly diluted 3 times before testing; all experimental samples of IFN-$\gamma$ are uniformly diluted 10 times and 100 times before testing.

Experimental results

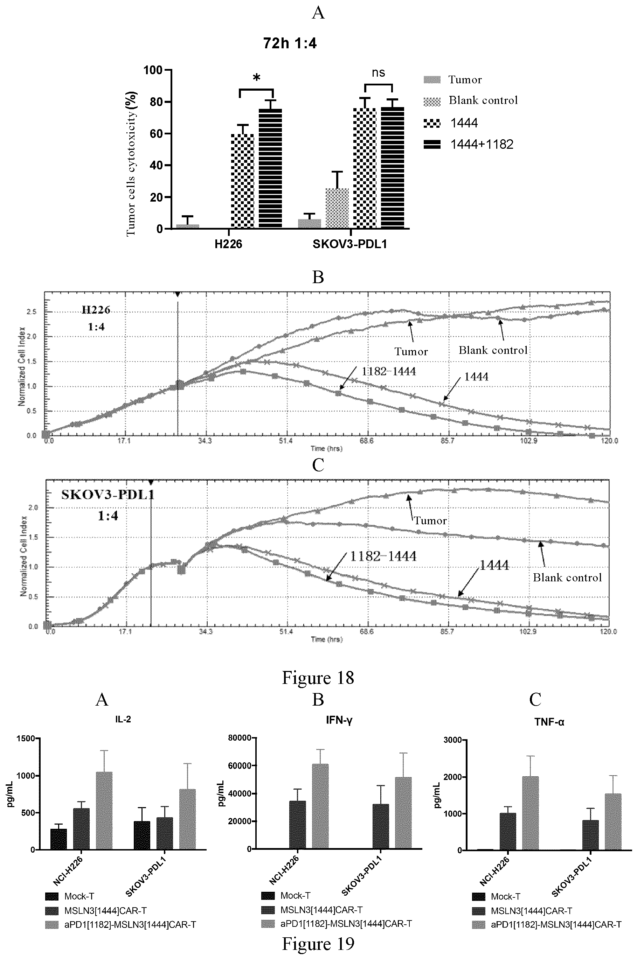

The results are shown in Figure 16-18 and Table 15. The results show that the self-secreting PD1 antibody can significantly improve the killing efficiency of mesothelin-targeted CAR-T on H226 cells (Figure 16), and has no significant effect on the killing efficiency of SKOV3-PDL1 cells (Figure 17). This shows that the mesothelin-targeted CAR-T cells with the self-secreting PD1 nanoantibody we prepared can effectively kill mesothelin-positive tumor cells, and can improve the killing effect of mesothelin-targeted CAR-T on certain types of mesothelin and PDL1 double-positive tumor cells. Co-culture with H226 cells and Skov3-PDL1 tumor cells can improve the ability of CAR-T cells to secrete cytokines IFN-$\gamma$ and TNF-$\alpha$; moreover, compared with mesothelin cells that do not secrete PD1 antibodies, the secretion of IFN-$\gamma$ and TNF-$\alpha$ is significantly increased after co-culture mesothelin-targeted CAR-T cells that secret PD1 antibodies with tumor cells (Figure 19).

Table 15, The release of cell cytokines of mock-T and MSLN CAR-T after co-incubation with mesothelin&PDL1-positive tumor cells for 48h

| Sample number | Type | co-incubation with Skov3-PDL1 | | | co-incubation with H226 | | |
|---|---|---|---|---|---|---|---|
| | | IL-2 | IFN-$\gamma$ | TNF-$\alpha$ | IL-2 | IFN-$\gamma$ | TNF-$\alpha$ |
| AC-201906A | Mock-T | 594.0 | 4.2 | 59.3 | 353.3 | 8.7 | 115.3 |
| | MSLN3[1444]CAR-T | 501.0 | 440.0 | 16969.5 | 470.0 | 803.5 | 26278.6 |
| | aPD1[1182]-MSLN3[1444]CAR-T | 481.7 | 957.8 | 31420.7 | 709.2 | 1432.9 | 53035.6 |

(continued)

| Sample number | Type | co-incubation with Skov3-PDL1 | | | co-incubation with H226 | | |
|---|---|---|---|---|---|---|---|
| | | IL-2 | IFN-$\gamma$ | TNF-$\alpha$ | IL-2 | IFN-$\gamma$ | TNF-$\alpha$ |
| AC-201908A | Mock-T | 310.4 | 5.0 | 46.8 | 210.2 | 20.0 | 367.4 |
| | MSLN3[1444]CAR-T | 535.7 | 1115.5 | 44080.5 | 660.5 | 1180.9 | 33064.4 |
| | aPD1[1182]-MSLN3[1444]CAR-T | 1175.1 | 1751.8 | 64091.2 | 1242.1 | 2045.8 | 55925.7 |
| AC-201909A | Mock-T | 227.3 | 12.2 | 273.6 | 262.3 | 16.3 | 117.4 |
| | MSLN3[1444]CAR-T | 246.4 | 860.8 | 34873.5 | 523.6 | 1023.8 | 43705.9 |
| | aPD1[1182]-MSLN3[1444]CAR-T | 775.5 | 1895.1 | 58957.2 | 1183.4 | 2542.9 | 73180.5 |

Example 3: in vivo efficacy experiments of MSLN CAR-T cells secreting D1 nanoantibodies

3.1 Experimental animals and cells

6-8 weeks NPSG severe combined immune deficiency mice prkdc[scid]Il2rg[null] (Beijing Phenotek Biotechnology Co., Ltd. )

Tumor cells

Skov3-PDL1 (ATCC) that highly expressed PDL1
NCI-H226-PDL1 (ATCC) that highly expressed PDL1

The process of constructing CAR-T cells self-secreting Fc-containing PD1 antibodies: The aPD1[1182] nanoantibody sequence was constructed into the pCDNA3.4-IgG4 vector to obtain the pCDNA3.4-1182-Fc vector, which was then expressed by the ExpiCHOTM (Thermo Fisher) expression system. After one week of expression, the supernatant was collected for protein A (GE) purification. Then Nanodrop was used to detect the protein quality, and HPLC was used to detect the protein purity. The 1182-Fc coding sequence was obtained from the pCDNA3.4-1182-FC vector by double enzyme digestion and introduced into the pS338B backbone vector. The remaining experimental instruments, experimental reagents, and experimental methods were the same as those in Example 2.

3.2 Vaccination and administration regimen

NPSG severe combined immunodeficient mice are inoculated subcutaneously with tumor cells.

Table 16, Administration regimen and grouping

| Mouse | Num ber | Seed cells | Dose (10^6) | Volume ($\mu$L) | Ad min istra tion rout e | Positi on | Comment |
|---|---|---|---|---|---|---|---|
| NPSG | 85 | Skov3-PDL 1 | 10 | 200 | Sub cuta neo us | Uppe r tight | Matrix 50% |
| NPSG | 85 | NCI-H226-PDL1 | 10 | 200 | Sub cuta neo us | Uppe r tight | Matrix 50% |

When the tumor volume reaches at 100-150 mm$^3$, the administration was started. The administration regimen is implemented according to Table 17.

Table 17, Administration regimen and grouping of NPSG mice

| Group | Number of mice | medicament | Dose (10^6) | Administration volume ($\mu$l) | Administration route | Frequency of administration |
|---|---|---|---|---|---|---|
| A1 | 6 | CD19-1182-VHH | 0.05 | 200 | Vein | Single |
| A2 | 6 | CD19-1182-VHH | 0.5 | 200 | Vein | Single |
| A3 | 6 | CD19-1182-VHH | 5 | 200 | Vein | Single |
| A4 | 6 | 1444 | 0.05 | 200 | Vein | Single |
| A5 | 6 | 1444 | 0.5 | 200 | Vein | Single |
| A6 | 6 | 1444 | 5 | 200 | Vein | Single |
| A7 | 6 | 1444-1182-VHH | 0.05 | 200 | Vein | Single |
| A8 | 6 | 1444-1182-VHH | 0.5 | 200 | Vein | Single |
| A9 | 6 | 1444-1182-VHH | 5 | 200 | Vein | Single |
| A10 | 6 | 1444-1182-FC | 0.05 | 200 | Vein | Single |
| A11 | 6 | 1444-1182-FC | 0.5 | 200 | Vein | Single |
| A12 | 6 | 1444-1182-FC | 5 | 200 | Vein | Single |

Among the medicament in Table 17, CD19-1182-VHH refers to a CD19-targeted CAR-T that secretes 1182-VHH, the amino acid sequence and sequence encoding CAR are shown in SEQ ID NO:9 and SEQ ID NO: 10 of CN109971712A. 1444 refers to MSLN3[1444]CAR-T, 1444-1182-VHH refers to aPD1[1182]-MSLN3[1444]CAR-T, and 1444-1182-FC refers to aPD1[1182-Fc]-MSLN3[1444]CAR-T, respectively.

3.3 Changes of tumor volume at high, medium and low doses

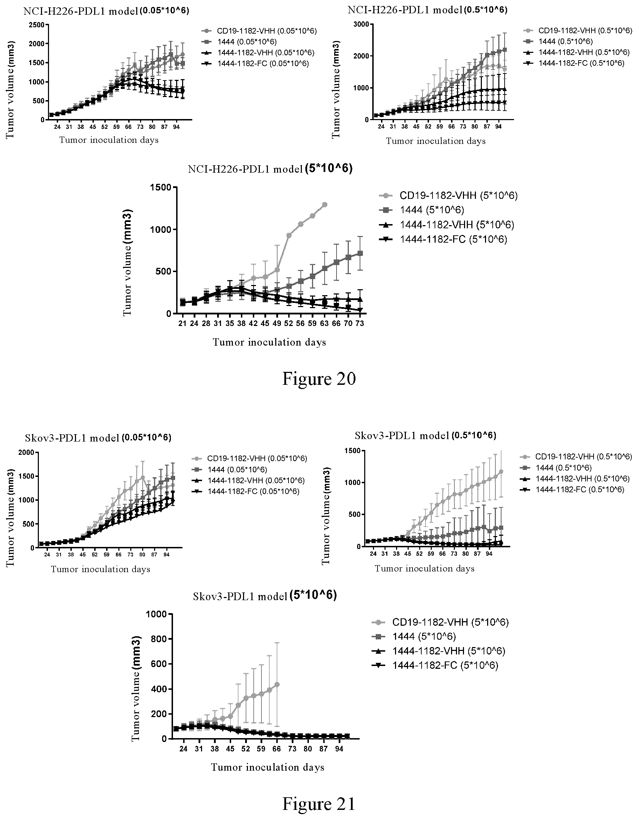

The results of H226 are shown in Figure 20. The self-secreting PD1 antibody administration groups show significant differences in efficacy at multiple doses, including the 0.05*10^6 dose group (1444 VS 1444-1182-VHH: P=0.0283, 1444 VS 1444-1182-FC: P=0.0010) , the 0.5*10^6 dose group (1444 VS 1444-1182-VHH: P=0.1722, 1444 VS 1444-1182-FC: P=0.0104), and the 5*10^6 dose group (1444 VS 1444-1182-VHH: P=0.0051, 1444 VS 1444-1182-FC: P=0.0006).

The results of Skov3 are shown in Figure 21. The self-secreting PD1 antibody administration groups show significant differences in efficacy at multiple doses, including the 0.05*10^6 dose group (1444 VS 1444-1182-VHH: P=0.7479, 1444 VS 1444-1182-FC: P=0.0757), the 0.5*10^6 dose group (1444 VS 1444-1182-VHH: P=0.0002, 1444 VS 1444-1182-FC: P<0.0001).

3.4 Changes of animal body weights at high, medium and low doses

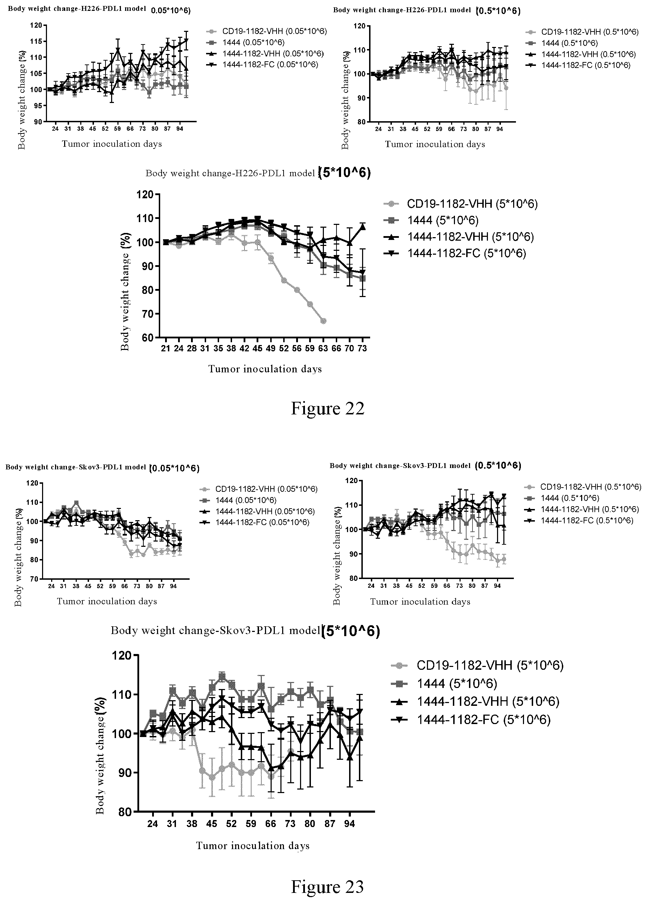

The results of H226 are shown in Figure 22. The weight change of the self-secreting PD1 antibody administration groups decreased less than that of the other groups, and is relatively stable in the long term. Except for the high-dose administration group, all groups remained stable or increased. Overall, the weight loss of the self-secreting PD1 antibody group does not exceed 20%.

The results of Skov3 are shown in Figure 23. The weight change of the self-secreting PD1 antibody administration groups decreased less than that of the other groups, and is relatively stable in the long term. Except for the low-dose administration group, all groups remained stable or increased. Overall, the weight loss of the group of self-secreting PD1 antibody does not exceed 20%.

3.5 Changes of survival rates at high, medium and low doses

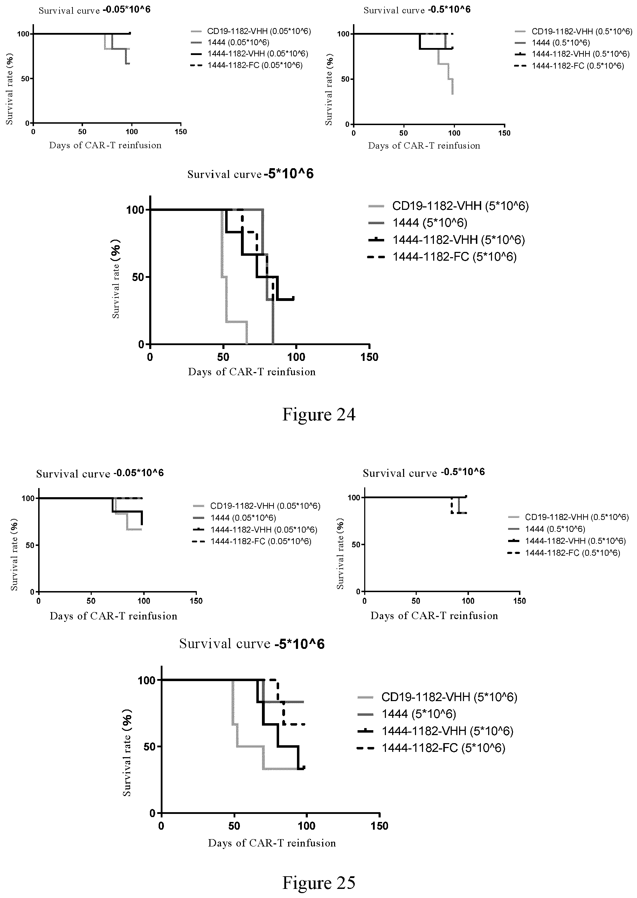

The results of H226 are shown in Figure 24. The survival period of the self-secreting PD1 antibody administration group is significantly better than that of the other groups, and is relatively stable in the long term. The mortality rate increases slightly with the increase of the dosage.

The results of Skov3 are shown in Figure 25. The survival period of the self-secreting PD1 antibody administration group is significantly better than that of the other groups, and is relatively stable in the long term. The mortality rate increases slightly with the increase of the dosage.

3.6 Changes of plasma concentrations of the PD1 antibody at high, medium and low doses

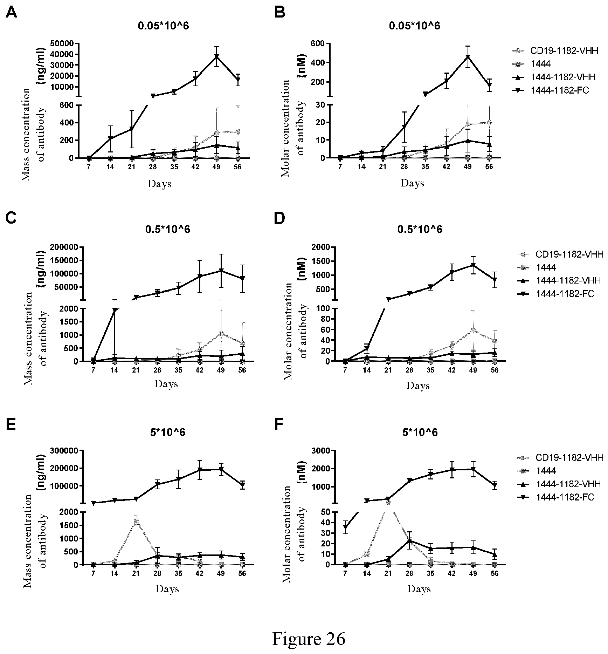

The results of H226 are shown in Figure 26. The plasma concentrations of the PD1 antibody of the self-secreting PD1 antibody administration groups is positively correlated with the dosage, and the blood concentration of the PD1 antibody carrying $F_c$ segment is significantly higher than that of the PD1 antibody without $F_c$ segment, and the half-life of the PD1 antibody carrying $F_c$ segment is longer in vivo.

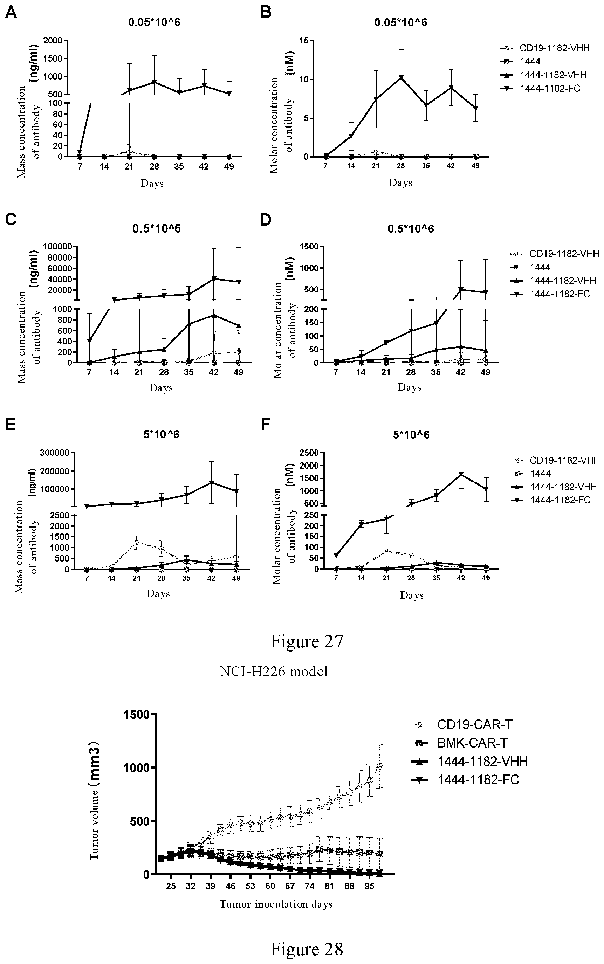

The results of Skov3 are shown in Figure 27. The plasma concentration of the PD1 antibody of the self-secreting PD1 antibody administration groups is positively correlated with the dosage, and the blood concentration of the PD1 antibody carrying $F_c$ segment is significantly higher than that of the PD1 antibody without $F_c$ segment, and the half-life of the PD1 antibody carrying $F_c$ segment is longer in vivo.

Example 4, High-dose safety of MSLN CAR-T cells secreting PD1 nanoantibodies

4.1 Experimental Materials

Experimental animals and cells: 6-8 weeks MHC-I KO B-NDG severe combined immune deficiency mice prkdc[scid]Il2rg[null] (Beijing Biocytogen Biotechnology Co., Ltd.) MHC-I knockout can minimize the graft-versus-host disease (GVHD) produced after CAR-T transfusion, which is conducive to long-term observation of the safety of the medicament.

Tumor cell: NCI-H226-PDL1 (ATCC) that highly express PDL1
Experimental process is the same as in Example 3.

4.2 Inoculation and administration regimen

Table 18, Inoculation regimen

| Group | Numbe r of mice | Inoculate cells | Dose (10^6 ) | Volu me (μL) | Administrati on route | Positio n | Com ment |
|---|---|---|---|---|---|---|---|
| -- | 40 | NCI-H226-PD L1 | 10 | 200 | Subcutaneou s | Upper tight | Matri x 50% |

Table 19, Administration regimen and grouping

| Group | Mouse Numb er | medicament | Dose (10^6 ) | Administrati on volume (μl) | Administrati on route | Frequency of administrati on |
|---|---|---|---|---|---|---|
| 1 | 5 | CD19-CAR-T | 20 | 200 | Vein | Single |
| 2 | 5 | BMK-CAR-T | 20 | 200 | Vein | Single |
| 3 | 5 | 1444-1182-V HH | 20 | 200 | Vein | Single |

(continued)

| Group | Mouse Number | medicament | Dose (10^6) | Administration volume (μl) | Administration route | Frequency of administration |
|-------|--------------|------------|-------------|---------------------------|----------------------|------------------------------|
| 4 | 5 | 1444-1182-FC | 20 | 200 | Vein | Single |

Among the medicaments in Table 19, CD19-CAR-T refers to CD19-targeted CAR-T, and the amino acid sequence and sequence encoding CAR are respectively as shown in SEQ ID NO: 9 and SEQ ID NO: 10 of CN109971712A, BMK-CAR-T refers to a CAR-T whose antigen binding domain is YP218scFv. The difference between BMK-CAR-T and MSLN3[1444]CAR-T is that YP218 is an antibody in the form of scFv and 1444 is a nanoantibody. Both of them bind to the mesothelin III region. YP218scFv is shown as YP218scFv of CN105026429A.

4.3 Changes in tumor volume

Figure 28 shows that the self-secreting PD1 antibody administration groups is able to completely regress the tumor at this dosage, and the efficacy is significantly different compared with the positive control medicament BMK (BMK VS 1444-1182-VHH: $P<0.0001$, BMK VS 1444-1182-FC: $P<0.0001$).

4.4 Changes in body weight

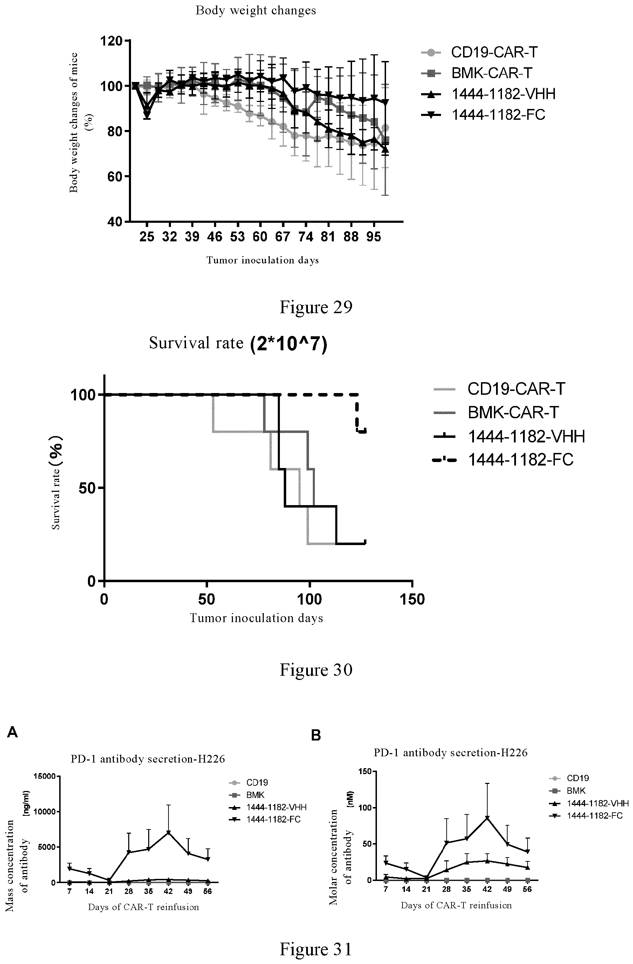

Figure 29 shows that the weight change of the administration group of self-secreting PD1 antibody having $F_C$ segment is smaller than that of the other groups and is relatively stable in the long term. After long-term observation for 100 days, most of the body weights have not dropped by 20%.

4.5 Survival period

Figure 30 shows that the survival period of the self-secreting PD1 antibody group is significantly better than that of the other groups, and the trend of change is relatively stable in the long term, among which the survival period of the mice in the group of the self-secreting PD1 antibody having $F_C$ segment exceeded 100 days.

4.6 The plasma concentration of PD-1 antibodies

Figure 31 shows that in the self-secreting PD1 antibody group, the plasma concentration of PD1 antibody carrying $F_C$ segment is significantly higher than that of PD1 antibody without $F_C$ segment, and the half-life of PD1 antibody carrying $F_C$ segment is longer in vivo. After administration, as the tumor disappears, the secretion of antibodies gradually decreases.

4.7 Changes in the number of CAR-T cells in peripheral blood

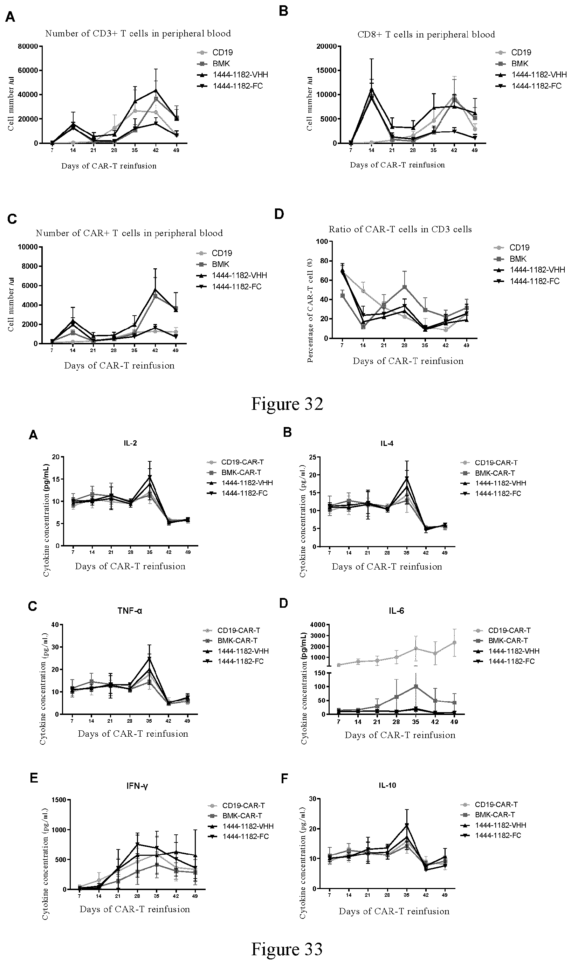

Figure 32 shows that after administration, CAR-T cells have a peak proliferation period, and then there is a downward trend as the tumor disappears. The change trends of CD3+, CD8+ and CAR+ cells are almost the same. The CAR positive rate drops briefly after administration, and then remains basically stable. The fewer the number of cells per microliter of blood, the greater the detection error.

4.8 Changes in peripheral blood cytokine concentrations

Figure 33 shows that after administration, interferon-γ and interleukin-6 are significantly secreted, among which interleukin-6 secretion is mainly concentrated in the negative control group and the BMK positive control group, while there is almost no secretion in the self-secreting PD1 antibody group. Interferon-γ is significantly secreted in all groups, which is related to the killing function of T cells. No obvious secretion is detected for the remaining cytokines, interleukin-2, interleukin-4, interleukin-10, and TNF, and the detection limit is about 10 pg/ml. It can be considered that it is not detected when it is close to this concentration.

4.9 Clinical observation of experimental animals

Figure 20: The clinical manifestations of NCI-H226-PDL1 tumor-bearing mice at high doses

| Group | Animal No. | BW (g) | a (mm) | b (mm) | TV=0.5 $\times a\times b^2$ | Comment |
|---|---|---|---|---|---|---|
| | 17 | - | - | - | - | Death was found on the 57th day after administration |
| Group 1 | 20 | - | - | - | - | Death was found on the 31th day after administration |
| CD19-CA R-T | 21 | 23.7 | 15.14 | 13.89 | **1460** | Hair loss, ulceration |
| i.v. single dose 200 μl | 33 | - | - | - | - | Death was found on the 78th day after administration |
| | 38 | - | - | - | - | Death was found on the 77th day after administration |
| | Mean | 23.7 | | | 1460 | |
| | SD | - | | | - | |
| | 18 | - | - | - | - | Death was found on the 56th day after administration |
| Group 2 | 23 | - | - | - | - | Death was found on the 77th day after administration |
| BMK-CA R-T | 26 | - | - | - | - | Death was found on the 80th day after administration |
| i.v. single dose 200 μl | 30 | 23.9 | 10.41 | 7.39 | **284** | Hair loss, hair erection |
| | 36 | - | - | - | - | Death was found on the 91th day after administration |
| | Mean | 23.9 | | | 284 | |
| | SD | - | | | - | |
| | 2 | - | - | - | - | Death was found on the 63th day after administration |
| Group 3 | 4 | 17.5 | 3.05 | 2.53 | **10** | Severe hair loss, hunched back |
| 1444-1182 -VHH | 12 | - | - | - | - | Death was found on the 76th day after administration |
| i.v. single dose 200 μl | 16 | - | - | - | - | Death was found on the 63th day after administration |
| | 25 | - | - | - | - | Death was found on the 91th day after administration |
| | Mean | 17.5 | | | 10 | |
| | SD | - | | | - | |
| | 7 | - | - | - | - | Death was found on the 102th day after administration |
| Group 4 | 10 | 21.0 | 3.01 | 2.00 | **6** | Hair loss |
| 1444-1182 -FC | 22 | 24.3 | 2.98 | 2.10 | **7** | Hair loss |
| i.v. single dose 200 μl | 24 | 25.1 | 3.37 | 2.91 | **14** | Hair loss |
| | 35 | 25.1 | 3.69 | 3.02 | **17** | Slight hair loss |

(continued)

| Group | Anima l No. | BW (g) | a (mm) | b (mm) | TV=0.5 $\times a \times b^2$ | Comment |
|---|---|---|---|---|---|---|
| | Mean | 23.9 | | | 11 | |
| | SD | 2.0 | | | 5 | |

Clinical observation results show that the survival of mice in the negative control group, BMK positive control group and 1444-1182-VHH (antibody without $F_C$ segment) is slightly worse than that in the 1444-1182-$F_C$ administration group. The survival period of animals in 1444-1182-$F_C$ administration group exceed 100 days.

Example 5, Efficacy, pharmacokinetics and safety verification test at commonly used dosage

5.1 Experimental Materials

Experimental animals and cells

Experimental mice is the same as in Example 4.

Tumor cells:

Skov3-PDL1 (ATCC) that highly express PDL1
NCI-H226-PDL1 (ATCC) that highly express PDL1

Experimental process is the same as in Example 3.
The preparation process of BMK-1182 CAR-T cells refers to the "CAR-T preparation operation step". The process is briefly described as follows: fresh or frozen PBMCs were revived and electroporated using a Lonza 4D electroporator. The electroporation system is 100 μl, the plasmid dosage is 4 μg pNB338B-1444-CAR + 4 μg pS338B-1182 plasmid, and the electroporation program is EO-115. The passage process, detection time and detection indicators are consistent with other CAR-T cells.

5.2 Inoculation and administration regimen

Table 21, Inoculation regimen and grouping

| Group | Number of mice | Inoculate cells | Dose (10^6) | Volume (μL) | Administration route | Position | Comment |
|---|---|---|---|---|---|---|---|
| A | 29 | NCI-H22 6-PDL1 | 10 | 200 | Subcutaneous | Upper tight | Adding Matrix |
| B | 29 | Skov3-PDL1 | 10 | 200 | Subcutaneous | Upper tight | Adding Matrix |

Table 22, Administration regimen and grouping (NCI-H226-PDL1)

| Group | Number of mice | Medicament | Dose (10^6) | Administration volume (μl) | Admini stration route | Frequency of administration |
|---|---|---|---|---|---|---|
| B1 | 5 | CD19-1182 | 10 | 200 | Vein | Single |
| B2 | 5 | Meso3CAR (BMK ) | 10 | 200 | Vein | Single |

(continued)

| Group | Number of mice | Medicament | Dose (10^6) | Administration volume (μl) | Admini stration route | Frequency of administration |
|---|---|---|---|---|---|---|
| B3 | 5 | Meso3CAR (BMK )-1182-FC | 10 | 200 | Vein | Single |
| B4 | 5 | 1444-1182-FC | 10 | 200 | Vein | Single |

Among the medicaments in Table 22, CD19-1182 is the same as the above-mentioned CD19-1182-VHH, Meso3CAR(BMK) is the same as the above-mentioned BMK-CAR-T, and the difference between Meso3CAR(BMK)-1182-FC and Meso3CAR(BMK) is that 1182-FC is also secreted.

Table 23, Administration regimen and grouping (Skov3-PDL1)

| Group | Number of mice | Medicament | Dose (10^6) | Administration volume (μl) | Administration route | Frequency of administra tion |
|---|---|---|---|---|---|---|
| B1 | 5 | CD19-1182 | 10 | 200 | Vein | Single |
| B2 | 5 | Meso3CAR (BMK) | 10 | 200 | Vein | Single |

5.3 Changes of tumor volume and body weight

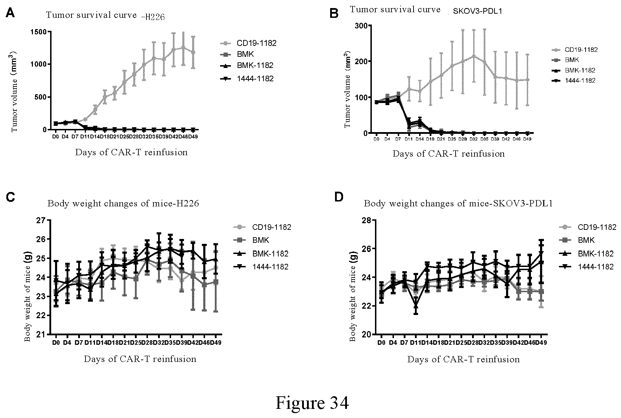

Figure 34 shows that both the positive control group and the test group are able to completely regress the tumor at this dosage.

5.4 The plasma concentration of PD-1 antibodies

The results are shown in Figure 45. The results show that in the groups administrated with the self-secreting PD1 antibody, the medicament metabolism characteristics of the PD1 antibody carrying the $F_C$ segment are relatively similar in vivo. With the clearance and regression of the tumor, they all show a trend of first rising and then falling, and this has been verified in two tumor-bearing mouse models. Moreover, without affecting the efficacy, the PD1 antibody has the lowest plasma concentration, the lowest risk of systemic toxic side effects, and the highest safety.

5.5 Changes in the number of CAR-T cells in peripheral blood

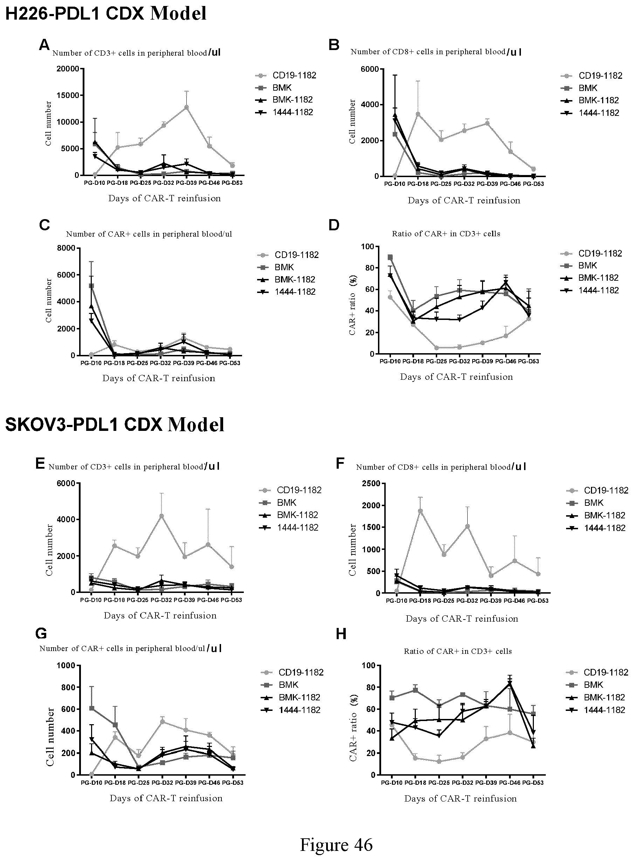

The results are shown in Figure 46. After administration, CAR-T cells have a peak proliferation period, and then there is a downward trend as the tumor regressed. The change trends of CD3+, CD8+ and CAR+ cells are almost the same. The CAR positive rate shows a short decline after administration, and then remains basically stable. Compared with blood-tumor-targeted CAR-T, the concentration of mesothelin-targeted CAR-T in peripheral blood is significantly lower, indicating that these CAR-T are mainly located in the tumor rather than in the peripheral blood, thus achieving the purpose of precise targeted therapy.

Example 6, In vitro experiments of MSLN CAR-T cells of other MSLN CAR-T cells and self-secreting PD1 nanoantibodies

In this embodiment, the expression sequence for expressing PB enzyme on the CAR plasmid used for CAR-T cells is removed, and PB enzyme is added to the

| B3 | 5 | Meso3CAR(BMK) -1182-FC | 10 | 200 | Vein | Single |
| B4 | 5 | 1444-1182-FC | 10 | 200 | Vein | Single |

electroporation system in the form of mRNA and electroporated into T cells together with the plasmid for expression; the PD1 antibody secretory plasmid is changed: the promoter is replaced from the CMV promoter to the IFNy driven promoter.

Brief description of preparation method of CAR-T: the electroporation system, electroporation procedure and passage process are the same as the previous CAR-T preparation. The addition of plasmids and other components in the electroporation system: single plasmid 6 $\mu$g + 20 $\mu$g PBase mRNA + 200 IU RNase inhibitor or dual plasmid 4 $\mu$g CAR plasmid + 4 $\mu$g secretory plasmid + 20 $\mu$g PBase mRNA + 200 IU RNase inhibitor.

The inventors have constructed:

CAR-T cells containing mesothelin nanoantibody MSLN3[1444] (abbreviated as 1444), CAR-T cells containing mesothelin nanoantibody MSLN3[1444] secreting PD1 nanoantibody 1194nla (SEQ ID NO: 131) (abbreviated as " 1444+1194nla"or" 1444-1194"), the CAR sequence of the above cells is shown in SEQ ID NO: 122; and

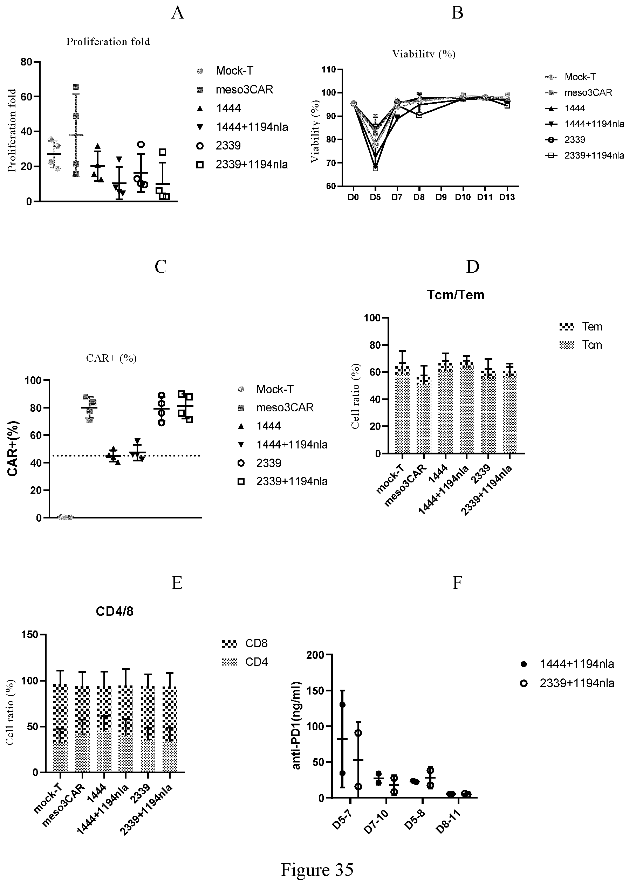

CAR-T cells containing mesothelin-targeted nanoantibody MSLN3[2339] (SEQ ID NO: 103)(abbreviated as 2339), CAR-T cells containing mesothelin-targeted nanoantibody MSLN3[2339] secreting PD1 nanoantibody 1194nla (abbreviated as "1444+ 1194nla" or "2339-1194"), the CAR sequence of the above cells is shown in SEQ ID NO: 121; and The results are shown in Figure 35-38.

Figure 35 shows that all CAR-T cells can proliferate effectively and have high viability (more than 90%) and high CAR positive rate; CD3/4/8 ratio, except for Donor 04A which is relatively low, the other donors have little difference, about 1:1; Tcm and Tem donors have little difference.

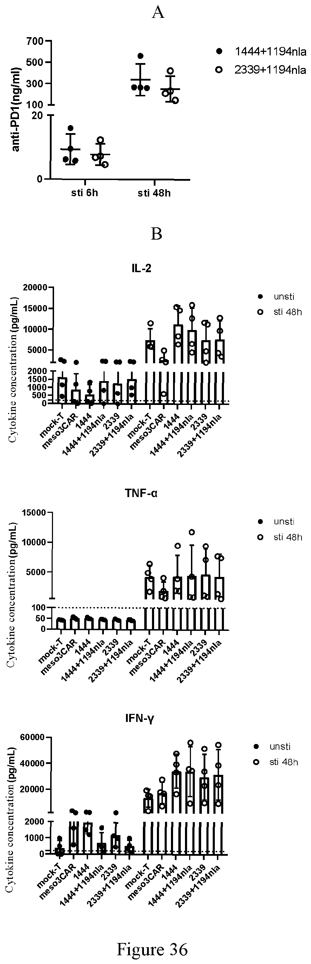

Figure 36 shows that the secretion level of anti-PD1 (1194nla) during the preparation process is low, between 0-150 ng/ml; over time, the secretion of 1194nla gradually decreased; there is no significant difference between the secretion of the 1444+1194nla group and the 2339+1194nla group. After stimulation for 6h, the secretion of 1194nla is low, below 100 ng/ml; after stimulation for 48 h, the secretion of 1194nla is significantly higher than that of 6 h; after stimulation for 48 h, the secretion of the 1444+1194nla group is slightly higher than that of the 2339+1194nla group. After antigen stimulation for 48 h, the ability of CAR-T cells in each group to produce cytokines is significantly increased compared with that before stimulation. The ability of CAR-T in the group 1444 and the group 2339 to produce IL-2, IFNy and TNF$\alpha$ is slightly different, all of which are higher than BMK (meso3CAR).

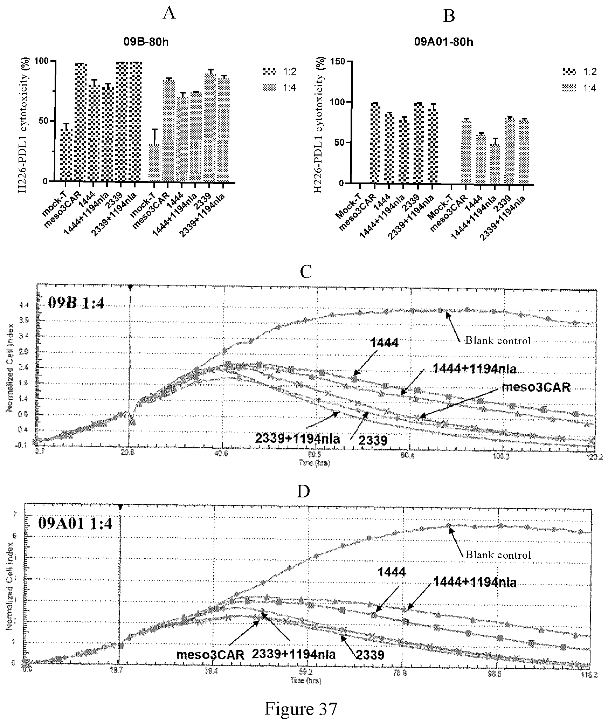

Figure 37 shows that both group 1444 and group 2339 can effectively kill H226-PDL1 cells; the killing ability of group 2339 on H226-PDL1 is stronger than that of group 1444; secreting 1194nla has a certain improvement on the killing ability of MSLN CAR-T.

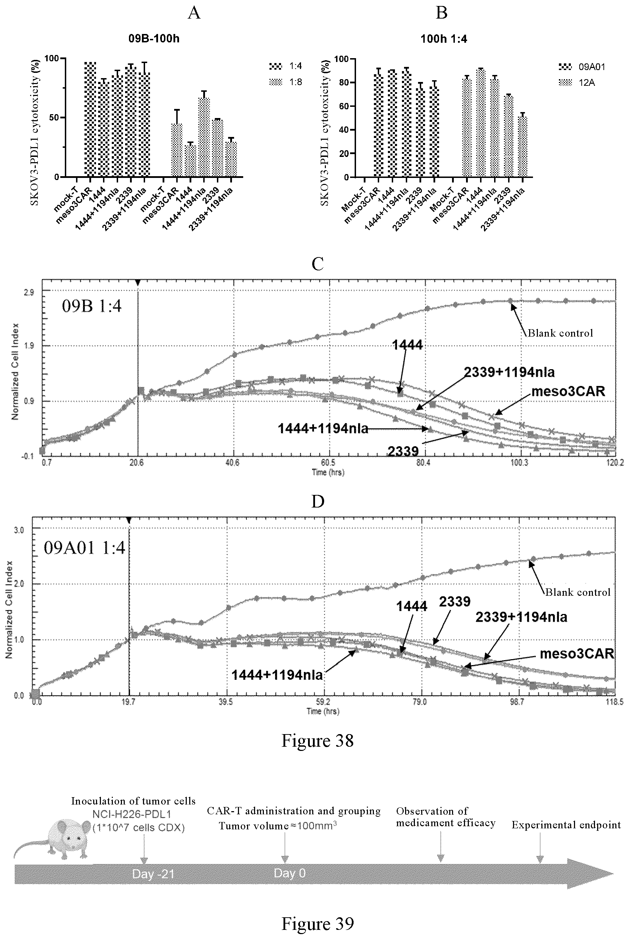

Figure 38 shows that both group 1444 and group 2339 can effectively kill SKOV3-PDL1 cells; the killing ability of group 1444 on SKOV3-PDL1 is stronger than that of group 2339; secreting 1194nla has a certain improvement on the killing ability of MSLN CAR-T.

Example 7, In vivo dose-response experiments of other MSLN CAR-T cells and MSLN CAR-T cells secreting PD1 nanoantibodies

Experimental process

Severely immunodeficient mice NPSG mice (Phenotek) are subcutaneously inoculated with 1E7 tumor cells of NCI-H226-PDL1. After about 21 days of inoculation, the tumor grows to 100-120mm$^3$, and the mice are divided into groups and intravenously infused with CAR-T. The infusion volume of each group is divided into 4 dose gradients (0.2E6, 1E6, 5E6, 20E6, respectively), and the efficacy, body weight and survival are observed. Peripheral blood is collected once a week to detect the proportion and number of CD3 in peripheral blood. The experimental regimen is shown in the following table and Figure 39.

| Group | Number of mice | medicament | Dose (10^6) |
|---|---|---|---|
| 1 | 6 | Vehicle | -- |
| 2 | 6 | MOCK-T | 0.2 |
| 3 | 6 | 2339-1194 | 0.2 |
| 4 | 6 | 1444-1194 | 0.2 |
| 5 | 6 | MOCK-T | 1 |
| 6 | 6 | 2339-1194 | 1 |
| 7 | 6 | 1444-1194 | 1 |
| 8 | 6 | MOCK-T | 5 |

(continued)

| Group | Number of mice | medicament | Dose (10^6) |
|---|---|---|---|
| 9 | 6 | 2339-1194 | 5 |
| 10 | 6 | 1444-1194 | 5 |
| 11 | 6 | MOCK-T | 20 |
| 12 | 6 | 2339-1194 | 20 |
| 13 | 6 | 1444-1194 | 20 |

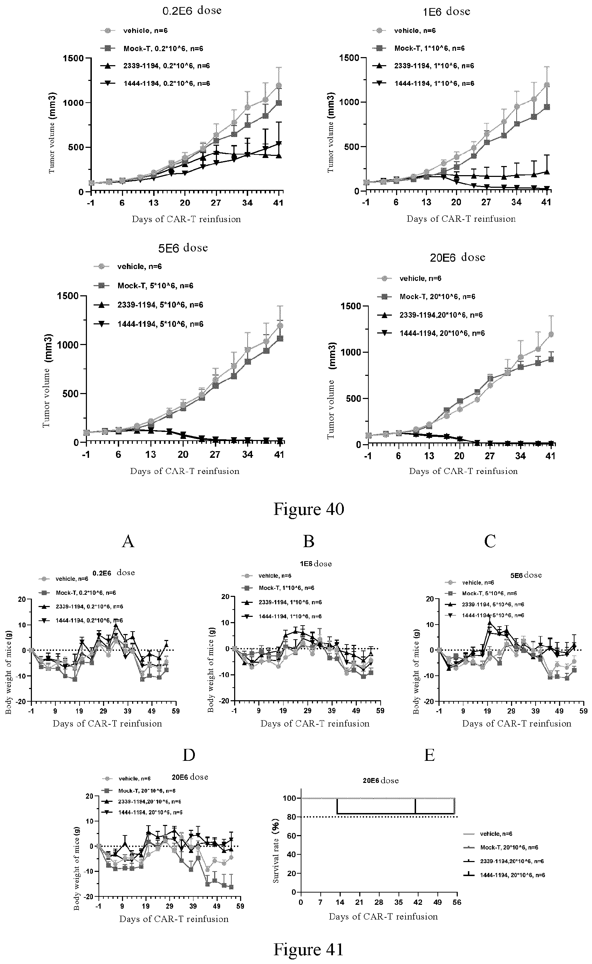

The experimental results are shown in Figure 40 to 43.

Figure 40 shows that 2339-1194 and 1444-1194 can inhibit tumor growth at different doses (1E6, 5E6 and 20E6), with an inhibition rate of more than 70%. 2339-1194 and 1444-1194 have comparable tumor inhibition effects.

Figure 41 shows that at different dosages, the weight change of mice is within 20%, with good safety. There is no significant difference in weight change and survival rate between 2339-1194 and 1444-1194.

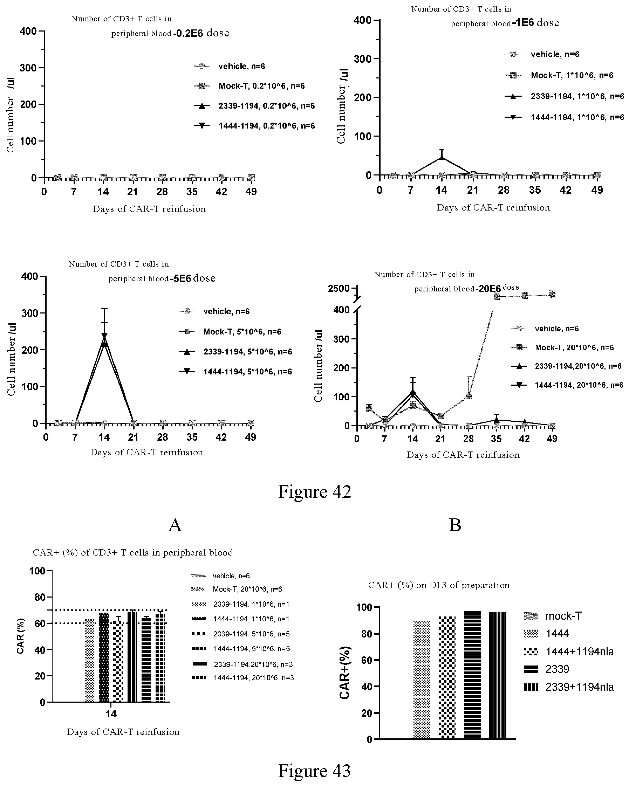

Figure 42 shows that the peripheral blood CD3+T cells reach a peak on day 14 and drop to a low point on day 21, at which time the tumor has basically regressed.

Figure 43 shows that 14 days after CAR-T infusion, the proportion of CAR+ in peripheral blood is relatively stable (between 60% and 70%) at different dosages.

Conclusions

MSLN CAR-T cells secreting PD-1 antibody can secrete PD-1 antibodies and express chimeric antigen receptors targeting mesothelin antigens. They have both the ability to secrete immune checkpoint antibodies and the specific killing ability targeting specific antigens. They have a stronger ability to secrete cytokines and a higher killing rate than ordinary CAR-T cells.

**Claims**

1. A chimeric antigen receptor whose antigen-binding domain comprises a mesothelin-binding molecule containing an anti-mesothelin single domain antibody,

   preferably, the anti-mesothelin single domain antibody comprises CDR1, CDR2 and CDR3, wherein CDR1 comprises the sequence shown in SEQ ID NO:1, CDR2 comprises the sequence shown in SEQ ID NO:2, and CDR3 comprises the sequence shown in SEQ ID NO:3, wherein
   SEQ ID NO: 1 is $X_1X_2X_3X_4X_5X_6X_7X_8X_9X_{10}X_{11}$, wherein $X_1$ is G, E, T or A, $X_2$ is S, N, P, A, H, D, K, I or F, $X_3$ is I, S, V, T, D, L, A, M or H, $X_4$ is F, S, I, A, L or G, $X_5$ is N, S, G, A, D, T, E, R or H, $X_6$ is I, L, F, Y, E or N, $X_7$ is N, A, G, D, K, Y, L or S, $X_8$ is A, Y, V, D or N, $X_9$ is E or null, $X_{10}$ is F or null, $X_{11}$ is A or null,
   SEQ ID NO: 2 is $X_1X_2X_3X_4X_5X_6X_7X_8X_9X_{10}X_{11}X_{12}$, wherein $X_1$ is I, M, A, T or L, $X_2$ is S, G, N, D, T or V, $X_3$ is S, N, A, R, G or T, $X_4$ is S, T, G, D or N, $X_5$ is N, G, T or I, $X_6$ is S, R, N, D, K, T or G, $X_7$ is D, T, S, I or K, $X_8$ is N, T, G, D or null, $X_9$ is T, K, G or null, $X_{10}$ is G, R or null, $X_{11}$ is V, T or null, $X_{12}$ is T or null,
   SEQ ID NO: 3 is $X_1X_2X_3X_4X_5X_6X_7X_8X_9X_{10}X_{11}X_{12}X_{13}X_{14}X_{15}X_{16}X_{17}X_{18}X_{19}X_{20}X_{21}X_{22}X_{23}$, wherein $X_1$ is N, H, A or Q, $X_2$ is L, A, G or V, $X_3$ is S, R, E, G, D or T, $X_4$ is N, A, R, G, D, K, V, T, I, E or Q, $X_5$ is Y, F, K, S, C, I, D, G, H or R, $X_6$ is D, A, V, G, R, N, P, T, C, Q or S, $X_7$ is R, Y, T, A, D, K, L, E, S or G, $X_8$ is K, S, F, T, D, Q, E, G, P, R, Y or N, $X_9$ is D, G, I, H, S, Y, Q, K, T, R or V, $X_{10}$ is R, Y, H, N, T, D, P, V, K, C, L or S, $X_{11}$ is Y, D, E, P, F, S, L, I or null, $X_{12}$ is P, V, Q, A, Y, F, N, D, R or null, $X_{13}$ is D, A, Y, V, Q, P or null, $X_{14}$ is Y, L, P, A, E or null, $X_{15}$ is C, S, M, P, T or null, $X_{16}$ is V, D, S, Y or null, $X_{17}$ is L, F, V, D or null, $X_{18}$ is R, G, M, S or null, $X_{19}$ is D, N, S or null, $X_{20}$ is Y, L or null, $X_{21}$ is Y or null, $X_{22}$ is A or null, $X_{23}$ is D or null;
   preferably, the chimeric antigen receptor comprises a mesothelin-binding molecule containing an anti-mesothelin single domain antibody, a hinge region, a transmembrane region, and an intracellular region.

2. The chimeric antigen receptor according to claim 1, wherein the mesothelin binding molecule is a monovalent or multivalent single domain antibody, a multispecific single domain antibody, a heavy chain antibody or an antigen binding fragment thereof, or an antibody or an antigen binding fragment thereof, comprising one, two or more of the single domain antibodies.

3. The chimeric antigen receptor according to claim 2, wherein

the CDR1 of the anti-mesothelin single domain antibody comprises the sequence shown in any one of SEQ ID NOs: 4-27, 73; and/or
the CDR2 of the anti-mesothelin single domain antibody comprises the sequence shown in any one of SEQ ID NOs: 28-47, 74; and/or
the CDR3 of the anti-mesothelin single domain antibody comprises the sequence shown in any one of SEQ ID NOs: 48-72, 75.

4. The chimeric antigen receptor according to claim 2, wherein the chimeric antigen receptor further comprises one or more features selected from the group consisting of:

the signal peptide comprises a CD8 signal peptide, a CD28 signal peptide, a CD4 signal peptide or a light chain signal peptide,
the hinge region comprises a CD8 hinge region, an IgD hinge region, an IgG1 Fc CH2CH3 hinge region, or an IgG4 Fc CH2CH3 hinge region,
the transmembrane region comprises a CD28 transmembrane region, a CD8 transmembrane region, a CD3$\zeta$ transmembrane region, a CD134 transmembrane region, a CD137 transmembrane region, an ICOS transmembrane region or a DAP 10 transmembrane region,
the intracellular domain comprises an intracellular co-stimulatory domain and/or an intracellular signaling domain,
the intracellular co-stimulatory domain comprises an intracellular domain of CD28, CD134/OX40, CD137/4-1BB, lymphocyte-specific protein tyrosine kinase, inducible T cell co-stimulator (ICOS) or DNAX activating protein 10,
the intracellular signaling domains comprises a CD3$\zeta$ intracellular signaling domain or a Fc$\epsilon$RI$\gamma$ intracellular signaling domain.

5. A nucleic acid molecule comprising a sequence selected from the group consisting of:

(1) a sequence encoding the chimeric antigen receptor according to any one of claims 1-4,
(2) a complementary sequence of (1), and
(3) a 5-50 bp fragment of any sequence of (1) or (2),

preferably,

the fragment is primer, and/or
the encoding sequence is DNA or RNA.

6. A nucleic acid construct, wherein the nucleic acid construct comprises the nucleic acid molecule according to claim 5, preferably, the nucleic acid construct further comprises one or more features selected from the group consisting of:

the nucleic acid construct further comprises a sequence encoding a PD-1 binding molecule,
the nucleic acid construct further comprises a sequence encoding a transposase, which is used to transpose the sequence encoding the chimeric antigen receptor and/or the PD-1 binding molecule,
the sequence encoding the transposase is DNA or RNA,
the sequence encoding the PD-1 binding molecule is DNA or RNA,
the nucleic acid construct is a cloning vector, an expression vector or an integration vector,
the transposase is a PiggyBac transposase.

7. A combination of encoding sequences, comprises a sequence coding the chimeric antigen receptor according to any one of claims 1 to 4, and further comprises a sequence encoding a transposase and/or a sequence encoding a PD-1 binding molecule, preferably, the encoding sequence is DNA or RNA, and the transposase is PiggyBac transposase.

8. A cell, wherein the cell:

(1)(a) comprises and/or expresses the chimeric antigen receptor according to any one of claims 1 to 4, and optionally (b) comprises, expresses and/or secrets a PD-1 binding molecule, and/or
(2) comprises the nucleic acid molecule according to claim 5 and/or the nucleic acid construct according to claim 6, and/or
(3) comprises the combination of encoding sequences according to claim 7;

preferably, the cell is a T cell.

9. A pharmaceutical composition, wherein the pharmaceutical composition comprises pharmaceutically acceptable excipients and:

(1)(i) the chimeric antigen receptor according to any one of claims 1 to 4 and/or a sequence encoding the chimeric antigen receptor, optionally comprises (ii) a transposase and/or a sequence encoding the transposase , and/or (iii) a PD-1 binding molecule and/or a sequence encoding the PD-1 binding molecule , or
(2) the nucleic acid molecule according to claim 5, the nucleic acid construct according to claim 6, the combination of encoding sequences according to claim 7, or the cells according to claim 8.

10. Use of a reagent in manufacture of an activated immune cell, wherein the reagent comprises:

(1)(i) the chimeric antigen receptor according to any one of claims 1 to 4 and/or a sequence encoding the chimeric antigen receptor, optionally comprises (ii) a transposase and/or a sequence encoding the transposase , and/or (iii) a PD-1 binding molecule and/or a sequence encoding the PD-1 binding molecule, or
(2) the nucleic acid molecule according to claim 5, the nucleic acid construct according to claim 6, or the combination of encoding sequences according to claim 7.

11. Use of a reagent in manufacture of a medicament for treating cancer, wherein the reagent comprises:

(1)(i) the chimeric antigen receptor according to any one of claims 1 to 4 and/or a sequence encoding the chimeric antigen receptor, optionally comprises (ii) a transposase and/or a sequence encoding the transposase , and/or (iii) a PD-1 binding molecule and/or a sequence encoding the PD-1 binding molecule, or
(2) the nucleic acid molecule according to claim 5, the nucleic acid construct according to claim 6, the combination of encoding sequences according to claim 7, or the cells according to claim 8,
preferably, the cancer comprises mesothelin-mediated cancers, optionally further comprises PD-1-mediated cancers,
more preferably, the cancer is selected from the group consisting of: mesothelioma, pancreatic cancer, ovarian cancer, cervical cancer, endometrial cancer, rectal cancer, esophageal cancer, lung adenocarcinoma, gastric cancer, melanoma, lung cancer, head and neck cancer, renal cell carcinoma, urothelial cancer, non-Hodgkin lymphoma.

A

AC201908B

B

AC-201909A

C

AC-201910A

D

Cell proliferation

E

Proliferation fold

Figure 1

Viability (%)

Figure 2

Figure 3

Figure 4

Figure 5

Figure 6

**A** SKOV3 cytotoxicity (1:1 72h)

**B** H226 cytotoxicity (1:1 72h)

Figure 7

**A** IFN-γ

**B** IL-2

**C** TNF-α

Figure 8

Figure 9

Figure 10

A  Cell number of CAR+

B  CAR+ (%) on Day 13

Figure 11

CD4/CD8 in CD3 cells

Figure 12

Memory T cell ratio (Day 13)

Figure 13

Figure 14

Figure 15

Figure 16

Figure 17

A

**72h 1:4**

B

C

Figure 18

A

IL-2

B

IFN-γ

C

TNF-α

Figure 19

Figure 20

Figure 21

Figure 22

Figure 23

Figure 24

Figure 25

Figure 26

Figure 27

NCI-H226 model

Figure 28

Body weight changes

Figure 29

Survival rate **(2\*10^7)**

Figure 30

A

PD-1 antibody secretion-H226

B

PD-1 antibody secretion-H226

Figure 31

**A**

Number of CD3+ T cells in peripheral blood

**B**

CD8+ T cells in peripheral blood

**C**

Number of CAR+ T cells in peripheral blood

**D**

Ratio of CAR-T cells in CD3 cells

Figure 32

**A**

IL-2

**B**

IL-4

**C**

TNF-α

**D**

IL-6

**E**

IFN-γ

**F**

IL-10

Figure 33

Figure 34

Figure 35

A

B

Figure 36

A

**09B-80h**

B

**09A01-80h**

C

D

Figure 37

Figure 38

Figure 39

Figure 40

Figure 41

Figure 42

A

B

Figure 43

Figure 44

Figure 45

# H226-PDL1 CDX Model

**A** Number of CD3+ cells in peripheral blood/ul

**B** Number of CD8+ cells in peripheral blood/u l

**C** Number of CAR+ cells in peripheral blood/ul

**D** Ratio of CAR+ in CD3+ cells

# SKOV3-PDL1 CDX Model

**E** Number of CD3+ cells in peripheral blood/u l

**F** Number of CD8+ cells in peripheral blood/u l

**G** Number of CAR+ cells in peripheral blood/ul/u l

**H** Ratio of CAR+ in CD3+ cells

Figure 46

## INTERNATIONAL SEARCH REPORT

| International application No. |
|---|
| **PCT/CN2022/143407** |

| **A.** | **CLASSIFICATION OF SUBJECT MATTER** |
|---|---|

C12N5/10(2006.01)i;C07K16/28(2006.01)i;C12N15/13(2006.01)i;C12N 15/62(2006.01)i;A61K39/395(2006.01)i;A61P35/00(2006.01)i

According to International Patent Classification (IPC) or to both national classification and IPC

| **B.** | **FIELDS SEARCHED** |
|---|---|

Minimum documentation searched (classification system followed by classification symbols)

C12N,C07K,A61K,A61P

Documentation searched other than minimum documentation to the extent that such documents are included in the fields searched

Electronic data base consulted during the international search (name of data base and, where practicable, search terms used)

CNTXT, VEN, VCN, WPABSC, EXTXT, WOTXT, USTXT, PATENTICS, CNKI, 万方, WANFANG, PUBMED, NCBI, 百度学术, BAIDU XUESHU, BING, STN: 间皮素, 单域抗体, 重链抗体, 纳米抗体, 嵌合抗原受体, MSLN, mesothelin, single domain antibody, sdAb, nanobody, Nb, CAR, PD-1, 序列4-75

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | WO 2022143550 A1 (ZHEJIANG NANOMAB TECHNOLOGY CENTER CO., LTD.) 07 July 2022 (2022-07-07) claims 1-15 | 1-11 |
| Y | CN 109971714 A (SHANGHAI CELL THERAPY RESEARCH INSTITUTE et al.) 05 July 2019 (2019-07-05) claims 1-10, and description, paragraph 85 | 1, 2, 4, 11 |
| Y | CN 108129566 A (WUHAN INSTITUTE OF VIROLOGY, CHINESE ACADEMY OF SCIENCES) 08 June 2018 (2018-06-08) claims 1-10 | 1, 2, 4-11 |
| Y | CN 109971721 A (SHANGHAI CELL THERAPY RESEARCH INSTITUTE et al.) 05 July 2019 (2019-07-05) claims 1-10 | 1, 2, 4-11 |
| Y | US 2015274836 A1 (The United States of America,as represented by the Secretary,Department of Health and Human Services) 01 October 2015 (2015-10-01) claims 1-26 | 1, 2, 4, 11 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **09 March 2023** | **16 March 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

| International application No. |
| --- |
| **PCT/CN2022/143407** |

### C.    DOCUMENTS CONSIDERED TO BE RELEVANT

| Category* | Citation of document, with indication, where appropriate, of the relevant passages | Relevant to claim No. |
| --- | --- | --- |
| A | CN 110507824 A (REMEGEN LTD.) 29 November 2019 (2019-11-29)<br>    entire document | 1-11 |
| Y | LI, Q. et al. "Preclinical Evaluation of a Novel Anti-Mesothelin Immunotoxin Based on a Single Domain Antibody as the Targeting Ligand"<br>*International Journal of Pharmaceutics,* 26 April 2021 (2021-04-26),<br>    article number 20647, pp. 1-12 | 1, 2, 4-11 |

Form PCT/ISA/210 (second sheet) (July 2022)

**INTERNATIONAL SEARCH REPORT**

International application No.

**PCT/CN2022/143407**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.   ☑   forming part of the international application as filed.

    b.   ☐   furnished subsequent to the international filing date for the purposes of international search (Rule 13*ter*.1(a)),

             ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2022/143407**

| Patent document cited in search report | | | Publication date (day/month/year) | Patent family member(s) | | | Publication date (day/month/year) |
|---|---|---|---|---|---|---|---|
| WO | 2022143550 | A1 | 07 July 2022 | None | | | |
| CN | 109971714 | A | 05 July 2019 | WO | 2019128998 | A1 | 04 July 2019 |
| CN | 108129566 | A | 08 June 2018 | None | | | |
| CN | 109971721 | A | 05 July 2019 | WO | 2019128996 | A1 | 04 July 2019 |
| US | 2015274836 | A1 | 01 October 2015 | HK | 1213000 | A1 | 24 June 2016 |
| | | | | WO | 2014052064 | A1 | 03 April 2014 |
| | | | | CA | 2885761 | A1 | 03 April 2014 |
| | | | | CA | 2885761 | C | 21 September 2021 |
| | | | | US | 9416190 | B2 | 16 August 2016 |
| | | | | JP | 2016500655 | A | 14 January 2016 |
| | | | | JP | 6307085 | B2 | 04 April 2018 |
| | | | | EP | 2900695 | A1 | 05 August 2015 |
| | | | | EP | 2900695 | B1 | 17 January 2018 |
| | | | | ES | 2666131 | T3 | 03 May 2018 |
| | | | | AU | 2013324049 | A1 | 19 March 2015 |
| | | | | AU | 2013324049 | B2 | 21 September 2017 |
| CN | 110507824 | A | 29 November 2019 | US | 2022056147 | A1 | 24 February 2022 |
| | | | | AU | 2019268215 | A1 | 19 December 2019 |
| | | | | AU | 2019268215 | B2 | 18 March 2021 |
| | | | | BR | 112020014380 | A2 | 29 December 2020 |
| | | | | RU | 2747995 | C1 | 18 May 2021 |
| | | | | SG | 11202006515 | VA | 28 August 2020 |
| | | | | EP | 3797795 | A1 | 31 March 2021 |
| | | | | EP | 3797795 | A4 | 06 April 2022 |
| | | | | CA | 3062265 | A1 | 21 November 2019 |
| | | | | CA | 3062265 | C | 22 March 2022 |
| | | | | BR | 112020005596 | A2 | 29 September 2020 |
| | | | | SG | 11202011159 | QA | 30 December 2020 |
| | | | | WO | 2019223653 | A1 | 28 November 2019 |
| | | | | JP | 2020533398 | A | 19 November 2020 |
| | | | | JP | 6949206 | B2 | 13 October 2021 |
| | | | | CA | 3082160 | A1 | 28 November 2019 |
| | | | | AU | 2019272250 | A1 | 19 March 2020 |
| | | | | AU | 2019272250 | B2 | 13 January 2022 |
| | | | | JP | 2021523874 | A | 09 September 2021 |
| | | | | TW | 202003040 | A | 16 January 2020 |
| | | | | TWI | 714092 | B | 21 December 2020 |
| | | | | KR | 20200098626 | A | 20 August 2020 |
| | | | | KR | 102454638 | B1 | 14 October 2022 |
| | | | | US | 2020138968 | A1 | 07 May 2020 |
| | | | | WO | 2019223579 | A1 | 28 November 2019 |
| | | | | RU | 2724436 | C1 | 23 June 2020 |
| | | | | EP | 3695852 | A1 | 19 August 2020 |
| | | | | EP | 3695852 | A4 | 15 September 2021 |
| | | | | KR | 20200120728 | A | 21 October 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 105154473 A
- CN 202011584014
- CN 202011582948
- CN 202011582908
- WO 0196584 A
- WO 0129058 A
- US 6326193 B

- CN 201510638974
- CN 111206043 A
- CN 114729361 A
- CN 109988759 A
- CN 109306014 A
- CN 109971712 A
- CN 105026429 A


**Non-patent literature cited in the description**

- **JIANG, H. et al.** *Protein Cell,* 2017, vol. 8, 926-931
- **ADUSUMILLI, P. S.** *Sci. Transl. Med,* 2014, vol. 6, 261-151
- **LANITIS, E.** *Mol. Ther.,* 2012, vol. 20, 633-643
- **MUYLDERMANS S.** *AnnuRev Biochem.,* 2013, vol. 82, 775-97

- **SARWISH RAFIQ et al.** *Nat Biotechnol,* October 2018, vol. 36 (9), 847-856
- **MARTIN N et al.** *Macromol Biosci.,* February 2017, vol. 17 (2
- **SAMBROOK et al.** Molecular Cloning: A Laboratory Manual. Cold Spring Harbor Laboratory, 2001
- **ROSENBERG et al.** *New Eng. J. of Med.,* 1988, vol. 319, 1676